# EUROPEAN PATENT APPLICATION

(11) **EP 4 043 444 A1**
(43) Date of publication of application: **17.08.2022**
(21) Application number: 21156573.4
(22) Date of filing: 11.02.2021
(51) Int. Cl.: C07D 261/04, C07D 413/04, C07D 413/12, C07D 471/12, A01N 43/80

(54) **SUBSTITUTED ISOXAZOLINE DERIVATIVES**

(71) Applicant: BASF SE, 67056 Ludwigshafen am Rhein (DE)
(72) Inventor: Huwyler, Nikolas, 67056 Lidwigshafen (DE); Koerber, Karsten, 67056 Lidwigshafen (DE); Gilberg, Erik, 67056 Lidwigshafen (DE); Sambasivan, Sunderraman, 400705 Navi Mumbai (IN)
(74) Representative: BASF IP Association

(57) **Abstract**

The invention relates to isoxazoline compounds of formula I 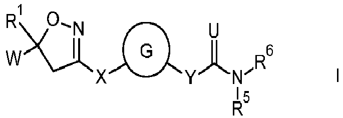 wherein the variables have the meanings as defined in the specification, to compositions comprising them, to active compound combinations comprising them, and to their use for protecting growing plants and animals from attack or infestation by invertebrate pests, furthermore, to seed comprising such compounds.

## Description

The invention relates to isoxazoline compounds of formula I 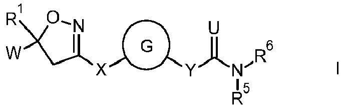 wherein
R¹ is C₁-C₂-haloalkyl;
W is phenyl, or 5- or 6-membered heteroaryl comprising as ring members 1, 2, or 3 heteroatoms selected from N, O, and S; wherein W is unsubstituted, partially or fully substituted with R²;
   R² is halogen, OR²¹, NR²²R²³, CN, NO₂, Si(CH₃)₃, SbF₅, C₁-C₄-alkyl, C₁-C₄-haloalkyl, C₂-C₄-alkenyl, C₂-C₄-haloalkenyl, C₂-C₄-alkynyl, C₂-C₄-haloalkynyl, C₁-C₃-alkoxy, C₁-C₃-haloalkoxy, C₁-C₃-alkyl-S(O)ₘ, C₁-C₃-haloalkyl-S(O)ₘ, C₁-C₃-alkoxy-C₁-C₄-alkyl, C₁-C₃-haloalkoxy-C₁-C₄-alkyl, C₁-C₃-alkyl-S(O)ₘ-C₁-C₄-alkyl, C₁-C₃-haloalkyl-S(O)ₘ-C₁-C₄-alkyl, C₃-C₆-cycloalkyl, C₃-C₆-halocycloalkyl; which groups are optionally substituted with R²¹¹;
   m is 0, 1, or 2;
   R²¹ H, C₁-C₆-alkyl, C₂-C₆-alkenyl, C₂-C₆-alkynyl, C₃-C₈-cycloalkyl, Si(C₁-C₄-alkyl)₃, which groups are unsubstituted, partially or fully substituted with R²¹¹;
      R²¹¹ halogen, C₁-C₄-alkoxy, C₁-C₄-haloalkoxy, C₃-C₄-alkenyloxy, C₃-C₄-haloalkenyloxy, C₃-C₄-alkynyloxy, C₃-C₄-haloalkynyloxy, C₁-C₄-alkyl-S(O)ₘ, C₁-C₄-haloalkyl-S(O)ₘ, C₃-C₄-alkenyl-S(O)ₘ, C₃-C₄-haloalkenyl-S(O)ₘ, C₃-C₄-alkynyl-S(O)ₘ, C₃-C₄-haloalkynyl-S(O)ₘ, and oxo; C3-C8-cycloalkyl, C₃-C₈-halocycloalkyl, C₃-C₈-cycloalkenyl, C₃-C₈-halocycloalkenyl;
   R²², R²³ H, C₁-C₆-alkyl, C₁-C₆-haloalkyl, C₂-C₆-alkenyl, C₂-C₆-haloalkenyl, C₂-C₆-alkynyl, C₂-C₆-haloalkynyl, C₁-C₆-alkoxy, C₁-C₆-haloalkoxy, which are unsubstituted or partially or fully substituted with R²²¹; or
      C₁-C₆-alkyl-C(=O)OR¹³, C₁-C₆-alkyl-C(=U)N(R^{12a})R^{12b}, C₁-C₆-alkyl-C(=NR¹²)N(R^{12a})R^{12b},
      S(O)ₘR¹³, S(O)ₘN(R^{12a})R^{12b}, C(=U)R¹¹, C(=O)OR¹³, C(=U)N(R^{12a})R^{12b}, C(=S)SR¹³, C(=NR¹²)R¹¹;
      C3-C8-cycloalkyl, C₃-C₈-halocycloalkyl;
      R²²¹ CN, NO₂, OH, SH, SCN, SF₅, Si(C₁-C₄-alkyl)₃, C₁-C₆-alkoxy, C₁-C₆-haloalkoxy, C₁-C₆-alkyl-S(O)ₘ, C₁-C₆-haloalkyl-S(O)ₘ, C(=O)N(R^{12a}₎R^{12b}; C₃-C₈-cycloalkyl which is unsubstituted, partially or fully halogenated and/or partially or fully substituted with C₁-C₄-alkyl, C₃-C₄-cycloalkyl, C₁-C₄-alkoxy, C₁-C₄-haloalkoxy, and oxo; or
      two R²²¹ present on the same carbon atom of an alkyl, alkenyl, alkynyl or cycloalkyl group may together be =O, =CH(C₁-C₄-alkyl), =C(C₁-C₄-alkyl)₂, =N(C₁-C₆-alkyl), or =NO(C₁-C₆-alkyl); or
   R²² and R²³ form together with the nitrogen atom they are bonded to a 3-, 4-, 5-, or 6-membered fully unsaturated heterocycle, which heterocycle may additionally contain one heteroatom selected from N, O, and S(O)ₘ as ring members, and which heterocycle is unsubstituted or partially or fully substituted with R¹⁴; or
   R²² and R²³ together form a group =C(R¹¹)₂, =S(O)ₘ(R¹³)₂, =S(O)ₘR¹³N(R^{12a})R^{12b};
X is NR³, O, or S(O)ₘ;
   R³ is OR²¹, or a group as defined for R⁵;
G is phenyl, or 5- or 6-membered heteroaryl comprising as ring members 1, 2, or 3 heteroatoms selected from N, O and S; wherein G is unsubstituted, partially, or fully substituted with R⁴;
   R⁴ is a group as defined for R²;
U is O or S;
R⁵ is H, C₁-C₆-alkyl, C₁-C₆-haloalkyl, C₂-C₆-alkenyl, C₂-C₆-haloalkenyl, C₂-C₆-alkynyl, C₂-C₆-haloalkynyl, C₁-C₆-alkoxy, C₁-C₆-haloalkoxy, C₁-C₆-alkylthio, C₁-C₆-haloalkylthio, which are unsubstituted or partially or fully substituted with R⁵¹; or
   C₁-C₆-alkyl-C(=O)OR¹³, C₁-C₆-alkyl-C(=U)N(R^{12a})R^{12b}, C₁-C₆-alkyl-C(=NR¹²)N(R^{12a})R^{12b}, N(R^{12a})R^{12b}, S(O)ₘR¹³, S(O)ₘN(R^{12a})R^{12b}, C(=U)R¹¹, C(=O)OR¹³, C(=U)N(R^{12a})R^{12b}, C(=S)SR¹³, C(=NR¹²)R¹¹;
   C₃-C₈-cycloalkyl, C₃-C₈-halocycloalkyl, phenyl, a 3-, 4-, 5-, 6- or 7-membered saturated or partially unsaturated heterocycle comprising 1, 2, 3 or 4 heteroatoms selected from N, O, and S(O)ₘ as ring members, or a 5- or 6-membered hetaryl comprising 1, 2, 3 or 4 heteroatoms selected from N, O, and S(O)ₘ as ring members, which rings are unsubstituted or partially or fully substituted with R⁵²;
   R⁵¹ halogen, CN, NO₂, OH, SH, SCN, SF₅, Si(C₁-C₄-alkyl)₃, N(R^{12a})R^{12b}, C₁-C₆-alkoxy, C₁-C₆-haloalkoxy, C₁-C₆-alkyl-S(O)ₘ, C₁-C₆-haloalkyl-S(O)ₘ, C(=O)N(R^{12a})R^{12b}; C₃-C₈-cycloalkyl which is unsubstituted, partially or fully halogenated and/or partially or fully substituted with CN, C₁-C₄-alkyl, C₃-C₄-cycloalkyl, C₁-C₄-alkoxy, C₁-C₄-haloalkoxy, and oxo;
      N(R^{12a})R^{12b}, S(O)ₘR¹³, S(O)ₘN(R^{12a})R^{12b}, C(=U)R¹¹, C(=O)OR¹³, C(=U)N(R^{12a})R^{12b}, C(=S)SR¹³, C(=NR¹²)R¹¹;
      phenyl, benzyl, phenoxy, or 3-, 4-, 5-, 6- or 7-membered saturated, partially or fully unsaturated heterocycle containing 1, 2, or 3 heteroatoms selected from N, O, S(O)ₘ as ring members, wherein the rings are unsubstituted or partially or fully substituted with R¹⁴; or
   two R⁵¹ present on the same carbon atom of an alkyl, alkenyl, alkynyl or cycloalkyl group may together be =O, =CH(C₁-C₄-alkyl), =C(C₁-C₄-alkyl)₂, =N(C₁-C₆-alkyl), or =NO(C₁-C₆-alkyl);
   R⁵² is a group as defined in R⁵¹ or selected from C₁-C₆-alkyl, C₂-C₆-alkenyl, and C₂-C₆-alkynyl, which groups are unsubstituted, partially or fully halogenated and/or substituted with one or two CN, C₃-C₄-cycloalkyl, C₁-C₄-alkoxy, C₁-C₄-haloalkoxy, or oxo;
R⁶ is a group as defined for R⁵; or
R⁵ and R⁶ form together with the nitrogen atom they are bonded to a 3-, 4-, 5-, 6-, 7- or 8-membered saturated, partially or fully unsaturated heterocycle which heterocycle may additionally contain one or two heteroatoms selected from N, O, and S(O)ₘ as ring members, and which heterocycle is unsubstituted or partially or fully substituted with R¹⁴; or
R⁵ and R⁶ together form a group =C(R¹¹)₂, =S(O)ₘ(R¹³)₂, =S(O)ₘN(R^{12a})R^{12b}, =NR¹², or =NOR¹³; or
R⁵ and R⁶ together with the nitrogen they are bound to form a mesoionic entity;
Y having at G one or two ring atoms of G between connection points of G to X and Y; Y being a direct bond or CR⁷R⁸;
   R⁷, R⁸ are each independently selected from H, halogen, CN, C₁-C₆-alkyl, C₃-C₈-cycloalkyl, C₂-C₆-alkenyl, and C₂-C₆-alkynyl, which groups may be partially or fully halogenated and/or may be substituted by one or more R⁸¹;
      R⁸¹ is CN, N₃, NO₂, SCN, SF₅, C₃-C₈-cycloalkyl, C₃-C₈-halocycloalkyl; Si(C₁-C₄-alkyl)₃, OR¹³, OSO₂R¹³, S(O)ₘR¹³, NR²²R²³, C(=O)NR²²R²³; or
   R⁷ and R⁸, together with the carbon atom they are bonded to, form a 3-, 4-, 5-, 6-, 7- or 8-membered saturated or partially unsaturated carbocycle or heterocycle, wherein the heterocycle comprises 1, 2, 3 or 4 heteroatoms or heteroatom groups independently selected from N, O, and S(O)ₘ as ring members, which rings are unsubstituted or substituted with 1 or 2 groups R¹⁴;
R¹¹ is CN, NO₂, OH, SH, SCN, SF₅, C₁-C₆-alkoxy, C₁-C₆-haloalkoxy, C₁-C₆-alkyl-S(O)ₘ, C₁-C₆-haloalkyl-S(O)ₘ, Si(C₁-C₄-alkyl)₃, C(=O)N(R^{12a})R^{12b},
   C₃-C₈-cycloalkyl which is unsubstituted or partially or fully halogenated and/or substituted with 1 or 2 C₁-C₄-alkyl, C₃-C₄-cycloalkyl, C₁-C₄-alkoxy, C₁-C₄-haloalkoxy, and oxo; phenyl, benzyl, phenoxy, a 3-, 4-, 5-, 6- or 7-membered saturated, partially or fully unsaturated heterocycle containing 1, 2 or 3 heteroatoms N, O, and S(O)ₘ as ring members, which rings are unsubstituted or partially or fully halogenated and/or substituted with R¹⁴; or
two R¹¹ present on the same carbon atom of an alkyl, alkenyl, alkynyl or cycloalkyl may together form =O, =CH(C₁-C₄-alkyl), =C(C₁-C₄-alkyl)C₁-C₄-alkyl, =N(C₁-C₆-alkyl), or =NO(C₁-C₆-alkyl); and
R¹¹ bonded to a cycloalkyl ring is additionally C₁-C₆-alkyl, C₂-C₆-alkenyl, and C₂-C₆-alkynyl, which are unsubstituted, partially or fully halogenated and/or substituted with 1 or 2 groups CN, C₃-C₄-cycloalkyl, C₁-C₄-alkoxy, C₁-C₄-haloalkoxy, and oxo; and
R¹¹ in groups =C(R¹¹)₂, N=C(R¹¹)₂, C(=U)R¹¹, and C(=NR¹²)R¹¹ is additionally selected from H, halogen, C₁-C₆-alkyl, C₂-C₆-alkenyl, and C₂-C₆-alkynyl, which are unsubstituted, partially or fully halogenated and/or substituted with 1 or 2 groups CN, C₃-C₄-cycloalkyl, C₁-C₄-alkoxy, C₁-C₄-haloalkoxy, and oxo;
R¹² is H, C₁-C₆-alkoxy, C₁-C₆-haloalkoxy, S(O)ₘ-C₁-C₄-alkyl, S(O)ₘ-C₁-C₄-haloalkyl, Si(C₁-C₄-alkyl)₃,
   C₁-C₆-alkyl, C₃-C₆-alkenyl, C₃-C₆-alkynyl, which are unsubstituted or partially or fully halogenated and/or substituted with CN, C₁-C₄-alkoxy, C₁-C₄-haloalkoxy, S(O)ₘ-C₁-C₄-alkyl, S(O)ₘ-C₁-C₄-haloalkyl, C₃-C₆-cycloalkyl which is unsubstituted or substituted with 1 or 2 halogen and/or CN; and oxo;
   C₃-C₈-cycloalkyl which is unsubstituted or partially or fully halogenated and/or substituted with CN, C₁-C₄-alkyl, C₁-C₄-alkoxy, C₁-C₄-haloalkoxy, S(O)ₘ-C₁-C₄-alkyl, C₃-C₄-cycloalkyl, C₃-C₄-cycloalkyl-C₁-C₄-alkyl which rings may be substituted with 1 or 2 halogen and/or CN; and oxo;
   phenyl, benzyl, pyridyl, phenoxy, which are unsubstituted or partially or fully halogenated and/or substituted with halogen, CN, NO₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, and (C₁-C₆-alkoxy)carbonyl; and a 3-, 4-, 5- or 6-membered saturated, partially or fully unsaturated heterocycle comprising 1, 2 or 3 heteroatoms selected from N, O, and S(O)ₘ as ring members, where the heterocycle is optionally substituted with one or more R¹⁴;
R^{12a} and R^{12b} have the meanings given for R¹²; or
R^{12a} and R^{12b}, together with the nitrogen atom to which they are bound, form a 3-, 4-, 5-, 6- or 7-membered saturated, partially unsaturated or maximally unsaturated heterocyclic ring, wherein the heterocyclic ring may additionally contain 1 or 2 heteroatoms or heteroatom groups selected from N, O, and S(O)ₘ as ring members, which heterocycle is unsubstituted or substituted with one or more substituents halogen, C₁-C₄-alkyl, C₁-C₄-haloalkyl, C₁-C₄-alkoxy, C₁-C₄-haloalkoxy, and oxo; or
R^{12a} and R¹², together with the nitrogen atoms to which they are bound in the group C(=NR¹²)N(R^{12a})R^{12b}, form a 3-, 4-, 5-, 6- or 7-membered saturated, partially unsaturated or maximally unsaturated heterocyclic ring, wherein the heterocyclic ring may additionally contain 1 or 2 heteroatoms or heteroatom groups selected from N, O, and S(O)ₘ as ring members, which heterocycle is unsubstituted or substituted with one or more substituents halogen, C₁-C₄-alkyl, C₁-C₄-haloalkyl, C₁-C₄-alkoxy, C₁-C₄-haloalkoxy, and oxo;
R¹³ is H, Si(C₁-C₄-alkyl)₃, C₁-C₆-alkyl, C₂-C₆-alkenyl, C₂-C₆-alkynyl, which are unsubstituted or partially or fully halogenated and/or substituted with C₃-C₄-cycloalkyl, C₁-C₄-alkoxy, C₁-C₄-haloalkoxy, S(O)ₘ-C₁-C₄-alkyl, and oxo;
   C₃-C₈-cycloalkyl which is unsubstituted or partially or fully halogenated and/or substituted with C₁-C₄-alkyl, C₃-C₄-cycloalkyl, C₁-C₄-alkoxy, C₁-C₄-haloalkoxy, S(O)ₘ-C₁-C₄-alkyl, and oxo;
   phenyl, benzyl, pyridyl and phenoxy, which are unsubstituted or partially or fully halogenated and/or substituted with C₁-C₆-alkyl, C₁-C₆-haloalkyl, C₁-C₆-alkoxy, C₁-C₆-haloalkoxy, C₁-C₆-alkylthio, C₁-C₆-haloalkylthio, and (C₁-C₆-alkoxy)carbonyl;
R¹⁴ is halogen, NO₂, CN, OH, SH, C₁-C₆-alkoxy, C₁-C₆-haloalkoxy, S(O)ₘ-C₁-C₄-alkyl, S(O)ₘ-C₁-C₄-haloalkyl, C₁-C₄-alkylcarbonyl, C₁-C₄-haloalkylcarbonyl, C₁-C₄-alkoxycarbonyl, C₁-C₄-haloalkoxycarbonyl, C(=O)NR^{12a}R^{12b}, Si(C₁-C₄-alkyl)₃;
   C₁-C₄-alkyl, C₂-C₆-alkenyl, C₂-C₆-alkynyl, which are unsubstituted or partially or fully halogenated and/or substituted with C₃-C₄-cycloalkyl, C₁-C₄-alkoxy, C₁-C₄-haloalkoxy, S(O)ₘ-C₁-C₄-alkyl, and oxo;
   C₃-C₈-cycloalkyl which is unsubstituted or partially or fully halogenated and/or substituted with C₁-C₄-alkyl, C₃-C₄-cycloalkyl, C₁-C₄-alkoxy, C₁-C₄-haloalkoxy, S(O)ₘ-C₁-C₄-alkyl, and oxo;
   phenyl, benzyl, pyridyl and phenoxy, which are unsubstituted or partially or fully halogenated and/or substituted with C₁-C₆-alkyl, C₁-C₆-haloalkyl, C₁-C₆-alkoxy, C₁-C₆-haloalkoxy, C₁-C₆-alkylthio, C₁-C₆-haloalkylthio, and (C₁-C₆-alkoxy)carbonyl; or
two R¹⁴ present together on the same atom of an unsaturated or partially unsaturated ring may be =O, =S, =N(C₁-C₆-alkyl), =NO(C₁-C₆-alkyl), =CH(C₁-C₄-alkyl) or =C(C₁-C₄-alkyl)C₁-C₄-alkyl; or
two R¹⁴ on two adjacent carbon atoms form together with the carbon atoms they are bonded to a 4-, 5-, 6-, 7- or 8-membered saturated, partially unsaturated or maximally unsaturated ring, wherein the ring may contain 1 or 2 heteroatoms or heteroatom groups selected from N, O, and S(O)ₘ as ring members, and wherein the ring is optionally substituted with one or more groups C₁-C₄-alkyl, C₁-C₄-haloalkyl, C₁-C₄-alkoxy, and/or C₁-C₄-haloalkoxy;
m is independently 0, 1 or 2; and
the N-oxides, stereoisomers and agriculturally or veterinarily acceptable salts thereof.

The invention also provides an agricultural composition comprising at least one compound of formula I, a stereoisomer thereof and/or an agriculturally acceptable salt thereof and at least one liquid and/or solid carrier, especially at least one inert liquid and/or solid agriculturally acceptable carrier.

The invention also provides a veterinary composition comprising at least one compound of formula I, a stereoisomer thereof and/or a veterinarily acceptable salt thereof and at least one liquid and/or solid carrier, especially at least one inert veterinarily liquid and/or solid acceptable carrier.

The invention also provides a method for controlling invertebrate pests which method comprises treating the pests, their food supply, their habitat or their breeding ground or a cultivated plant, plant propagation materials (such as seed), soil, area, material or environment in which the pests are growing or may grow, or the materials, cultivated plants, plant propagation materials (such as seed), soils, surfaces or spaces to be protected from pest attack or infestation with a pesticidally effective amount of a compound of formula I or a salt thereof as defined herein. The present invention also relates to plant propagation material, in particular seed, comprising at least one compound of formula I and/or an agriculturally acceptable salt thereof.

The invention further relates to a method for treating or protecting an animal from infestation or infection by parasites which comprises bringing the animal in contact with a parasiticidally effective amount of a compound of formula I or a veterinarily acceptable salt thereof. Bringing the animal in contact with the compound I, its salt or the veterinary composition of the invention means applying or administering it to the animal.

WO 2010/020522 and WO 2010/135360 describe structurally closely related active compounds. These compounds are mentioned to be useful for combating invertebrate pests.

Nevertheless, there remains a need for highly effective and versatile agents for combating invertebrate pests. It is therefore an object of the present invention to provide compounds having a good pesticidal activity and showing a broad activity spectrum against a large number of different invertebrate pests, especially against difficult to control pests, such as insects.

It has been found that these objects can be achieved by compounds of formula I as depicted and defined below, and by their stereoisomers, salts, tautomers and N-oxides, in particular their agriculturally acceptable salts.

Compounds of formula I wherein U is O (formula I.O) can be prepared by reacting an activated carboxylic acid derivative of formula II or the corresponding carboxylic acid Ila with a compound of formula III in an amidation reaction. R in formula II denotes a leaving group, preferably halogen such as e.g. Cl or Br, or C₁-C₆-alkoxy such as OCH₃ or OC₂H₅. Amine III is preferably used as its ammonium salt ⁺HNR⁵R⁶ A⁻, wherein A is an anion, preferably a halogenide such as Cl or Br.

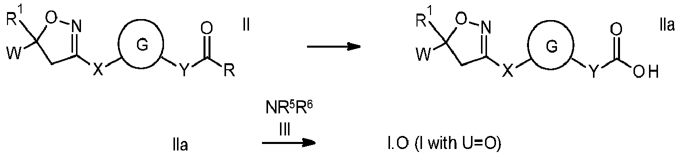

The saponification is carried out under conditions generally known in the art, e.g. dissolving the compound II in either tetrahydrofuran (THF), methanol or water or a mixture of the solvents, and adding alkali metal hydroxides, such as LiOH, NaOH, and KOH either as solid or in solution to the mixture. The reaction may proceed at room temperature (20-25°C) or at elevated temperatures. Workup in a customary manner by aqueous extraction typically yields the compound Ila [cf. WO 2013/032804].

The amidation of compounds II is usually carried out with the acid chlorides or by prior transformation of carboxylic acids of formula IIa with oxalyl chloride [(COCl)₂] or thionylchloride (SOCl₂) to the corresponding acid chlorides, followed by reaction with the amine of formula III. Suitable reaction conditions are described in literature, e.g. in WO 2004/22536. The reaction is generally carried out in the presence of an organic base such as triethylamine (Et₃N), N,N-diisopropylethylamine (iPr₂NEt), pyridine, substituted pyridines such as collidine or lutidine, or the like. Optionally a nucleophilic catalyst such as 4-(N,N-dimethylamino)pyridine (DMAP) can be employed in the reaction. Suitable solvents are halogenated hydrocarbons such as, dichloromethane, chloroform, and chlorobenzene, or polar aprotic solvents such as THF, and N,N-dimethylformamide (DMF), or aromatic hydrocarbons such as benzene, toluene, o-, m-, and p-xylene, or mixtures thereof. The transformation is usually carried out at temperatures from -40°C to 120°C, preferably from 0°C to 40°C. The starting materials are generally reacted with one another in equimolar amounts. In terms of yield, it may be advantageous to employ an excess of III, based on II.

Alternatively, amidation of a carboxylic acid IIa is carried out in the presence of a coupling reagent. Suitable coupling reagents (activators) are known and are, e.g. selected from carbodiimides, such as N,N-dicyclohexylcarbodiimide (DCC) and N,N-diisopropylcarbodiimide (DCI), benzotriazole derivatives such as 1-[bis(dimethylami¬no)methylene]-1H-1,2,3-triazolo[4,5-b]pyridinium 3-oxid hexafluorophosphate (HATU), O-(benzotriazol-1-yl)-N,N,N',N'-tetramethyluronium hexafluorophosphate (HBTU), and 1-[bis(dimethylamino)methylen]-5-chlorobenzotriazolium 3-oxide hex-afluorophosphate (HCTU), or phosphonium-derived activators, such as 2,4,6-Tripropyl-1,3,5,2λ5,4λ5,6λ5-trioxatriphosphinane 2,4,6-trioxide (T3P), (Benzotriazol-1-yl-oxy)tris(dimethylamino)phosphonium hexafluorophosphate (BOP), (benzotriazol-1-yl-oxytripyrrolidinophosphonium hexafluorophosphate) (Py-BOP), bromotripyrrolidinophosphonium hexafluorophosphate (Py-BrOP). Generally, the activator is used in excess. The benzotriazole and phosphonium coupling reagents are generally used in a basic medium. Preferably, 2,4,6-tripropyl-1,3,5,2λ5,4λ5,6λ5-trioxatriphosphinane 2,4,6-trioxide (T3P) is used as the coupling reagent (activator). Suitable reaction conditions are described in the literature, e.g. in WO2015/128358. The reaction is generally carried out in the presence of a base such as a tertiary amine base like iPr₂NEt, Et₃N. Suitable solvents are e.g. halogenated hydrocarbons such as dichloromethane, chloroform, and chlorobenzene. The transformation is usually carried out at temperatures from 0°C to 160°C, preferably from 25°C to 100°C. The starting materials are generally reacted with one another in equimolar amounts. In terms of yield, it may be advantageous to employ an excess of III, based on II. Amines of formula III are commercially available or can be obtained by methods known in the art (e.g. WO 2016/168056, WO 2016/168058, WO 2016/168059, WO 2018/071327, WO 2019/194982, WO 2011/067272, WO 2005/085216).

Compounds II with X being NR³ (formula II.1) can be prepared by reaction of an isoxazole amine of formula IIIA with a halogenide of formula IV, wherein X^{H} is a halogen, preferably a bromide or iodide, in a Buchwald-Hartwig reaction (e.g. WO 2017/069980).

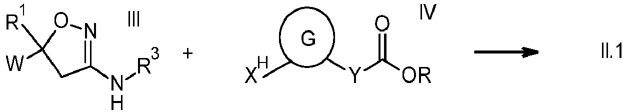

This transformation is usually carried out at temperatures of from 20°C to 180°C, preferably from 60°C to 100°C, in an inert solvent, in the presence of a base and a palladium catalyst such as, e.g., tris(dibenzylideneacetone)dipalladium(0) [Pd₂(dba)₃], 9,9-dimethyl-4,5-bis(diphenyl-phosphino)xanthene (XantPhos), or [2-(di-tert-butylphosphino)-2',4',6'-triisopropyl-1,1'-biphenyl][2-(2-aminoethyl)-phenyl)]palladium(II) chlorid (tBuXPhos Pd G1) [cf. WO 2017/069980]. Suitable solvents are ethers such as dioxane and THF, and the like, or alcohols such as butanol and tert-amyl alcohol, and the like, or polar aprotic solvents such as DMF, dimethylacetamide (DMA), N-methyl-2-pyrrolidon (NMP), and dimethyl sulfoxide (DMSO), and the like.

Preferably, 1,4-dioxane is employed. It is also possible to use mixtures of the solvents mentioned.

Suitable bases are, in general, inorganic compounds, such as alkali metal and alkaline earth metal carbonates, such as Li₂CO₃, K₂CO₃, CaCO₃, and CsCO₃, or organic compounds, such as alkali metal alcoholates, such as lithium tert-butylate, sodium tert-butylate, and potassium tert-butylate, and the like. Particular preference is given to CsCO₃. The bases are generally employed in equimolar amounts; however, they can also be used in in excess.

The starting materials are generally reacted with one another in equimolar amounts. In terms of yield, it may be advantageous to employ an excess of III, based on IV.

The compounds IV are commercially available or known from the literature [cf. e.g. US2011/0251247; THL 1997, 38, 1559] or can be prepared in accordance with the literature cited.

Compounds IIIA, wherein R³ is H, can be prepared by oxo-Michael addition of hydroxyl amine, hydroxyurea, or a salt of either one of them (collectively compounds VA), to an acrylonitrile of formula V.

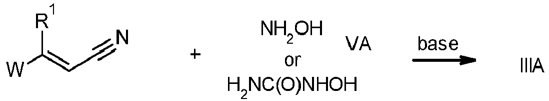

The reaction of an acrylonitrile of formula V with a compound VA is usually carried out at temperatures of from -30°C to 120°C, preferably from 25°C to 80°C, in an inert solvent, in the presence of a base.

Suitable solvents are alcohols such as methanol, ethanol, n-propanol, isopropanol, n-butanol, tert-butanol, and the like, or halogenated hydrocarbons such as methylene chloride, chloroform, chlorobenzene, and the like, or aromatic hydrocarbons such as toluene, o-, m-, p-xylene, and the like, or ethers such as diethyl ether, methyl tert-butyl ether (MTBE), dioxane, anisole, and THF, or nitriles such as acetonitrile, and propionitrile. Moreover, DMSO, DMF, DMA, and water.

Preferably, alcohols such as methanol are used, and it is also possible to use mixtures of the solvents mentioned.

Suitable bases are, in general, inorganic compounds, such as alkali metal and alkaline earth metal hydroxides, such as LiOH, NaOH, KOH, Ca(OH)₂, and the like, or alkali metal and alkaline earth metal carbonates, such as Li₂CO₃, K₂CO₃, and CaCO₃, and the like, or alkali metal and alkaline earth metal hydrides, such as LiH, NaH, KH, CaH₂, and the like. Moreover, organic bases, e.g. alkali metal alcoholates, such as sodium methoxide, sodium ethoxide, lithium tert-butoxide, sodium tert-butoxide, potassium tert-butoxide, and the like, or alkali metal amide bases, such as lithium bis(trimethylsilyl)amide, sodium bis(trimethylsilyl)amide, potassium bis(trimethylsilyl)amide, and the like. Particular preference is given to alkali metal alcoholates such as sodium methoxide. The bases are generally employed in equimolar amounts; however, they can also be used in excess or, if appropriate, as solvent.

The starting materials are generally reacted with one another in equimolar amounts. In terms of yield, it may be advantageous to employ an excess of VA, based on V.

Furthermore, in certain cases it may be advantageous to employ a phase transfer catalyst in the reaction, e.g. a quaternary ammonium salt such as tetra-n-butylammonium bromide, tetra-n-butylammonium iodide, cetyl trimethylammonium chloride, cetyl trimethylammonium bromide, and the like. And, if desired, chiral phase transfer catalysts [cf. Org. Process Res. Dev. 2015, 19, 1731-1746; and references therein] can be employed in the reaction, such as cinchona derived quaternary ammonium salts like (R)-[(1S,2S,4S,5R)-1-(acridin-9-ylmethyl)-5-vinyl-quinuclidin-1-ium-2-yl]-(6-methoxy-4-quinolyl)methanol bromide (CAS-No. 466639-23-6; e.g. US 2014/0350261) for the synthesis of enantiomerically enriched compounds III.

Compounds V can be prepared by Horner-Wadsworth-Emmons reaction of a ketone of formula VI with a compound VII.

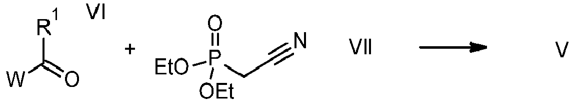

This transformation is usually carried out at temperatures of from 0°C to 120°C, preferably from 25°C to 80°C, in an inert solvent, in the presence of a base [cf. J. Org. Chem. 1985, 50, 15, 2624, and references therein].

Suitable solvents are ethers such as THF, diethyl ether, MTBE, dimethoxy ethane (DME), dioxane and the like, or nitriles such as acetonitrile, propionitrile, and the like, or aromatic hydrocarbons such as benzene, toluene, o-, m-, p-xylene, and the like. Preferably, THF is employed, and it is also possible to use mixtures of the solvents mentioned.

Suitable bases are, in general, inorganic compounds, such as alkali metal and alkaline earth metal hydrides, such as LiH, NaH, KH, CaH₂, and the like.

Moreover, organic bases, e.g. alkali metal amide bases, such as lithium bis(trimethylsilyl)amide, sodium bis(trimethylsilyl)amide, potassium bis(trimethylsilyl)amide, and the like, or alkali metal alcoholates, such as lithium tert-butylate, sodium tert-butylate, and potassium tert-butylate, and the like, or tertiary amines, such as triethylamine, diisopropylethylamine and N-methylpiperidine, or amidine bases such as 1,8-diazabicyclo[5.4.0]undec-7-ene (DBU), and the like. Particular preference is given to triethylamine. The bases are generally employed in equimolar amounts; however, they can also be used in excess or, if appropriate, as solvent.

Furthermore, in certain cases, it may be advantageous to employ an alkali metal or earth alkali metal additive such as LiCI, LiBr, MgCl₂, MgBr₂, and the like, in the reaction.

The starting materials are generally reacted with one another in equimolar amounts. In terms of yield, it may be advantageous to employ an excess of VII, based on VI.

Compounds VI and VII are commercially available or known from the literature [cf. e.g. WO 2005/085216, WO 2011/067272] or can be prepared in accordance with the literature cited.

Alternatively, compounds of formula II.1 can be prepared by reaction of an isoxazoline halogen compound of formula VIII, wherein X^{H} is a halogen, preferably a bromide or chloride, with an amine of formula IXa (X = NHR³) in an acid-promoted ipso-substitution reaction:

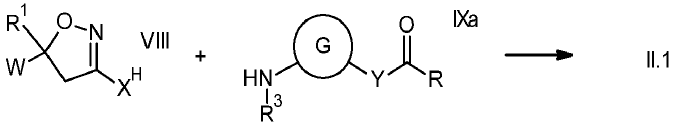

This transformation is usually carried out at temperatures of from 25 °C to 200 °C, preferably from 60 °C to 150 °C, in an inert solvent, in the presence of an acid.

Suitable solvents are alcohols such as 2,4-dimethylpentan-3-ol, n-butanol, sec-butanol, tert-butanol, and the like, or aromatic hydrocarbons such as toluene, o-, m-, p-xylene, chlorobenzene, dichlorobenzene, and the like, moreover, DMSO, DMF, and DMA, NMP, and the like, preferably alcohols such as 2,4-dimethylpentan-3-ol are employed. It is also possible to use mixtures of the solvents mentioned.

Suitable acids and acidic catalysts are in general inorganic acids such as, HCI, HBr, H₂SO₄, HClO₄, and the like, moreover, organic acids such as toluenesulfonic acid, benzenesulfonic acid, camphor sulfonic acid, trifluoromethanesulfonic acid, trifluoroacetic acid, and the like. The acids are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of IXa, based on VIII.

Compounds IX are commercially available or known from the literature [cf. WO 2016/168056, WO 2016/168058, WO 2016/168059, WO 2018/071327, WO 2019/194982, WO 2010/135360] or can be prepared in accordance with the literature cited.

Compounds of formula VIII, on the other hand, can be prepared by reacting an olefin of formula X with a dihaloformaldoxime of formula XI in a 1,3-dipolar cycloaddition reaction:

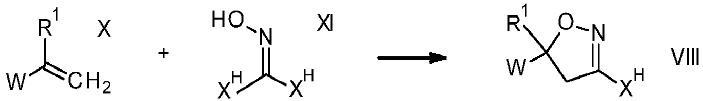

This transformation is usually carried out at temperatures of from 0°C to 100°C, preferably from 25°C to 80°C, in an inert solvent, in the presence of a base [cf. WO 2010/135360]. Suitable solvents are esters such as ethyl acetate, and the like, or water, and it is also possible to use mixtures of the solvents mentioned. Suitable bases are, in general, inorganic compounds, such as alkali metal and alkaline earth metal carbonates, such as Li₂CO₃, K₂CO₃, and CaCO₃, and also alkali metal bicarbonates such as NaHCO₃. Particular preference is given to alkali metal bicarbonates such as NaHCO₃. The bases are generally employed in excess; however, they can also be used in equimolar amounts.

The starting materials are generally reacted with one another in equimolar amounts. In terms of yield, it may be advantageous to employ an excess of XI, based on X.

Compounds of formula X, in turn, can be prepared by reacting a ketone of formula VI with methyltriphenylphosphonium bromide in a Wittig reaction:

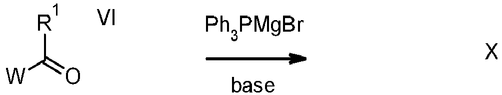

The Wittig reaction is conducted under conditions known in the art, preferably at temperatures of from 0°C to 60°C, in an inert solvent such as THF, in the presence of a base such as potassium tert-butoxide.

In addition, compounds of formula II can also be prepared by reaction of an isoxazoline halogen compound of formula VIII, wherein X^{H} is a halogen, preferably a bromide or chloride, with a compound of formula IX in a base-promoted ipso-substitution reaction:

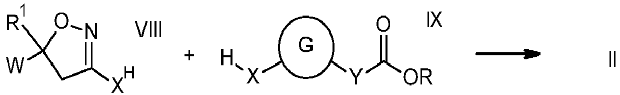

This transformation is usually carried out at temperatures of from 25°C to 200°C, preferably from 60°C to 150°C, in an inert solvent, in the presence of a base [cf. WO 2010/135360].

Suitable solvents are DMF, DMA, NMP, DMSO, and the like, preferably DMF or NMP are used. It is also possible to use mixtures of the solvents mentioned.

Suitable bases are, in general, inorganic compounds, such as alkali metal and alkaline earth metal hydroxides, such as LiOH, NaOH, KOH, Ca(OH)₂, and the like, or alkali metal and alkaline earth metal hydrides, such as LiH, NaH, KH, CaH₂, and the like, or alkali metal and alkaline earth metal carbonates, such as Li₂CO₃, K₂CO₃, and CaCO₃. The bases are generally employed in equimolar amounts; however, they can also be used in in excess or, if appropriate, as solvent.

Furthermore, compounds of formula II can be prepared by reaction of an olefin of formula X, with an oxime of formula XII, wherein Ar is an aromatic group, in a 1,3-dipolar cycloaddition:

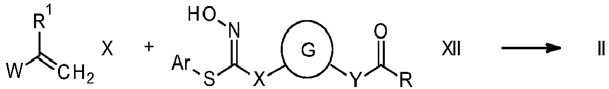

This transformation is usually carried out at temperatures of from 0°C to 100°C, preferably from 25°C to 60°C, in an inert solvent, in the presence of a base and a silver(I) salt, such as e.g. silver nitrate [cf. WO 2010/135360].

Suitable solvents are nitriles such as acetonitrile, propionitrile, and the like. Suitable bases are, in general, inorganic compounds, such as alkali metal and alkaline earth metal carbonates, such as Li₂CO₃, K₂CO₃, and CaCO₃, and also alkali metal bicarbonates, such as NaHCO₃. Particular preference is given to K₂CO₃. The bases are generally employed in equimolar amounts; however, they can also be used in excess or, if appropriate, as solvent.

The starting materials are generally reacted with one another in equimolar amounts. In terms of yield, it may be advantageous to employ an excess of X, based on XII.

Compounds XII can be prepared as described in the literature [cf. WO 2010/135360] or can be prepared in accordance with the literature cited.

Compounds of formula I', that is compounds of formula I wherein U is S, can be prepared by reacting the corresponding oxocompound (U is O) with Lawesson's reagent (CAS 19172-47-5), see, e.g. Jesberger et al. Synthesis, 2003, 1929-1958 and references therein. Solvents such as HMPA or THF at an elevated temperature such as 60°C to 100°C can be used. Preferred reaction conditions are in THF at 65°C.

The starting materials required for preparing the compounds I are commercially available or known from the literature [cf. e.g. WO 2005/085216, WO 2010/135360, WO 2011/067272, WO 2016/168056, WO 2016/168058, WO 2016/168059, WO 2018/071327, WO 2019/194982] or can be prepared in accordance with the literature cited.

The reaction mixtures are worked up in a customary manner, for example by mixing with water, separating the phases and, if appropriate, chromatographic purification of the crude products. Some of the intermediates and end products are obtained in the form of colorless or slightly brownish viscous oils which are purified or freed from volatile components under reduced pressure and at moderately elevated temperature. If the intermediates and end products are obtained as solids, purification can also be carried out by recrystallization or digestion.

If individual compounds I cannot be obtained by the routes described above, they can be prepared by derivatization of other compounds I.

However, if the synthesis yields mixtures of isomers, a separation is generally not necessarily required since in some cases the individual isomers can be interconverted during work-up for use or during application (e.g. under the action of light, acids or bases). Such conversions may also take place after use, e.g. in the treatment of plants in the treated plant, or in the pest to be controlled.

In a preferred embodiment, the compounds I are present in form of a mixture of compounds I.A and I.B, wherein compound I.A with S-configuration in the isoxazoline ring is present in an amount of more than 50% by weight, in particular of at least 70% by weight, more particularly of at least 85% by weight, specifically of at least 90% by weight, based on the total weight of compounds I.A and I.B.

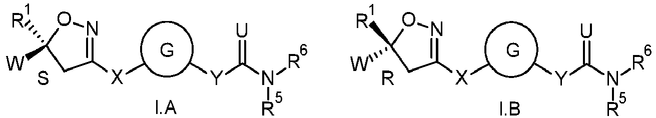

In one particularly preferred embodiment of the invention, the method comprises the step of contacting the plant, parts of it, its propagation material, the pests, their food supply, habitat or breeding grounds a pesticidally effective amount of a compound of formula I.A.

Compounds of formula I.A, and I.B, resp., can be obtained in enantiopure form by known separation methods, preferably by chiral chromatography. This is preferably applied to intermediate compounds of formula III and IIIa, or to compounds of formula II and Ila.

The organic moieties groups mentioned in the above definitions of the variables are - like the term halogen - collective terms for individual listings of the individual group members. The prefix Cn-Cm indicates in each case the possible number of carbon atoms in the group.

The term "partially or fully substituted" by a radical means that in general the group is substituted with same or different radicals.

The term "halogen" denotes in each case fluorine, bromine, chlorine, or iodine, in particular fluo-rine, chlorine, or bromine.

The term "alkyl" as used herein and in the alkyl moieties of alkylamino, alkylcarbonyl, alkylthio, alkylsulfinyl, alkylsulfonyl and alkoxyalkyl denotes in each case a straight-chain or branched alkyl group having usually from 1 to 10 carbon atoms, frequently from 1 to 6 carbon atoms, preferably 1 to 4 carbon atoms, more preferably from 1 to 3 carbon atoms. Examples of an alkyl group are methyl (Me), ethyl (Et), n-propyl (n-Pr), iso-propyl, n-butyl, 2-butyl, iso-butyl, tert-butyl, n-pentyl, 1-me¬thylbutyl, 2 methylbutyl, 3 methylbutyl, 2,2-di¬methylpropyl, 1 ethylpropyl, n-hexyl, 1,1-dimethyl-propyl, 1,2-dimethylpropyl, 1-methylpentyl, 2-methylpentyl, 3-methylpentyl, 4-methylpentyl, 1,1-dimethy¬butyl, 1,2-dimethylbutyl, 1,3-dimethy¬butyl, 2,2-dimethylbutyl, 2,3-dimethylbutyl, 3,3-dimethyl¬butyl, 1-ethylbutyl, 2-ethylbutyl, 1,1,2-trimethylpropyl, 1,2,2-trimethylpropyl, 1-ethyl-1-methyl¬propyl, and 1-ethyl-2-methylpropyl.

The term "haloalkyl" as used herein and in the haloalkyl moieties of haloalkylcarbonyl, haloalkoxycarbonyl, haloalkylthio, haloalkylsulfonyl, haloalkylsulfinyl, haloalkoxy and haloalkoxyalkyl, denotes in each case a straight-chain or branched alkyl group having usually from 1 to 10 carbon atoms, frequently from 1 to 6 carbon atoms, preferably from 1 to 4 carbon atoms, wherein the hydrogen atoms of this group are partially or totally replaced with halogen atoms. Preferred haloalkyl moieties are selected from C₁-C₄-halo¬alkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2 difluoroethyl, 2,2,2-trifluoroethyl, pentafluoroethyl, and the like.

The term "alkoxy" as used herein denotes in each case a straight-chain or branched alkyl group which is bonded via an oxygen atom and has usually from 1 to 10 carbon atoms, frequently from 1 to 6 carbon atoms, preferably 1 to 4 carbon atoms. Examples of an alkoxy group are methoxy, ethoxy, n-propoxy, iso-prop¬oxy, n-butyloxy, 2-butyloxy, iso-butyloxy, tert.-butyloxy, and the like.

The term "alkoxyalkyl" as used herein refers to alkyl usually comprising 1 to 10, frequently 1 to 4, preferably 1 to 2 carbon atoms, wherein 1 carbon atom carries an alkoxy radical usually comprising 1 to 4, preferably 1 or 2 carbon atoms as defined above. Examples are CH₂OCH₃, CH₂-OC₂H₅, 2-(methoxy)ethyl, and 2-(ethoxy)ethyl.

The term "haloalkoxy" as used herein denotes in each case a straight-chain or branched alkoxy group having from 1 to 10 carbon atoms, frequently from 1 to 6 carbon atoms, preferably 1 to 4 carbon atoms, wherein the hydrogen atoms of this group are partially or totally replaced with halogen atoms, in particular fluorine atoms. Preferred haloalkoxy moieties include C₁-C₄-haloalkoxy, in particular C₁-C₂-fluoroalkoxy, such as fluoromethoxy, difluoromethoxy, trifluoromethoxy, 1 fluoroethoxy, 2-fluoroethoxy, 2,2 difluoroethoxy, 2,2,2-trifluoroethoxy, 2-chloro-2-fluoroethoxy, 2-chloro-2,2-difluoro-ethoxy, 2,2dichloro-2-fluorethoxy, 2,2,2-trichloroethoxy, penta¬fluoroethoxy and the like.

The term "alkylthio "(alkylsulfanyl: alkyl-S-)" as used herein refers to a straight-chain or branched saturated alkyl group having 1 to 10 carbon atoms, preferably 1 to 4 carbon atoms (= C₁-C₄-alkylthio), more preferably 1 to 3 carbon atoms, which is attached via a sulfur atom.

The term "haloalkylthio" as used herein refers to an alkylthio group as mentioned above wherein the hydrogen atoms are partially or fully substituted by fluorine, chlorine, bromine and/or iodine.

The term "alkylsulfinyl" (alkylsulfoxyl: C₁-C₆-alkyl-S(O)-), as used herein refers to a straight-chain or branched saturated alkyl group (as mentioned above) having 1 to 10 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "haloalkylsulfinyl" as used herein refers to an alkylsulfinyl group as mentioned above wherein the hydrogen atoms are partially or fully substituted by fluorine, chlorine, bromine and/or iodine.

The term "alkylsulfonyl" (alkyl-S(O)₂-) as used herein refers to a straight-chain or branched saturated alkyl group having 1 to 10 carbon atoms, preferably 1 to 4 carbon atoms (= C₁-C₄-alkylsulfonyl), preferably 1 to 3 carbon atoms, which is bonded via the sulfur atom of the sulfonyl group at any position in the alkyl group.

The term "haloalkylsulfonyl" as used herein refers to an alkylsulfonyl group as mentioned above wherein the hydrogen atoms are partially or fully substituted by fluorine, chlorine, bromine and/or iodine.

The term "alkylcarbonyl" refers to an alkyl group as defined above, which is bonded via the car-bon atom of a carbonyl group (C=O) to the remainder of the molecule.

The term "haloalkylcarbonyl" refers to an alkylcarbonyl group as mentioned above, wherein the hydrogen atoms are partially or fully substituted by fluorine, chlorine, bromine and/or iodine. The term "alkoxycarbonyl" refers to an alkylcarbonyl group as defined above, which is bonded via an oxygen atom to the remainder of the molecule.

The term "haloalkoxycarbonyl" refers to an alkoxycarbonyl group as mentioned above, wherein the hydrogen atoms are partially or fully substituted by fluorine, chlorine, bromine and/or iodine.

The term "alkenyl" as used herein denotes in each case a singly unsaturated hydrocarbon radical having usually 2 to 10, frequently 2 to 6, preferably 2 to 4 carbon atoms, e.g. vinyl, allyl (2-propen-1-yl), 1-propen-1-yl, 2 propen-2-yl, methallyl (2-methylprop-2-en-1-yl), 2-buten-1-yl, 3-buten-1-yl, 2-penten-1-yl, 3-penten-1-yl, 4-penten-1-yl, 1-methylbut-2-en-1-yl, 2-ethylprop-2-en-1-yl and the like.

The term "haloalkenyl" as used herein refers to an alkenyl group as defined above, wherein the hydrogen atoms are partially or totally replaced with halogen atoms.

The term "alkynyl" as used herein denotes in each case a singly unsaturated hydrocarbon radi-cal having usually 2 to 10, frequently 2 to 6, preferably 2 to 4 carbon atoms, e.g. ethynyl, propar-gyl (2-propyn-1-yl), 1-propyn-1-yl, 1-methylprop-2-yn-1-yl), 2-butyn-1-yl, 3-butyn-1-yl, 1-pentyn-1-yl, 3-pentyn-1-yl, 4-pentyn-1-yl, 1-methylbut-2-yn-1-yl, 1-ethylprop-2-yn-1-yl and the like.

The term "haloalkynyl" as used herein refers to an alkynyl group as defined above, wherein the hydrogen atoms are partially or totally replaced with halogen atoms.

The term "cycloalkyl" as used herein and in the cycloalkyl moieties of cycloalkoxy and cycloalkylthio denotes in each case a monocyclic cycloaliphatic radical having usually from 3 to 10 or from 3 to 6 carbon atoms, such as cyclopropyl (c-C₃H₅), cyclobutyl, cyclopentyl, cyclohexyl, cycloheptyl, cyclooctyl, cyclononyl and cyclodecyl or cyclopropyl, cyclobutyl, cyclopentyl and cyclo-hexyl.

The term "halocycloalkyl" as used herein and in the halocycloalkyl moieties of halocycloalkoxy and halocycloalkylthio denotes in each case a monocyclic cycloaliphatic radical having usually from 3 to 10 C atoms or 3 to 6 C atoms, wherein at least one, e.g. 1, 2, 3, 4 or 5 of the hydrogen atoms, are replaced by halogen, in particular by fluorine or chlorine. Examples are 1- and 2-fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "halocycloalkenyl" as used herein and in the halocycloalkenyl moieties of halocycloalkenyloxy and halocycloalkenylthio denotes in each case a monocyclic singly unsaturated non-aromatic radical having usually from 3 to 10, e.g. 3 or 4 or from 5 to 10 carbon atoms, preferably from 3- to 8 carbon atoms, wherein at least one, e.g. 1, 2, 3, 4 or 5 of the hydrogen atoms, are replaced by halogen, in particular by fluorine or chlorine. Examples are 3,3-difluorocyclopropen-1-yl and 3,3-dichlorocyclopropen-1-yl.

The term "cycloalkenylalkyl" refers to a cycloalkenyl group as defined above which is bonded via an alkyl group, such as a C₁-C₅-alkyl group or a C₁-C₄-alkyl group, in particular a methyl group (= cycloalkenylmethyl), to the remainder of the molecule.

The term "carbocycle" or "carbocyclyl" includes in general a 3- to 12-membered, preferably a 3- to 8-membered or a 5- to 8-membered, more preferably a 5- or 6-membered monocyclic, non-aromatic ring comprising 3 to 12, preferably 3 to 8 or 5 to 8, more preferably 5 or 6 carbon atoms. Preferably, the term "carbocycle" covers cycloalkyl and cycloalkenyl groups as defined above.

The term "heterocycle" or "heterocyclyl" includes in general 3- to 12-membered, preferably 3-to 6-membered, in particular 6-membered monocyclic heterocyclic non-aromatic radicals. The heterocyclic non-aromatic radicals usually comprise 1, 2, 3, 4 or 5, preferably 1, 2 or 3 hetero¬atoms selected from N, O and S as ring members, wherein S-atoms as ring members may be present as S, SO or SO₂. Examples of 5- or 6-membered heterocyclic radicals comprise satu¬rated or unsaturated, non-aromatic heterocyclic rings, such as oxiranyl, oxetanyl, thietanyl, thietanyl-S-oxid (S-oxothietanyl), thietanyl-S-dioxid (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxo¬thiolanyl, dihydrothienyl, S-oxodihydrothienyl, S-dioxodihydro¬thienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S.oxothiopyranyl, S-dioxothiopyranyl, dihydrothio¬pyranyl, S-oxo¬dihydrothiopyranyl, S-dioxodihydrothiopyranyl, tetrahydrothiopyranyl, S-oxotetra¬hydrothiopyranyl, S-dioxotetrahydrothiopyranyl, morpholinyl, thiomorpholinyl, S-oxothio¬morpho¬linyl, S-dioxothiomorpholinyl, thiazinyl and the like. Examples for heterocyclic ring also comprising 1 or 2 carbonyl groups as ring members comprise pyrrolidin-2-onyl, pyrrolidin-2,5-dionyl, imidazolidin-2-onyl, oxazolidin-2-onyl, thiazolidin-2-only, and the like.

The term "hetaryl" includes monocyclic 5- or 6-membered heteroaromatic radicals comprising as ring members 1, 2, 3 or 4 heteroatoms selected from N, O and S. N- or S-containing hetaryl groups may be present as positively charged onium, and form together with a neighbouring atom a mesoionic entity. Examples of 5- or 6 membered heteroaromatic radicals include pyridyl, i.e. 2-, 3-, or 4 pyridyl, pyrimidinyl, i.e. 2 , 4- or 5-pyrimidinyl, pyrazinyl, pyridazinyl, i.e. 3- or 4 pyridazinyl, thienyl, i.e. 2- or 3-thienyl, furyl, i.e. 2-or 3-furyl, pyrrolyl, i.e. 2- or 3 pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5 isothiazolyl, pyrazolyl, i.e. 1-, 3-, 4- or 5-pyrazolyl, i.e. 1-, 2-, 4- or 5-imidazolyl, oxadiazolyl, e.g. 2- or 5 [1,3,4]oxadiazolyl, 4- or 5-(1,2,3-oxa¬diazol)yl, 3- or 5-(1,2,4-oxadiazol)yl, 2- or 5 (1,3,4-thiadiazol)yl, thiadiazolyl, e.g. 2- or 5-(1,3,4-thia¬diazol)yl, 4- or 5 (1,2,3 thiadiazol)yl, 3- or 5-(1,2,4-thiadiazol)yl, triazolyl, e.g. 1H-, 2H- or 3H 1,2,3 triazol-4-yl, 2H-triazol-3-yl, 1H-, 2H-, or 4H-1,2,4-triazolyl and tetrazolyl, i.e. 1H- or 2H tetrazolyl. The term "hetaryl" also includes bicyclic 8 to 10-membered heteroaromatic radicals comprising as ring members 1, 2 or 3 heteroatoms selected from N, O and S, wherein a 5- or 6-membered heteroaromatic ring is fused to a phenyl ring or to a 5- or 6-membered heteroaromatic radical. Examples of a 5- or 6-membered heteroaromatic ring fused to a phenyl ring or to a 5- or 6-membered heteroaromatic radical include benzofuranyl, benzo¬thienyl, indolyl, ind¬azolyl, benzimidazolyl, benzoxathiazolyl, benzoxadiazolyl, benzothiadiazolyl, benzoxazinyl, chinolinyl, iso-chinolinyl, purinyl, 1,8-naphthyridyl, pteridyl, pyrido[3,2 d]pyrimidyl or pyridoimidazolyl and the like. These fused hetaryl radicals may be bonded to the remainder of the molecule via any ring atom of 5- or 6-membered heteroaromatic ring or via a carbon atom of the fused phenyl moiety. The terms "heterocyclylalkyl" and "hetarylalkyl" refer to heterocyclyl or hetaryl, respectively, as defined above which are bonded via a C₁-C₅-alkyl group or a C₁-C₄-alkyl group, in particular a methyl group (= heterocyclylmethyl or hetarylmethyl, respectively), to the remainder of the molecule.

The term "arylalkyl" and "phenylalkyl" refer to aryl as defined above and phenyl, respectively, which are bonded via C₁-C₅-alkyl group or a C₁-C₄-alkyl group, in particular a methyl group (= arylmethyl or phenylmethyl), to the remainder of the molecule, examples including benzyl, 1-phenylethyl, 2-phenylethyl, 2-phenoxyethyl etc.

The terms "alkylene", "cycloalkylene", "heterocycloalkylene", "alkenylene", "cycloalkenylene", "heterocycloalkenylene" and "alkynylene" refer to alkyl, cycloalkyl, heterocycloalkyl, alkenyl, cycloalkenyl, heterocycloalkenyl and alkynyl as defined above, respectively, which are bonded to the remainder of the molecule, via two atoms, preferably via two carbon atoms, of the respective group, so that they represent a linker between two moieties of the molecule.

In a particular embodiment, the variables of the compounds of the formula I have the following meanings, these meanings, both on their own and in combination with one another, being particular embodiments of the compounds of the formula I.

Embodiments and preferred compounds of the invention for use in pesticidal methods and for insecticidal application purposes are outlined in the following paragraphs.

With respect to the variables, the particularly preferred embodiments of the intermediates correspond to those of the compounds of the formula I.

In one embodiment W is a group WA, 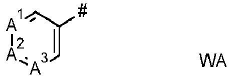 wherein
A¹,A²,A³ are N or CR², provided that at least two are CR²;
R² are independently from each other preferably H, halogen, C₁-C₄-haloalkyl, C₁-C₄-halocycloalkyl, C₁-C₃-haloalkoxy, S(O)ₘ-C₁-C₃-alkyl, S(O)ₘ-C₁-C₃-haloalkyl.
W is preferably a group WP: 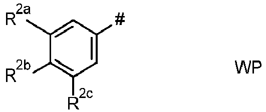
   wherein R^{2a}, R^{2b}, and R^{2c} are a group R².
   R^{2a} is preferably selected from F, Cl, Br, CF₃, SCF₃, and OCF₃.
   R^{2b} and R^{2c} are independently preferably selected from H, F, Cl, Br, CF₃, SCF₃, and OCF₃.

Particularly preferred is each one of the following combinations of R^{2a}, R^{2b} and R^{2c} wherein each line of Table W denotes a substitution pattern of the phenyl ring ("WP") bearing the R^{2a}, R^{2b} and R^{2c} moieties.

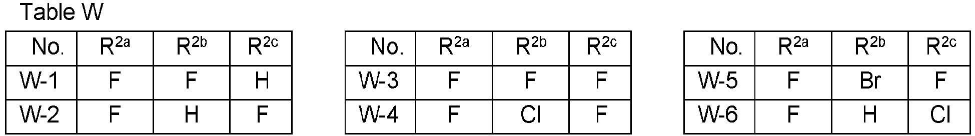

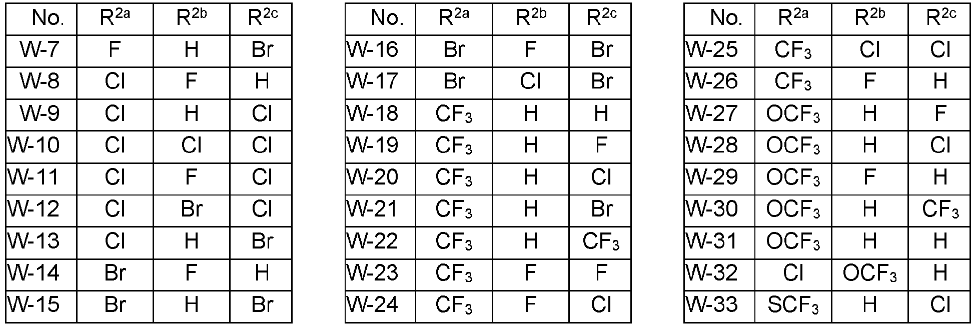

Groups W-8, W-9, and W-11 are more preferred patterns in formula I compounds. W-11 is particularly preferred.

Another embodiment of W is a 5- or 6-membered hetaryl, such as thiophene, pyrazole, imidazole, or pyridine, which rings are substituted with 1 or 2 groups R^{2a} or C₁-C₂-alkoxy-C₁-C₂-alkyl.

R¹ is preferably CF₃.

X is preferably N-R³.

R³ is preferably H, C₁-C₄-alkyl, unsubstituted or substituted with 1 or 2 groups CN, C₃-C₄-cycloalkyl, C₁-C₂-alkoxy, 4-membered herocyclyl comprising as ring members a hetero¬atom selected from N, O, and S(O)ₘ. R³ being H or CH₃ is particularly preferred.

In another embodiment R³ is OH, C₁-C₄-alkoxy, or C₁-C₄-alkoxycarbonyl.

In one embodiment G is a group GQ: 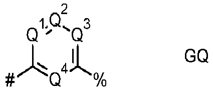 wherein
Q¹,Q²,Q³,Q⁴ are N or CR⁴; provided that at least three are CR⁴;
R⁴ are independently from each other preferably H, halogen, CN, C₁-C₄-alkyl, C₁-C₄-cycloalkyl, C₁-C₄-haloalkyl, C₁-C₄-halocycloalkyl, C₁-C₃-alkoxy, C₁-C₃-haloalkoxy, S(O)ₘ-C₁-C₃-alkyl, S(O)ₘ-C₁-C₃-haloalkyl.
G is preferably a group G1, G2, G3, or G4: 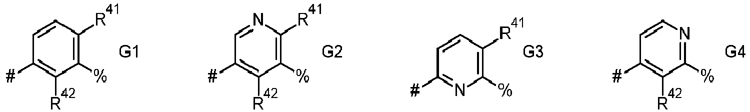 wherein # is the bond to X, % is the bond to Y which is a direct bond, and R⁴¹ and R⁴² are H or a group R⁴, preferably H, or halogen, CN, halomethyl, halomethoxy. In a particularly preferred embodiment R⁴¹ is halogen such as Cl, and R⁴² is H.

Another embodiment of G is a 5-membered hetaryl, such as thiophene, or imidazole, which rings are substituted with a group R^{2a} or C₁-C₂-alkoxy.

Y is preferably a direct bond.

In another embodiment Y is a spacer CR⁷R⁸. In such group R⁷ and R⁸ are preferably both H or halogen such as F, or both R⁷ and R⁸ together form a 3- or 4-membered saturated carbocycle.

Preferably R⁵ is C₁-C₄-alkyl, C₁-C₄-haloalkyl, C₃-C₄-cycloalkyl optionally substituted, phenyl substituted with 1 or 2 halogen, NH₂, NHC(O)-C₁-C₄-alkyl, 6-membered hetaryl comprising as ring members 1-3 N atoms.

In another embodiment R⁵ is cyclopropyl which is substituted with CN, C₁-C₂-alkyl, C₁-C₂-haloalkyl, or 3-, 4-, 5-, or 6-membered partially or fully unsubstituted heterocycle comprising as ring members 1 to 4 hetero¬atoms selected from N, O, and S(O)ₘ which heterocycle is optionally substituted with halogen, CN, C₁-C₄-alkyl, cyano-C₁-C₄-alkyl, C₁-C₄-haloalkyl, C₁-C₂-alkoxy-C₁-C₂-alkyl, and/or oxo.

In another embodiment R⁵ is phenyl which is substituted with 1 or 2 substituents halogen, CN, C₁-C₄-alkyl, C₁-C₄-haloalkyl, NH₂, NHC(O)-C₁-C₄-alkyl, NHC(O)-C₁-C₄-haloalkyl, NHC(O)-C₁-C₄-alkoxy, NHC(O)-C₁-C₂-alkyl-C₃-C₄-cycloalkyl

R⁶ is preferably H or C₁-C₄-alkyl, C₃-C₄-cycloalkyl-C₁-C₂-alkyl, C₁-C₄-alkylcarbonyl, or C₁-C₄-alkoxycarbonyl. Particularly preferred is R⁶ is H or C₁-C₂-alkyl.

A preferred embodiment are compounds of formula I, wherein
R¹ is C₁-C₂-haloalkyl;
W is phenyl, or 5- or 6-membered heteroaryl comprising as ring members 1, 2, or 3 heteroatoms selected from N, O, and S; wherein W is unsubstituted, partially or fully substituted with R²;
   R² is halogen, OR²¹, C₁-C₄-haloalkyl, C₁-C₃-alkoxy, C₁-C₃-haloalkoxy, C₁-C₃-alkyl-S(O)ₘ, C₁-C₃-haloalkyl-S(O)ₘ, C₁-C₃-alkoxy-C₁-C₄-alkyl; which groups are optionally substituted with R²¹¹; m is 0, 1, or 2;
   R²¹ H, C₁-C₆-alkyl, which groups are unsubstituted, partially or fully substituted with R²¹¹; R²¹¹ halogen;
X is NR³, O, or S(O)ₘ; R³ is OR²¹ or a group as defined for R⁵;
G is phenyl, or 5- or 6-membered heteroaryl comprising as ring members 1, 2, or 3 heteroatoms selected from N, O and S; wherein G is unsubstituted, partially, or fully substituted with R⁴; R⁴ is a group as defined for R²;
U is O;
R⁵ is H, C₁-C₆-alkyl, C₁-C₆-haloalkyl, which are unsubstituted or partially or fully substituted with R⁵¹; or
   N(R^{12a})R^{12b}, S(O)ₘR¹³, C(=O)OR¹³, C(=U)N(R^{12a})R^{12b},
   C₃-C₈-cycloalkyl, C₃-C₈-halocycloalkyl, phenyl, a 3-, 4-, 5-, 6- or 7-membered saturated or partially unsaturated heterocycle comprising 1, 2, 3 or 4 heteroatoms selected from N, O, and S(O)ₘ as ring members, or a 5- or 6-membered hetaryl comprising 1, 2, 3 or 4 heteroatoms selected from N, O, and S(O)ₘ as ring members, which rings are unsubstituted or partially or fully substituted with R⁵²;
   R⁵¹ halogen, CN, N(R^{12a})R^{12b}, C(=O)N(R^{12a})R^{12b};
      C₃-C₈-cycloalkyl which is unsubstituted, partially halogenated and/or substituted with CN;
      S(O)ₘR¹³, C(=U)N(R^{12a})R^{12b}; phenyl, or 3-, 4-, 5-, 6- or 7-membered saturated, partially or fully unsaturated heterocycle containing 1, 2, or 3 heteroatoms selected from N, O, S(O)ₘ as ring members; or
   R⁵² is a group as defined in R⁵¹;
R⁶ is a group as defined for R⁵; or
R⁵ and R⁶ together with the nitrogen they are bound to form a mesoionic entity;
Y having at G one or two ring atoms of G between connection points of G to X and Y; Y being a direct bond or CR⁷R⁸; R⁷, R⁸ are H, or halogen.

A preferred embodiment are compounds of formula I which correspond to formula I' as depicted below, wherein the variables are defined and preferred as above.

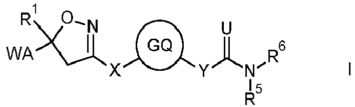

Another preferred embodiment are compounds of formula I which correspond to formula I.1 as depicted below, wherein the variables are defined and preferred as above.

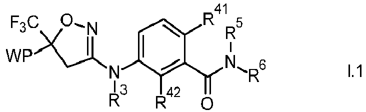

In particular with a view to their use, preference is given to the compounds of formula I compiled in the tables below, which compounds correspond to formula I.1 wherein R⁴² denotes H. Each of the groups mentioned for a substituent in the tables is furthermore per se, independently of the combination in which it is mentioned, a particularly preferred aspect of the substituent in question.
Table 1: Compounds of formula I.1 in which R⁵ is H, R⁶ is c-C₃H₅, and the other variables for a compound correspond in each case to one row of Table A
Table 2: Compounds of formula I.1 in which R⁵ is CH₃, R⁶ is c-C₃H₅, and the other variables for a compound correspond in each case to one row of Table A
Table 3: Compounds of formula I.1 in which R⁵ is CH₂CH₃, R⁶ is c-C₃H₅, and the other variables for a compound correspond in each case to one row of Table A
Table 4: Compounds of formula I.1 in which R⁵ is H, R⁶ is (1-CN)-c-C₃H₄, and the other variables for a compound correspond in each case to one row of Table A
Table 5: Compounds of formula I.1 in which R⁵ is CH₃, R⁶ is (1-CN)-c-C₃H₄, and the other variables for a compound correspond in each case to one row of Table A
Table 6: Compounds of formula I.1 in which R⁵ is CH₂CH₃, R⁶ is (1-CN)-c-C₃H₄, and the other variables for a compound correspond in each case to one row of Table A
Table 7: Compounds of formula I.1 in which R⁵ is H, R⁶ is CH₂CF₃, and the other variables for a compound correspond in each case to one row of Table A
Table 8: Compounds of formula I.1 in which R⁵ is CH₃, R⁶ is CH₂CF₃, and the other variables for a compound correspond in each case to one row of Table A
Table 9: Compounds of formula I.1 in which R⁵ is CH₂CH₃, R⁶ is CH₂CF₃, and the other variables for a compound correspond in each case to one row of Table A
Table 10: Compounds of formula I.1 in which R⁵ is H, R⁶ is 2,4-F₂-C₆H₃, and the other variables for a compound correspond in each case to one row of Table A
Table 11: Compounds of formula I.1 in which R⁵ is CH₃, R⁶ is 2,4-F₂-C₆H₃, and the other variables for a compound correspond in each case to one row of Table A
Table 12: Compounds of formula I.1 in which R⁵ is CH₂CH₃, R⁶ is 2,4-F₂-C₆H₃, and the other variables for a compound correspond in each case to one row of Table A
Table 13: Compounds of formula I.1 in which R⁵ is H, R⁶ is (4R)-2-ethyl-3-oxo-isoxazolidin-4-yl, and the other variables for a compound correspond in each case to one row of Table A
Table 14: Compounds of formula I.1 in which R⁵ is CH₃, R⁶ is (4R)-2-ethyl-3-oxo-isoxazolidin-4-yl, and the other variables for a compound correspond in each case to one row of Table A
Table 15: Compounds of formula I.1 in which R⁵ is CH₂CH₃, R⁶ is (4R)-2-ethyl-3-oxo-isoxazolidin-4-yl, and the other variables for a compound correspond in each case to one row of Table A

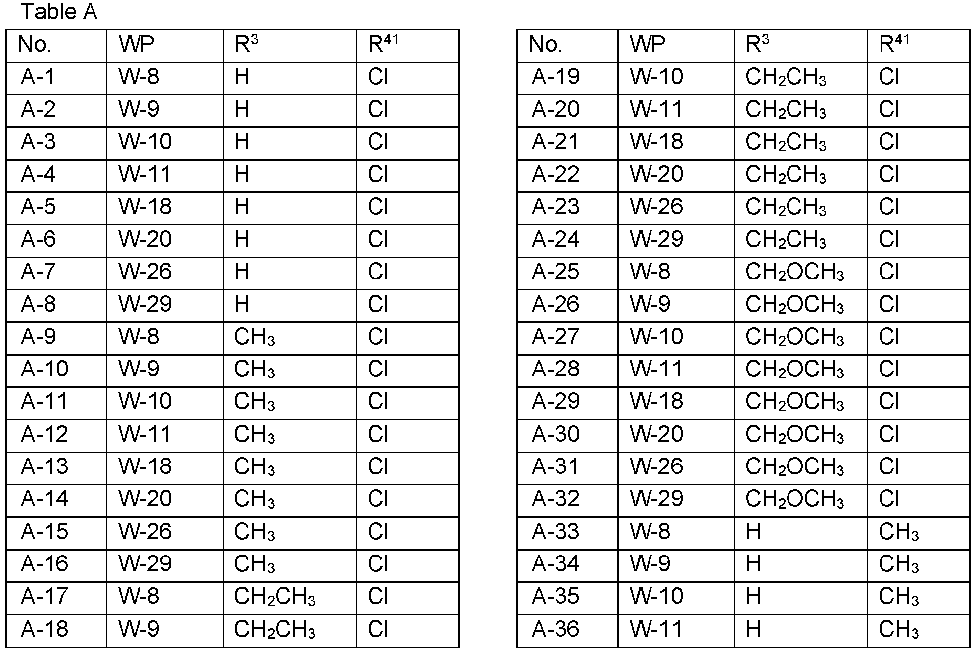

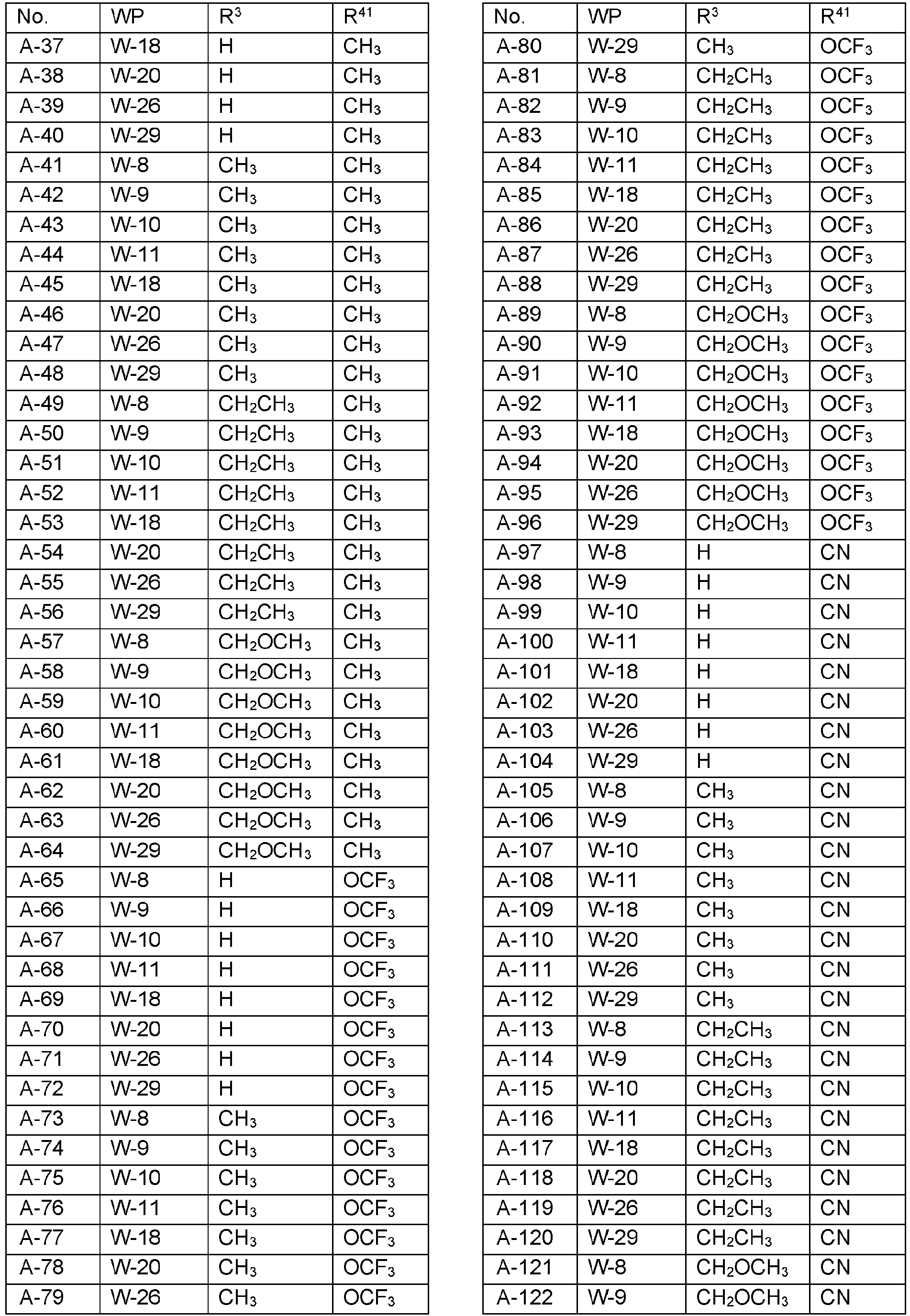

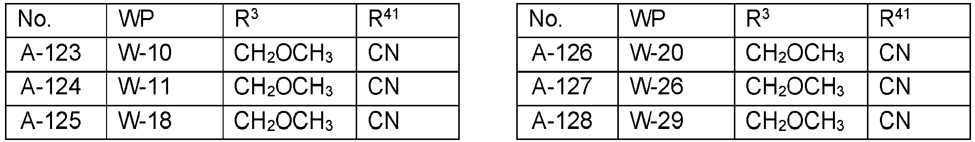

As used herein, the term "compound(s) of the invention" or "compound(s) according to the invention" refers to the compound(s) of formula (I) as defined above, which are also referred to as "compound(s) of formula I" or "compound(s) I" or "formula I compound(s)", and includes their salts, tautomers, stereoisomers, and N-oxides.

The invention also relates to a mixture of at least one compound of the invention with at least one mixing partner. Preferred are binary mixtures of one compound of the invention as component I with one mixing partner herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides, and fungicides.

The following list M of pesticides, grouped according the Mode of Action Classification of the Insecticide Resistance Action Committee (IRAC), together with which the compounds of the invention can be used and with which potential synergistic effects might be produced, illustrates the possible combinations:
M.1 AChE inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifosmethyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathionmethyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
M.2. GABA-gated chloride channel antagonists: cyclodiene organochlorine compounds: endosulfan, chlordane; phenylpyrazoles: ethiprole, fipronil, flufiprole, pyrafluprole, pyriprole;
M.3 Sodium channel modulators: pyrethroids: acrinathrin, allethrin, d-cis-trans allethrin, d-trans allethrin, bifenthrin, kappa-bifenthrin, bioallethrin, bioallethrin S-cylclopentenyl, bio-resmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin,metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; sodium channel modulators, e.g.: DDT, methoxychlor;
M.4 nAChR agonists: neonicotinoids: acetamiprid, clothianidin, cycloxaprid, dinotefuran, imidacloprid, nitenpyram, thiacloprid, thiamethoxam; 4,5-Dihydro-N-nitro-1-(2-oxiranylmethyl)-1H-imidazol-2-amine, (2E-)-1-[(6-Chloropyridin-3-yl)methyl]-N'-nitro-2-pentylidenehydrazinecarboximidamide; 1-[(6-Chloropyridin-3-yl)methyl]-7-methyl-8-nitro-5-propoxy-1,2,3,5,6,7-hexahydroimidazo[1,2-a]pyridine; nicotine; sulfoxaflor; flupyradifurone; triflumezopyrim, (3R)-3-(2-chlorothiazol-5-yl)-8-methyl-5-oxo-6-phenyl-2,3-dihydrothiazolo[3,2-a]pyrimidin-8-ium-7-olate, (3S)-3-(6-chloro-3-pyridyl)-8-methyl-5-oxo-6-phenyl-2,3-dihydrothiazolo[3,2-a]pyrimidin-8-ium-7-olate, (3S)-8-methyl-5-oxo-6-phenyl-3-pyrimidin-5-yl-2,3-dihydrothiazolo[3,2-a]pyrimidin-8-ium-7-olate, (3R)-3-(2-chlorothiazol-5-yl)-8-methyl-5-oxo-6-[3-(trifluoromethyl)phenyl]-2,3-dihydrothiazolo[3,2-a]pyrimidin-8-ium-7-olate; (3R)-3-(2-chlorothiazol-5-yl)-6-(3,5-dichlorophenyl)-8-methyl-5-oxo-2,3-dihydrothiazolo[3,2-a]pyrimidin-8-ium-7-olate, (3R)-3-(2-chlorothiazol-5-yl)-8-ethyl-5-oxo-6-phenyl-2,3-dihydrothiazolo[3,2-a]pyrimidin-8-ium-7-olate;
M.5 Nicotinic acetylcholine receptor allosteric activators: spinosad, spinetoram;
M.6 Chloride channel activators: abamectin, emamectin benzoate, ivermectin, lepimectin, milbemectin;
M.7 Juvenile hormone mimics, e.g.: hydroprene, kino¬prene, methoprene; fenoxycarb, pyriproxyfen;
M.8 miscellaneous multi-site inhibitors: CH₃Br, other alkyl halides, chloropicrin, sulfuryl fluoride, borax, tartar emetic;
M.9 Chordotonal organ TRPV channel modulators: pymetrozine; pyrifluquinazon;
M.10 Mite growth inhibitors: clofentezine, hexythiazox, diflovidazin, etoxazole;
M.11 Microbial disruptors of insect midgut membranes: *bacillus thuringiensis, bacillus sphaericus,* and insecticdal proteins they produce e.g.: *bacillus thuringiensis* subsp. *israelensis, bacillus sphaericus, bacillus thuringiensis* subsp. *aizawai, bacillus thuringiensis* subsp. *kurstaki, bacillus thuringiensis* subsp. *tenebrionis,* Bt crop proteins: Cry1Ab, Cry1Ac, Cry1Fa, Cry2Ab, mCry3A, Cry3Ab, Cry3Bb, Cry34/35Ab1;
M.12 Inhibitors of mitochondrial ATP synthase: diafenthiuron, organotin miticides, e.g.: azocyclotin, cyhexatin, fenbutatin oxide, propargite, tetradifon;
M.13 Uncouplers of oxidative phosphorylation via disruption of the proton gradient:
   chlorfenapyr, DNOC, sulfluramid;
M.14 nAChR channel blockers: nereistoxin analogues bensultap, cartap hydrochloride, thiocyclam, thiosultap-sodium;
M.15 Inhibitors of the chitin biosynthesis type 0, e.g.: bistrifluron, chlorfluazuron, diflubenzuron, flucycloxuron, flufenoxuron, hexaflumuron, lufenuron, novaluron, noviflumuron, teflubenzuron, triflumuron;
M.16 Inhibitors of the chitin biosynthesis type 1: buprofezin;
M.17 Moulting disruptors: Dipteran, cyromazine;
M.18 Ecdyson receptor agonists, e.g.: methoxyfenozide, tebufenozide, halofenozide, fufenozide, chromafenozide;
M.19 Octopamin receptor agonists: amitraz;
M.20 Mitochondrial complex III electron transport inhibitors: hydramethylnon, acequinocyl, fluacrypyrim; bifenazate;
M.21 METI acaricides and insecticides, e.g.: fenazaquin, fenpyroximate, pyrimidifen, pyridaben, tebufenpyrad, tolfenpyrad, rotenone;
M.22 Voltage-dependent sodium channel blockers: indoxacarb, metaflumizone, 2-[2-(4-cyanophenyl)-1-[3-(trifluoromethyl)phenyl]¬ethylidene]-N-[4-(difluoromethoxy)phenyl]-hydrazinecarboxamide, N-(3-chloro-2-methyl¬phenyl)-2-[(4-chlorophenyl)[4-[methyl(methylsulfonyl)amino]phenyl]¬methylene]-hydrazinecarboxamide;
M.23 Inhibitors of the of acetyl CoA carboxylase, e.g.: spirodiclofen, spiromesifen, spirotetramat; spiropidion; spirobudiclofen, 11-(4-chloro-2,6-dimethylphenyl)-12-hydroxy-1,4-dioxa-9-azadispiro[4.2.4.2]tetradec-11-en-10-one, 3-(4'-fluoro-2,4-dimethyl[1,1'-biphenyl]-3-yl)-4-hydroxy-8-Oxa-1-azaspiro[4.5]dec-3-en-2-one;
M.24 Mitochondrial complex IV electron transport inhibitors: e.g. aluminium phosphide, calcium phosphide, zinc phosphide, cyanide;
M.25 Mitochondrial complex II electron transport inhibitors, e.g.: cyenopyrafen, cyflumetofen, cyetpyrafen;
M.28 Ryanodine receptor-modulators: flubendiamide, chlor¬antraniliprole, cyantraniliprole, tetra¬niliprole, (R)-3-chloro-N1-{2-methyl-4-[1,2,2,2-tetrafluoro-1-(trifluoromethyl)ethyl]phenyl}-N2-(1-methyl-2-methylsulfonylethyl)phthalamid, (S)-3-chloro-N1-{2-methyl-4-[1,2,2,2-tetrafluoro-1-(trifluoromethyl)ethyl]phenyl}-N2-(1-methyl-2-methylsulfonylethyl)phthalamide, cyclaniliprole, methyl-2-[3,5-dibromo-2-({[3-bromo-1-(3-chlorpyridin-2-yl)-1H-pyrazol-5-yl]carbonyl}¬amino)benzoyl]-1,2-dimethylhydrazine-carboxylate; N-[2-(5-amino-1,3,4-thiadiazol-2-yl)-4-chloro-6-methyl¬phenyl]-3-bromo-1-(3-chloro-2-pyridinyl)-1H-pyrazole-5-carboxamide; 3-chloro-1-(3-chloro-2-pyridinyl)-N-[2,4-dichloro-6-[[(1-cyano-1-methylethyl)amino]carbonyl]phenyl]-1H-pyrazole-5-carboxamide; tetrachlorantraniliprole; N-[4-chloro-2-[[(1,1-dimethylethyl)amino]carbonyl]-6-methyl-phenyl]-1-(3-chloro-2-pyridinyl)-3-(fluoromethoxy)-1H-pyrazole-5-carboxamide; cyhalodiamide;
M.29: Chordotonal organ Modulators: flonicamid;
M.UN. Unknown mode of action: afido¬pyro¬pen, afoxolaner, azadirachtin, amidoflumet, benzoximate, broflanilide, bromopropylate, chino¬methionat, cryolite, cyproflanilide, dicloromezotiaz, dicofol, dimpropyridaz, flufenerim, flometoquin, fluensulfone, fluhexafon, fluopyram, fluralaner, metaldehyde, metoxadiazone, piperonyl butoxide, pyflubumide, pyridalyl, tioxazafen, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1H-1,2,4-triazole-5-amine, actives on basis of *bacillus firmus* (Votivo, I-1582); flupyrimin; fluazaindolizine; fluxametamide; 5-[3-[2,6-dichloro-4-(3,3-dichloroallyloxy)phenoxy]propoxy]-1H-pyrazole; 4-cyano-N-[2-cyano-5-[[2,6-dibromo-4-[1,2,2,3,3,3-hexafluoro-1-(trifluoromethyl)¬propyl]phenyl]carbamoyl]phenyl]-2-methyl-benzamide; 4-cyano-3-[(4-cyano-2-methyl-benzoyl)amino]-N-[2,6-dichloro-4-[1,2,2,3,3,3-hexafluoro-1-(trifluoromethyl)-propyl]phenyl]-2-fluoro-benzamide; N-[5-[[2-chloro-6-cyano-4-[1,2,2,3,3,3-hexafluoro-1-(trifluoromethyl)propyl]phenyl]carbamoyl]-2-cyanophenyl]-4-cyano-2-methyl-benzamide; N-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; N-[5-[[2-bromo-6-chloro-4-[1,2,2,3,3,3-hexafluoro-1-(trifluoromethyl)-propyl]phenyl]carbamoyl]-2-cyanophenyl]-4-cyano-2-methyl-benzamide; 4-cyano-N-[2-cyano-5-[[2,6-dichloro-4-[1,2,2,3,3,3-hexafluoro-1-(trifluoromethyl)-propyl]phenyl]carbamoyl]phenyl]-2-methyl-benzamide; 4-cyano-N-[2-cyano-5-[[2,6-dichloro-4-[1,2,2,2-tetrafluoro-1-(trifluoromethyl)ethyl]phenyl]carbamoyl]¬phenyl]-2-methyl-benzamide; N-[5-[[2-bromo-6-chloro-4-[1,2,2,2-tetrafluoro-1-(trifluoromethyl)ethyl]phenyl]carba¬moyl]-2-cyano-phenyl]-4-cyano-2-methyl-benzamide;
   2-(1,3-dioxan-2-yl)-6-[2-(3-pyridinyl)-5-thiazolyl]-pyridine; 2-[6-[2-(5-fluoro-3-pyridinyl)-5-thiazo¬lyl]-2-pyridinyl]-pyrimidine; 2-[6-[2-(3-pyridinyl)-5-thiazolyl]-2-pyridinyl]-pyrimidine; N-methylsul¬fonyl-6-[2-(3-pyridyl)thiazol-5-yl]pyridine-2-carboxamide; N-methylsulfonyl-6-[2-(3-pyridyl)thiazol-5-yl]pyridine-2-carboxamide; 1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; N-(1-methylethyl)-2-(3-pyridinyl)-2H-indazole-4-carboxamide; N-cyclopropyl-2-(3-pyridinyl)-2H-indazole-4-carboxamide; N-cyclohexyl-2-(3-pyridinyl)-2H-indazole-4-carboxamide; 2-(3-pyridinyl)-N-(2,2,2-trifluoroethyl)-2H-indazole-4-carboxamide; 2-(3-pyridinyl)-N-[(tetrahydro-2-furanyl)methyl]-2H-indazole-5-carboxamide; methyl 2-[[2-(3-pyridinyl)-2H-indazol-5-yl]carbonyl]hydrazinecarboxylate; N-[(2,2-difluorocyclopropyl)methyl]-2-(3-pyridinyl)-2H-indazole-5-carboxamide; N-(2,2-difluoropropyl)-2-(3-pyridinyl)-2H-indazole-5-carboxamide; 2-(3-pyridinyl)-N-(2-pyrimidinylmethyl)-2H-indazole-5-carboxamide; N-[(5-methyl-2-pyrazinyl)methyl]-2-(3-pyridinyl)-2H-indazole-5-carboxamide, tyclopyrazoflor; sarolaner, lotilaner; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; 2-(3-ethylsulfonyl-2-pyridyl)-3-methyl-6-(tri-fluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine; Isocycloseram; N-[4-chloro-3-(cyclopropylcarbamo¬yl)phenyl]-2-methyl-5-(1,1,2,2,2-pentafluoroethyl)-4-(trifluoromethyl)pyrazole-3-carboxamide, N-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; acynonapyr; benzpyrimoxan; tigolaner; oxazosulfyl; [(2S,3R,4R,5S,6S)-3,5-dimethoxy-6-methyl-4-propoxy-tetrahydropyran-2-yl] N-[4-[1-[4-(trifluoromethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(trifluoromethoxy)phenyl]-1,2,4-triazol-3-yl]phenyl]carbamate; [(2S,3R,4R,5S,6S)-3,5-dimethoxy-6-methyl-4-propoxy-tetrahydropyran-2-yl] N-[4-[1-[4-(1,1,2,2,2-pentafluoroethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(1,1,2,2,2-pentafluoroethoxy)phenyl]-1,2,4-triazol-3-yl]phenyl]carbamate; (2Z)-3-(2-isopropylphenyl)-2-[(E)-[4-[1-[4-(trifluoromethoxy)phenyl]-1,2,4-triazol-3-yl]phenyl]methylenehydrazono]thiazolidin-4-one, (2Z)-3-(2-isopro¬pyl¬phenyl)-2-[(E)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-[3-ethylsulfonyl-6-(trifluoromethyl)imidazo[1,2-a]pyridin-2-yl]-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine; N-[[2-fluoro-4-[(2S,3S)-2-hydroxy-3-(3,4,5-trichlorophenyl)-3-(trifluoromethyl)pyrrolidin-1-yl]phenyl]methyl]cyclopropanecarboxamide; 2-[2-fluoro-4-methyl-5-(2,2,2-trifluoroethylsulfinyl)phenyl]imino-3-(2,2,2-trifluoroethyl)thiazolidin-4-one; flupentiofenox, N-[3-chloro-1-(3-pyridyl)pyrazol-4-yl]-2-methylsulfonyl-propanamide, cyclobutrifluram, N-[1-(difluoromethyl)cyclopropyl]-2-(3-pyridyl)indazole-4-carboxamide; N-(1,1-dimethyl-2-methylsulfinyl-ethyl)-7-fluoro-2-(3-pyridyl)indazole-4-carboxamide; N-(1,1-dimethyl-2-methylsulfonyl-ethyl)-7-fluoro-2-(3-pyridyl)indazole-4-carboxamide; N-(1,1-dimethyl-2-methylsulfanylethyl)-7-fluoro-2-(3-pyridyl)indazole-4-carboxamide; 2-chloro-N-cyclopropyl-5-[1-[2,6-dichloro-4-[1,2,2,2-tetrafluoro-1-(trifluoromethyl)ethyl]phenyl]pyrazol-4-yl]-N-methyl-pyridine-3-carboxamide; 2-chloro-5-[1-[2-chloro-4-[1,2,2,2-tetrafluoro-1-(trifluoromethyl)ethyl]-6-(trifluoromethyl)phenyl]pyrazol-4-yl]-N-cyclopropyl-pyridine-3-carboxamide; 2-chloro-5-[1-[2-chloro-4-[1,2,2,2-tetrafluoro-1-(trifluoromethyl)ethyl]-6-(trifluoromethoxy)phenyl]pyrazol-4-yl]-N-cyclopropyl-pyridine-3-carboxamide; 5-[1-[2-bromo-4-[1,2,2,2-tetrafluoro-1-(trifluoromethyl)ethyl]-6-(trifluoromethoxy)phenyl]pyrazol-4-yl]-2-chloro-N-cyclopropyl-pyridine-3-carboxamide; N-[4-chloro-3-[(1-cyanocyclopropyl)carbamoyl]phenyl]-2-methyl-4-methylsulfonyl-5-(1,1,2,2,2-pentafluoroethyl)pyrazole-3-carboxamide.

The commercially available compounds M listed above may be found in The Pesticide Manual, 18th Edition, C. MacBean, British Crop Protection Council (2018), or http://bcpcdata.com/pesticide-manual.html, http://www.alanwood.net/pesticides.

The active compounds described by IUPAC nomenclature are known from CN103814937; WO2013/003977, WO2007/101369, WO2018/177970, CN10171577, CN102126994, WO2007/101540, WO2007/043677, WO2011/085575, WO2008/134969, WO2012/034403, WO2006/089633, WO2008/067911, WO2006/043635, WO2009/124707, WO2013/050317, WO2010/060379, WO2010/127926, WO2010/006713, WO2012/000896, WO2007/101369, WO2012/143317, WO2015/038503, EP2910126, WO2015/059039, WO2015/190316, WO2012/126766, WO2009/102736, WO2013/116053, WO2018/052136, WO2016/180802, WO2013092350, WO2019236274, WO2006089633, EP2099751, WO2020/018362, EP3586630, EP 3608311, WO2020/001067.

The following list of fungicides, in conjunction with which the compounds of the invention can be used, illustrates the possible combinations:
A) Respiration inhibitors
   - Inhibitors of complex III at Qo site: azoxystrobin (A.1.1), coumeth¬oxy¬strobin (A.1.2), coumoxystrobin (A.1.3), dimoxystrobin (A.1.4), enestroburin (A.1.5), fenaminstrobin (A.1.6), fenoxy¬strobin/flufenoxystrobin (A.1.7), fluoxastro¬bin (A.1.8), kresoxim-methyl (A.1.9), mandestrobin (A.1.10), meto¬minostrobin (A.1.11), orysastrobin (A.1.12), picoxy¬strobin (A.1.13), pyraclostrobin (A.1.14), pyrametostrobin (A.1.15), pyraoxystrobin (A.1.16), trifloxystrobin (A.1.17), 2 (2-(3-(2,6-di¬chlorophenyl)-1-methyl-allylidene¬aminooxy¬methyl)-phenyl)-2 methoxyimino-N methyl-acetamide (A.1.18), pyribencarb (A.1.19), triclopyricarb/chloro-dincarb (A.1.20), famoxadone (A.1.21), fenamidone (A.1.21), methyl-N-[2-[(1,4-dimethyl-5 phenyl-pyrazol-3-yl)oxylmethyl]phenyl]-N-methoxy-carbamate (A.1.22), metyltetraprole (A.1.25), (Z,2E) 5 [1-(2,4-dichloro¬phenyl)pyrazol-3-yl]-oxy-2-methoxyimino-N,3-dimethyl-pent-3-en¬amide (A.1.34), (Z,2E) 5 [1 (4-chlorophenyl)pyrazol-3-yl]oxy-2-methoxyimino-N,3-dimethyl-pent-3-enamide (A.1.35), pyriminostrobin (A.1.36), bifujunzhi (A.1.37), 2-(ortho-((2,5-dimeth¬ylphenyl-oxy¬methylen)phenyl)-3-methoxy-acrylic acid methylester (A.1.38);
   - inhibitors of complex III at Qi site: cyazofamid (A.2.1), amisulbrom (A.2.2), [(6S,7R,8R) 8 benzyl-3-[(3-hydroxy-4-methoxy-pyridine-2-carbonyl)amino]-6-methyl-4,9-di¬oxo-1,5-dioxonan-7-yl] 2-methylpropanoate (A.2.3), fenpicoxamid (A.2.4), florylpicoxamid (A.2.5), [(1S,2S)-2-(4-fluoro-2-methyl-phenyl)-1,3-dimethyl-butyl] (2S)-2-[(3-hydroxy-4-methoxy-pyridine-2-carbonyl)amino]propanoate, [(1S,2S)-2-(2,4-dimethylphenyl)-1,3-di¬methyl-butyl](2S)-2-[(3-hydroxy-4-methoxy-pyridine-2-carbonyl)amino]propanoate,
      [(1S,2S)-2-(2,4-di¬fluorophenyl)-1,3-dimethyl-butyl] (2S)-2-[(3-hydroxy-4-methoxy-pyridine-2 carbonyl)amino]¬propanoate, [(1S,2S)-2-(2-fluoro-4-methyl-phenyl)-1 ,3-dimethyl-butyl]
      (2S)-2-[(3-hydroxy-4-methoxy-pyridine-2-carbonyl)amino]propanoate, [(1S,2S)-2-(4-fluoro-2 methyl-phenyl)-1,3-dimethyl-butyl] (2S)-2-[(3-acetoxy-4-methoxy-pyridine-2-carbonyl)¬amino]-propanoate, [(1S,2S)-2-(2,4-dimethylphenyl)-1,3-dimethyl-butyl] (2S)-2-[(3-acetoxy-4 methoxy-pyridine-2-carbonyl)amino]propanoate, [(1S,2S)-2-(2,4-difluorophenyl)-1,3-di¬methyl-butyl] (2S)-2-[(3-acetoxy-4-methoxy-pyridine-2-carbonyl)amino]propanoate, [(1S,2S)-2-(2-fluoro-4-methyl-phenyl)-1,3-dimethyl-butyl] (2S)-2-[(3-acetoxy-4-methoxy-pyridine-2-car-bonyl)amino]propanoate, [2-[[(1S)-2-[(1S,2S)-2-(4-fluoro-2-methyl-phenyl)-1,3-dimethyl-but-oxy]-1-methyl-2-oxoethyl]carbamoyl]-4-methoxy-3-pyridyl]oxymethyl 2-methylpropanoate, [2-[[(1S)-2-[(1S,2S)-2-(2,4-dimethylphenyl)-1,3-dimethyl-butoxy]-1-methyl-2-oxo-ethyl]carba-moyl]-4-methoxy-3-pyridyl]oxymethyl 2-methylpropanoate, [2-[[(1S)-2-[(1S,2S)-2-(2,4-difluo-rophenyl)-1,3-dimethyl-butoxy]-1-methyl-2-oxo-ethyl]carbamoyl]-4-methoxy-3-pyridyl]oxy-methyl 2-methylpropanoate, [2-[[(1S)-2-[(1S,2S)-2-(2-fluoro-4-methyl-phenyl)-1,3-dimethyl-but-oxy]-1-methyl-2-oxo-ethyl]carbamoyl]-4-methoxy-3-pyridyl]oxymethyl 2-methylpropa¬no¬ate, [(1S,2S)-1-methyl-2-(o-tolyl)propyl] (2S)-2-[(3-acetoxy-4-methoxy-pyridine-2-carbonyl)¬amino]-propanoate, [(1S,2S)-1-methyl-2-(o-tolyl)propyl] (2S)-2-[(4-methoxy-3-propanoyloxy-pyridine-2-carbonyl)amino]propanoate, [(1S,2S)-1-methyl-2-(o-tolyl)propyl] (2S)-2-[(3-hydro¬xy-4-methoxy-pyridine-2-carbonyl)amino]propanoate, [4-methoxy-2-[[(1S)-1-methyl-2-[(1S,2S)-1-methyl-2-(o-tolyl)propoxy]-2-oxoethyl]carbamoyl]-3-pyridyl] 2-methylpropanoate, [(1S,2S)-2-(2,4-dimethylphenyl)-1-methyl-propyl] (2S)-2-[(3-acetoxy-4-methoxy-pyridine-2-carbonyl)amino]propanoate, [2-[[(1S)-2-[(1S,2S)-2-(2,4-dimethylphenyl)-1-methyl-propoxy]-1-methyl-2-oxo-ethyl]carbamoyl]-4-methoxy-3-pyridyl] 2-methylpropanoate,
      [(1S,2S)-2-(2,4-dimethylphenyl)-1-methyl-propyl] (2S)-2-[(3-hydroxy-4-methoxy-pyridine-2 carbonyl)amino]propanoate, [(1S,2S)-2-(2,6-dimethylphenyl)-1-methyl-propyl](2S)-2-[(3-acetoxy-4-methoxy-pyridine-2-carbonyl)amino]propanoate, [2-[[(1S)-2-[(1S,2S)-2-(2,6-di-methylphenyl)-1-methyl-propoxy]-1-methyl-2-oxo-ethyl]carbamoyl]-4-methoxy-3-pyridyl] 2 methylpropanoate, [(1S,2S)-2-(2,6-dimethylphenyl)-1-methyl-propyl] (2S)-2-[(3-hydroxy-4 methoxy-pyridine-2-carbonyl)amino]propanoate, [(1S,2S)-2-[4-fluoro-2-(trifluoromethyl)-phenyl]-1-methyl-propyl] (2S)-2-[(3-acetoxy-4-methoxy-pyridine-2-carbonyl)amino]propano¬ate, [2-[[(1S)-2-[(1S,2S)-2-[4-fluoro-2-(trifluoromethyl)phenyl]-1-methyl-propoxy]-1-methyl-2 oxo-ethyl]carbamoyl]-4-methoxy-3-pyridyl] 2-methylpropanoate, [(1S,2S)-2-[4-fluoro-2-(tri-fluoromethyl)phenyl]-1-methyl-propyl] (2S)-2-[(3-hydroxy-4-methoxy-pyridine-2-carbo¬nyl)-amino]propanoate, [(1S,2S)-2-(4-fluoro-2-methylphenyl)-1-methyl-propyl] (2S)-2-[(3-acet¬oxy-4-methoxy-pyridine-2-carbonyl)amino]propanoate, [2-[[(1S)-2-[(1S,2S)-2-(4-fluoro-2-methyl-phenyl)-1-methyl-propoxy]-1-methyl-2-oxo-ethyl]carbamoyl]-4-methoxy-3-pyridyl] 2-methylpropanoate, [(1S,2S)-2-(4-fluoro-2-methyl-phenyl)-1-methyl-propyl] (2S)-2-[(3-hydroxy-4-methoxy-pyridine-2-carbonyl)amino]propanoate, [(1S,2S)-1-methyl-2-[2-(trifluoromethyl)-phenyl]propyl] (2S)-2-[(3-acetoxy-4-methoxy-pyridine-2-carbonyl)amino]propanoate, [4-methoxy-2-[[(1S)-1-methyl-2-[(1S,2S)-1-methyl-2-[2-(trifluoromethyl)phenyl]propoxy]-2-oxo-ethyl]carbamoyl]-3-pyridyl]2-methylpropanoate, [(1S,2S)-1-methyl-2-[2-(trifluoromethyl)-phenyl]¬propyl] (2S)-2-[(3-hydroxy-4-methoxy-pyridine-2-carbonyl)amino]propanoate, [(1S,2S)-2-(4-fluoro-2,6-dimethyl-phenyl)-1-methyl-propyl] (2S)-2-[(3-acetoxy-4-methoxy-pyridine-2-carbonyl)amino]propanoate, [2-[[(1S)-2-[(1S,2S)-2-(4-fluoro-2,6-dimethyl-phenyl)-1-methyl-propoxy]-1-methyl-2-oxo-ethyl]carbamoyl]-4-methoxy-3-pyridyl] 2-methy!propano¬ate, [(1S,2S)-2-(4-fluoro-2,6-dimethyl-phenyl)-1-methyl-propyl] (2S)-2-[(3-hydroxy-4-meth¬oxy-pyridine-2-carbonyl)amino]propanoate;
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoro-methyl)phenoxy]phenyl]-3-(difluoromethyl)-5-fluoro-1-methyl-pyrazole-4-carboxamide (A.3.29), methyl (E)-2-[2-[(5-cyano-2-methyl-phenoxy)methyl]phenyl]-3-methoxy-prop-2 enoate (A.3.30), isoflucypram (A.3.31), 2-(difluoromethyl)-N-(1,1,3-trimethyl-indan-4 yl)¬pyridine-3-carboxamide (A.3.32), 2-(difluoromethyl)-N-[(3R)-1,1,3-trimethylindan-4-yl]¬pyridine-3-carboxamide (A.3.33), 2-(difluoromethyl)-N-(3-ethyl-1,1-dimethyl-indan-4-yl)¬pyridine-3-carboxamide (A.3.34), 2-(difluoromethyl)-N-[(3R)-3-ethyl-1,1-dimethyl-indan-4-yl]¬pyridine-3-carboxamide (A.3.35), 2-(difluoromethyl)-N-(1,1-dimethyl-3-propyl-indan-4-yl)¬py¬ridine-3-carboxamide (A.3.36), 2-(difluoromethyl)-N-[(3R)-1,1-dimethyl-3-propyl-indan-4-yl]¬pyridine-3-carboxamide (A.3.37), 2-(difluoromethyl)-N-(3-isobutyl-1,1-dimethyl-indan-4-yl)¬pyridine-3-carboxamide (A.3.38), 2-(difluoromethyl)-N-[(3R)-3-isobutyl-1,1-dimethyl-indan-4 yl]pyridine-3-carboxamide (A.3.39);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothio¬conazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2 (2,4-difluorophenyl)-1,1-difluoro-3-(tetrazol-1-yl)-1-[5-[4-(2,2,2-trifluoroethoxy)phenyl]-2 pyridyl]propan-2-ol (B.1.31), 2-(2,4-difluorophenyl)-1,1-difluoro-3-(tetrazol-1-yl)-1 [5 [4 (trifluoromethoxy)phenyl]-2-pyridyl]propan-2-ol (B.1.32), 4-[[6-[2-(2,4-difluorophenyl)-1,1-difluoro-2-hydroxy-3-(5-sulfanyl-1,2,4-triazol-1-yl)propyl]-3-pyridyl]oxy]benzonitrile (B.1.33), ipfentrifluconazole (B.1.37), mefentrifluconazole (B.1.38), 2-(chloromethyl)-2-methyl-5-(p-tolylmethyl)-1 (1,2,4-triazol-1 ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fe¬narimol (B.1.49), pyrifenox (B.1.50), triforine (B.1.51), [3-(4-chloro-2-fluoro-phenyl)-5-(2,4-di¬fluoro-phenyl)isoxazol-4-yl]-(3-pyridyl)methanol (B.1.52), 4-[[6-[2-(2,4-difluorophenyl)-1,1-difluoro-2-hydroxy-3-(1,2,4-triazol-1-yl)propyl]-3-pyridyl]oxy]-benzo¬nitrile (B.1.53), 2-[6-(4-bromophenoxy)-2-(trifluoromethyl)-3-pyridyl]-1-(1,2,4-triazol-1 yl)propan-2-ol (B.1.54), 2-[6-(4-chlorophenoxy)-2-(trifluoromethyl)-3-pyridyl]-1-(1,2,4-tri¬azol-1-yl)propan-2-ol (B.1.55);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spirox-amine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4 amine (C.2.6), 5-fluoro-2-(4-fluorophenylmethoxy)pyrimidin-4 amine (C.2.7), 5-fluoro-2 (4 chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), N-eth¬yl-2-[(3-ethynyl-8-methyl-6-quinolyl)oxy]butanamide (D.1.8), N-ethyl-2-[(3-ethynyl-8 methyl-6 quinolyl)oxy]-2-methylsulfanyl-acetamide (D.1.9), 2-[(3-ethynyl-8-methyl-6-quinol¬yl)oxy]-N (2-fluoroethyl)butanamide (D.1.10), 2-[(3-ethynyl-8-methyl-6-quinolyl)oxy]-N-(2-flu¬oroeth¬yl)-2-methoxy-acetamide (D.1.11), 2-[(3-ethynyl-8-methyl-6-quinolyl)oxy]-N-propyl-butanam¬ide (D.1.12), 2-[(3-ethynyl-8-methyl-6-quinolyl)oxy]-2-methoxy-N-propyl-acetamide (D.1.13), 2-[(3-ethynyl-8-methyl-6-quinolyl)oxy]-2-methylsulfanyl-N-propyl-acetamide (D.1.14), 2 [(3 ethynyl-8-methyl-6-quinolyl)oxy]-N-(2-fluoroethyl)-2-methylsulfanyl-acetamide (D.1.15), 4-(2-bromo-4-fluoro-phenyl)-N-(2-chloro-6-fluoro-phenyl)-2,5-dimethyl-pyrazol-3 amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepani¬pyrim (E.1.2), pyrimethanil (E.1.3);
   - protein synthesis inhibitors: blasticidin-S (E.2.1), kasugamycin (E.2.2), kasugamycin hydro-chloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-N-te¬tralin-1-yl-pyridine-2-carboxamide (G.5.4), 4-[1-[2-[3,5-bis(difluoromethyl)pyrazol-1-yl]acetyl]-4-piperidyl]-N-tetralin-1-yl-pyridine-2-carboxamide (G.5.5), 4-[1-[2-[3-(difluoromethyl)-5-(tri¬fluoromethyl)pyrazol-1-yl]acetyl]-4-piperidyl]-N-tetralin-1-yl-pyridine-2-carboxamide (G.5.6), 4-[1-[2-[5-cyclopropyl-3-(difluoromethyl)pyrazol-1-yl]acetyl]-4-piperidyl]-N-tetralin-1-yl-pyri¬dine-2-carboxamide (G.5.7), 4-[1-[2-[5-methyl-3-(trifluoromethyl)pyrazol-1-yl]acetyl]-4-pi¬peri¬dyl]-N-tetralin-1-yl-pyridine-2-carboxamide (G.5.8), 4-[1-[2-[5-(difluoromethyl)-3-(trifluoro¬methyl)pyrazol-1-yl]acetyl]-4-piperidyl]-N-tetralin-1-yl-pyridine-2-carboxamide (G.5.9), 4 [1[2-[3,5-bis(trifluoromethyl)pyrazol-1-yl]acetyl]-4-piperidyl]-N-tetralin-1-yl-pyridine-2-car¬boxamide (G.5.10), (4-[1-[2-[5-cyclopropyl-3-(trifluoromethyl)pyrazol-1-yl]acetyl]-4-piperidyl]-N-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachloro¬benzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-di¬methyl-1H,5H-[1,4]di¬thiino[2,3-c:5,6-c']dipyrrole-1,3,5,7(2H,6H)-tetraone (H.4.10);
I) Cell wall synthesis inhibitors
   - inhibitors of glucan synthesis: validamycin (1.1.1), polyoxin B (1.1.2);
   - melanin synthesis inhibitors: pyroquilon (1.2.1), tricyclazole (1.2.2), carpropamid (1.2.3), dicyclomet (1.2.4), fenoxanil (1.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phospho¬nate (J.1.12), potassium or sodium bicarbonate (J.1.9), 4 cyclopropyl-N-(2,4-di¬methoxy¬phenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclo¬mezine (K.1.8), difenzoquat (K.1.9), di-fenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), metha-sulfocarb (K.1.18), nitrapyrin (K.1.19), nitrothal-isopropyl (K.1.20), tolprocarb (K.1.21), oxin-copper (K.1.22), proquinazid (K.1.23), tebufloquin (K.1.24), tecloftalam (K.1.25), triazoxide (K.1.26), N'-(4-(4-chloro-3-trifluoro¬methyl-phen¬oxy)-2,5-dimethyl-phenyl)-N-ethyl-N methyl formamidine (K.1.27), N' (4-(4-fluoro-3-trifluoro¬methyl-phenoxy)-2,5-dimethyl-phenyl)-N-eth¬yl-N-methyl formamidine (K.1.28), N'-[4-[[3-[(4-chlorophenyl)methyl]-1,2,4-thiadiazol-5-yl]¬oxy]-2,5-dimethylphenyl]-N-ethyl-N-methyl-formamidine (K.1.29), N'-(5-bromo-6-indan-2-yl¬oxy-2-methyl-3-pyridyl)-N-ethyl-N-methyl-formamidine (K.1.30), N'-[5-bromo-6-[1-(3,5-diflu-orophenyl)ethoxy]-2-methyl-3-pyridyl]-N-ethyl-N-methyl-formamidine (K.1.31), N'-[5-bromo-6-(4-isopropylcyclohexoxy)-2-methyl-3-pyridyl]-N-ethyl-N-methyl-formamidine (K.1.32), N' [5 bromo-2-methyl-6-(1-phenylethoxy)-3-pyridyl]-N-ethyl-N-methyl-formamidine (K.1.33), N'-(2-methyl-5-trifluoromethyl-4-(3-trimethyl¬silanyl-prop¬oxy)-phenyl)-N-ethyl-N-methyl forma-midine (K.1.34), N'-(5-difluoromethyl-2 methyl-4-(3-tri¬methylsilanyl-propoxy)-phenyl)-N-ethyl-N-methyl formamidine (K.1.35), 2-(4-chloro-phenyl)-N-[4-(3,4-dimeth¬oxy-phenyl)-isoxazol-5 yl]-2-prop-2-ynyloxy-acetamide (K.1.36), 3 [5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3 yl]-pyridine (K.1.38), 5-chloro-1 (4,6-di¬methoxy-pyrimidin-2-yl)-2-methyl-1H-benzoimidazole (K.1.39), ethyl (Z) 3 amino-2-cyano-3-phenyl-prop-2-enoate (K.1.40), picarbutrazox (K.1.41), pentyl N-[6-[[(Z)-[(1-methyltetrazol-5-yl)-phenylmethylene]amino]oxy¬methyl]-2-pyridyl]carba-mate (K.1.42), but-3-ynyl N-[6-[[(Z)-[(1-methyltetrazol-5-yl)-phenyl-methylene]amino]oxy-methyl]-2-pyridyl]carbamate (K.1.43), ipflufenoquin (K.1.44), quinofumelin (K.1.47), benziothiazolinone (K.1.48), bromothalonil (K.1.49), 2-(6-benzyl-2-pyridyl)quinazoline (K.1.50), 2-[6-(3-fluoro-4 methoxy-phenyl)-5-methyl-2-pyridyl]¬quinazoline (K.1.51), dichlobentiazox (K.1.52), N'-(2,5-dimethyl-4-phen¬oxy-phenyl)-N-ethyl-N-methyl-formamidine (K.1.53), aminopyrifen (K.1.54), fluopimomide (K.1.55), N'-[5-bromo-2-methyl-6-(1-methyl-2 propoxy-ethoxy)-3-pyridyl]-N-ethyl-N-methyl-formamidine (K.1.56), N'-[4-(4,5-dichloro-thiazol-2-yl)oxy-2,5-dimethyl-phenyl]-N-ethyl-N-methyl-formamidine (K.1.57), N-(2-fluorophenyl)-4-[5-(trifluoromethyl)-1,2,4-oxadiazol-3-yl]benzamide (K.1.58), N-methyl-4-[5-(trifluoromethyl)-1,2,4-oxadiazol-3-yl]benzenecarbothioamide (K.1.59);
L) Biopesticides
   L1) Microbial pesticides with fungicidal, bactericidal, viricidal and/or plant defense activator activity: Ampelomyces quisqualis, Aspergillus flavus, Aureobasidium pullulans, Bacillus altitudinis, B. amyloliquefaciens, B. amyloliquefaciens ssp. plantarum (also referred to as B. velezensis), B. megaterium, B. mojavensis, B. mycoides, B. pum¬lus, B. simplex, B. solisalsi, B. subtilis, B. subtilis var. amyloliquefaciens, B. velezensis, Candida oleo¬phila, C. saitoana, Clavibacter michiga¬nensis (bacteriophages), Coniothyrium minitans, Cryphonectria parasitica, Cryptococcus albidus, Dilophosphora alopecuri, Fusarium oxysporum, Clonostachys rosea f. catenulate (also named Gliocladium catenulatum), Gliocladium roseum, Lysobacter antibioticus, L. enzymogenes, Metschnikowia fructi¬cola, Microdochium dimerum, Microsphaeropsis ochracea, Muscodor albus, Paen¬bacillus alvei, Paenibacillus epiphyticus, P. polymyxa, Pantoea vagans, Penicillium bilaiae, Phlebiopsis gigantea, Pseudomonas sp., Pseudomonas chloraphis, Pseudo¬zyma flocculosa, Pichia anomala, Pythium oligandrum, Sphaerodes myco¬parasitica, Streptomyces griseoviridis, S. lydicus, S. violaceusniger, Talaromyces flavus, Tricho-derma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia, zucchini yellow mosaic virus (avirulent strain);
   L2) Biochemical pesticides with fungicidal, bactericidal, viricidal and/or plant defense activator activity: harpin protein, Reynoutria sachalinensis extract;
   L3) Microbial pesticides with insecticidal, acaricidal, molluscidal and/or nematicidal activity: Agrobacterium radiobacter, Bacillus cereus, B. firmus, B. thuringiensis, B. thuringiensis ssp. aizawai, B. t. ssp. israelensis, B. t. ssp. galleriae, B. t. ssp. kurstaki, B. t. ssp. tene-brionis, Beauveria bassiana, B. brongniartii, Burkholderia spp., Chromobacterium sub-tsugae, Cydia pomonella granulovirus (CpGV), Cryptophlebia leucotreta granulovirus (CrleGV), Flavobacterium spp., Helicoverpa armigera nucleopolyhedrovirus (HearNPV), Helicoverpa zea nucleopolyhedrovirus (HzNPV), Helicoverpa zea single capsid nucleopolyhedrovirus (HzSNPV), Heterorhabditis bacteriophora, Isaria fumoso¬rosea, Lecanicillium longispo¬rum, L. muscarium, Metarhizium anisopliae, M. anisopliae var. anisopliae, M. anisopliae var. acridum, Nomuraea rileyi, Paecilomyces fumoso¬roseus, P. lilacinus, Paenibacillus popilliae, Pasteuria spp., P. nishizawae, P. pen¬trans, P. ramosa, P. thornea, P. usgae, Pseudomonas fluorescens, Spodoptera litto¬ralis nucleopoly-hedrovirus (SpliNPV), Steinernema carpocapsae, S. feltiae, S. kraussei, Streptomyces galbus, S. microflavus;
   L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl butyrate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadeca¬dien-1-ol acetate, (E,Z)-3,13-octadecadien-1-ol, (R)-1-octen-3-ol, pentatermanone, (E,Z,Z) 3,8,11-tetradecatrienyl acetate, (Z,E) 9,12-tetradecadien-1-yl acetate, (Z) 7 tetradecen-2-one, (Z)-9-tetradecen-1-yl acetate, (Z)-11-tetradecenal, (Z)-11-tetra¬decen-1-ol, extract of Chenopodium ambrosiodes, Neem oil, Quillay extract; L5) Microbial pesticides with plant stress reducing, plant growth regulator, plant growth promoting and/or yield enhancing activity: Azospirillum amazonense, A. brasilense, A. lipoferum, A. irakense, A. halopraeferens, Bradyrhizobium spp., B. elkanii, B. japo¬ni¬cum, B. liaoningense, B. lupini, Delftia acidovorans, Glomus intraradices, Mesorhizo¬bium spp., Rhizobium leguminosarum bv. pha¬seoli, R. I. bv. trifolii, R. I. bv. viciae, R. tropici, Sinorhizobium meliloti. The fungicides described by common names, their preparation and their activity e.g. against harmful fungi is known (cf.: http://www.alanwood.net/pesticides/); these substances are commercially available.

The active substances referred to as component 2, their preparation and their activity e.g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP 141 317; EP 152 031; EP 226 917; EP 243 970; EP 256 503; EP 428 941; EP 532 022; EP 1 028 125; EP 1 035 122; EP 1 201 648; EP 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441). Some compounds are identified by their CAS Registry Number.

Suitable mixing partners for the compounds of the invention also include biopesticides. Biopesticides have been defined as a form of pesticides based on micro-organisms (bacteria, fungi, viruses, nematodes, etc.) or natural products (compounds, e.g. metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances or or structurally-similar and functionally identical to a naturally-occurring substance and extracts from biological sources that control pests or provide other crop protection uses as defined below, but have non-toxic mode of actions (e.g. growth or developmental regulation, attractants, repellents or defense activators (e.g. induced resistance) and are relatively non-toxic to mammals.

The following list of biopesticides, in conjunction with which the compounds of the invention can be used, illustrates the possible combinations:

### L) Biopesticides

L1) Microbial pesticides with fungicidal, bactericidal, viricidal and/or plant defense activator activity: Ampelomyces quisqualis, Aspergillus flavus, Aureobasidium pullulans, Bacillus altitudinis, B. amyloliquefaciens, B. amyloliquefaciens ssp. plantarum (B. velezensis), B. megaterium, B. mojavensis, B. mycoides, B. pum¬lus, B. simplex, B. solisalsi, B. subtilis, B. subtilis var. amyloliquefaciens, B. velezensis, Candida oleo¬phila, C. saitoana, Clavibacter michiga¬nensis (bacteriophages), Coniothyrium minitans, Cryphonectria parasitica, Cryptococcus albidus, Dilophosphora alopecuri, Fusarium oxysporum, Clonostachys rosea f. catenulate (Gliocladium catenulatum), Gliocladium roseum, Lysobacter antibioticus, L. enzymogenes, Metschnikowia fructi¬cola, Microdochium dimerum, Microsphaeropsis ochracea, Muscodor albus, Paeni-bacillus alvei, Paenibacillus epiphyticus, P. polymyxa, Pantoea vagans, Penicillium bilaiae, Phlebiopsis gigantea, Pseudomonas sp., Pseudomonas chloraphis, Pseudo¬zyma flocculosa, Pichia anomala, Pythium oligandrum, Sphaerodes myco¬parasitica, Streptomyces griseoviridis, S. lydicus, S. violaceus¬niger, Talaromyces flavus, Tricho¬derma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia, zucchini yellow mosaic virus (avirulent strain);
L2) Biochemical pesticides with fungicidal, bactericidal, viricidal and/or plant defense activator activity: harpin protein, Reynoutria sachalinensis extract;
L3) Microbial pesticides with insecticidal, acaricidal, molluscidal and/or nematicidal activity: Agrobacterium radiobacter, Bacillus cereus, B. firmus, B. thuringiensis, B. thuringiensis ssp. aizawai, B. t. ssp. israelensis, B. t. ssp. galleriae, B. t. ssp. kurstaki, B. t. ssp. tene¬brionis, Beauveria bassiana, B. brongniartii, Burkholderia spp., Chromobacterium sub¬tsugae, Cydia pomonella granulovirus (CpGV), Cryptophlebia leucotreta granulovirus (CrleGV), Flavobacte-rium spp., Helicoverpa armigera nucleopolyhedrovirus (HearNPV), Helicoverpa zea nucleopolyhedrovirus (HzNPV), Helicoverpa zea single capsid nucleopolyhedrovirus (HzSNPV), Heterorhabditis bacteriophora, Isaria fumoso¬rosea, Lecanicillium longispo¬rum, L. muscarium, Metarhizium anisopliae, M. anisopliae var. anisopliae, M. anisopliae var. acridum, Nomuraea rileyi, Paecilomyces fumoso¬roseus, P. lilacinus, Paenibacillus popilliae, Pasteuria spp., P. nishizawae, P. pene¬trans, P. ramosa, P. thornea, P. usgae, Pseudomonas fluorescens, Spodoptera litto¬ralis nucleopoly¬hedrovirus (SpliNPV), Steinernema carpocapsae, S. feltiae, S. kraussei, Streptomyces galbus, S. microflavus;
L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl buty¬rate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadeca¬dien-1-ol acetate, (E,Z)-3,13-octadecadien-1-ol, (R)-1-octen-3-ol, pentatermanone, (E,Z,Z) 3,8,11-tetradecatrienyl acetate, (Z,E) 9,12-tetra¬de¬cadien-1-yl acetate, (Z) 7 tetradecen-2-one, (Z)-9-tetradecen-1-yl acetate, (Z)-11-tetradece¬nal, (Z)-11-tetra¬decen-¬-ol, extract of Chenopodium ambrosiodes, Neem oil, Quillay extract;
L5) Microbial pesticides with plant stress reducing, plant growth regulator, plant growth promoting and/or yield enhancing activity: Azospirillum amazonense, A. brasilense, A. lipoferum, A. irakense, A. halopraeferens, Bradyrhizobium spp., B. elkanii, B. japo¬ni¬cum, B. liaoningense, B. lupini, Delftia acidovorans, Glomus intraradices, Mesorhizo¬bium spp., Rhizobium leguminosarum bv. pha¬seoli, R. I. bv. trifolii, R. I. bv. viciae, R. tropici, Sinorhizobium meliloti;

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

Many of these biopesticides have been deposited under deposition numbers mentioned herein (the prefices e.g. ATCC or DSM refer to the acronym of the respective culture collection, for details see e.g. here: http://www. wfcc.info/ccinfo/collection/by_acronymn, are referred to in literature, registered and/or are commercially available: mixtures of Aureobasidium pullulans DSM 14940 and DSM 14941 isolated in 1989 in Konstanz, Germany (e.g. blastospores in Blossom-Protect^{®} from bio-ferm GmbH, Austria), Azospirillum brasilense Sp245 originally isolated in wheat reagion of South Brazil (Passo Fundo) at least prior to 1980 (BR 11005; e.g. GELFIX^{®} Gramíneas from BASF Agricultural Specialties Ltd., Brazil), A. brasilense strains Ab-V5 and Ab-V6 (e.g. in AzoMax from Novozymes BioAg Produtos papra Agricultura Ltda., Quattro Barras, Brazil or Simbiose-Maíz^{®} from Simbiose-Agro, Brazil; Plant Soil 331, 413-425, 2010), Bacillus amyloliquefaciens strain AP-188 (NRRL B-50615 and B-50331; US8,445,255); B. amylo-liquefaciens ssp. plantarum strains formerly also sometimes referred to as B. subtilis, recently together with B. methylotrophicus, and B. velezensis classified as B. velezensis (Int. J. Syst. Evol. Microbiol. 66, 1212-1217, 2016): B. a. ssp. plantarum or B. velezensis D747 isolated from air in Kikugawashi, Japan (US 20130236522 A1; FERM BP 8234; e.g. Double Nickel^{™} 55 WDG from Certis LLC, USA), B. a. ssp. plantarum or B. velezensis FZB24 isolated from soil in Brandenburg, Germany (also called SB3615; DSM 96-2; J. Plant Dis. Prot. 105, 181-197, 1998; e.g. Taegro^{®} from Novozyme Biologicals, Inc., USA), B. a. ssp. plantarum or B. velezensis FZB42 isolated from soil in Brandenburg, Germany (DSM 23117; J. Plant Dis. Prot. 105, 181-197, 1998; e.g. RhizoVital^{®} 42 from AbiTEP GmbH, Germany), B. a. ssp. plantarum or B. velezensis MBI600 isolated from faba bean in Sutton Bonington, Nottinghamshire, U.K. at least before 1988 (also called 1430; NRRL B 50595; US 2012/0149571 A1; e.g. Integral^{®} from BASF Corp., USA), B. a. ssp. plantarum or B. velezensis QST-713 isolated from peach orchard in 1995 in California, U.S.A. (NRRL B 21661; e.g. Serenade^{®} MAX from Bayer Crop Science LP, USA), B. a. ssp. plantarum or B. velezensis TJ1000 isolated in 1992 in South Dakoda, U.S.A. (also called 1BE; ATCC BAA-390; CA 2471555 A1; e.g. QuickRoots^{™} from TJ Technologies, Watertown, SD, USA); B. firmus CNCM 1-1582, a variant of parental strain EIP-N1 (CNCM I-1556) isolated from soil of central plain area of Israel (WO 2009/126473, US6,406,690; e.g. Votivo^{®} from Bayer CropScience LP, USA), B. pumilus GHA 180 isolated from apple tree rhizo-sphere in Mexico (IDAC 260707-01; e.g. PRO-MIX^{®} BX from Premier Horticulture, Quebec, Canada), B. pumilus INR-7 otherwise referred to as BU F22 and BU-F33 isolated at least be-fore 1993 from cucumber infested by Erwinia tracheiphila (NRRL B-50185, NRRL B-50153; US 8,445,255), B. pumilus KFP9F isolated from the rhizosphere of grasses in South Africa at least before 2008 (NRRL B-50754; WO 2014/029697; e.g. BAC-UP or FUSION-P from BASF Agricultural Specialities (Pty) Ltd., South Africa), B. pumilus QST 2808 was isolated from soil collected in Pohnpei, Federated States of Micronesia, in 1998 (NRRL B 30087; e.g. Sonata^{®} or Ballad^{®} Plus from Bayer Crop Science LP, USA), B. simplex ABU 288 (NRRL B-50304; US8,445,255), B. subtilis FB17 also called UD 1022 or UD10-22 isolated from red beet roots in North America (ATCC PTA-11857; System. Appl. Microbiol. 27, 372-379, 2004; US2010/0260735; WO 2011/109395); B. thurin¬giensis ssp. aizawai ABTS-1857 isolated from soil taken from a lawn in Ephraim, Wisconsin, U.S.A., in 1987 (also called ABG 6346; ATCC SD-1372; e.g. XenTari^{®} from BioFa AG, Munsingen, Germany), B. t. ssp. kurstaki ABTS-351 identical to HD-1 isolated in 1967 from diseased Pink Bollworm black larvae in Brownsville, Texas, U.S.A. (ATCC SD-1275; e.g. Dipel^{®} DF from Valent BioSciences, IL, USA), B. t. ssp. kurstaki SB4 isolated from E. saccharina larval cadavers (NRRL B-50753; e.g. Beta Pro^{®} from BASF Agricultural Specialities (Pty) Ltd., South Africa), B. t. ssp. tenebrionis NB-176-1, a mutant of strain NB-125, a wild type strain isolated in 1982 from a dead pupa of the beetle Tenebrio molitor (DSM 5480; EP 585 215 B1; e.g. Novodor^{®} from Valent BioSciences, Switzerland), Beauveria bassiana GHA (ATCC 74250; e.g. BotaniGard^{®} 22WGP from Laverlam Int. Corp., USA), B. bassiana JW-1 (ATCC 74040; e.g. Naturalis^{®} from CBC (Europe) S.r.l., Italy), B. bassiana PPRI 5339 isolated from the larva of the tortoise beetle Conchyloctenia punctata (NRRL 50757; e.g. BroadBand^{®} from BASF Agricultural Specialities (Pty) Ltd., South Africa), Brady¬rhizobium elkanii strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e.g. GELFIX 5 from BASF Agricultural Specialties Ltd., Brazil), B. japonicum 532c isolated from Wisconsin field in U.S.A. (Nitragin 61A152; Can. J. Plant. Sci. 70, 661-666, 1990; e.g. in Rhizoflo^{®}, Histick^{®}, Hicoat^{®} Super from BASF Agricultural Specialties Ltd., Canada), B. japonicum E-109 variant of strain USDA 138 (INTA E109, SEMIA 5085; Eur. J. Soil Biol. 45, 28-35, 2009; Biol. Fertil. Soils 47, 81-89, 2011); B. japonicum strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e.g. GELFIX 5 or ADHERE 60 from BASF Agricultural Specialties Ltd., Brazil), B. japonicum SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e.g. GELFIX 5 or ADHERE 60 from BASF Agricultural Specialties Ltd., Brazil); Burkholderia sp. A396 isolated from soil in Nikko, Japan, in 2008 (NRRL B-50319; WO 2013/032693; Marrone Bio Innovations, Inc., USA), Coniothyrium minitans CON/M/91-08 isolated from oilseed rape (WO 1996/021358; DSM 9660; e.g. Contans^{®} WG, Intercept^{®} WG from Bayer CropScience AG, Germany), harpin (alpha-beta) protein (Science 257, 85-88, 1992; e.g. Messenger^{™} or HARP-N Tek from Plant Health Care plc, U.K.), Helicoverpa armigera nucleopolyhedrovirus (HearNPV) (J. Invertebrate Pathol. 107, 112-126, 2011; e.g. Helicovex^{®} from Adermatt Biocontrol, Switzerland; Diplomata^{®} from Koppert, Brazil; Vivus^{®} Max from AgBiTech Pty Ltd., Queensland, Australia), Helicoverpa zea single capsid nucleopolyhedrovirus (HzSNPV) (e.g. Gemstar^{®} from Certis LLC, USA), Helicoverpa zea nucleopolyhedrovirus ABA-NPV-U (e.g. Heligen^{®} from AgBiTech Pty Ltd., Queensland, Australia), Heterorhabditis bacteriophora (e.g. Nemasys^{®} G from BASF Agricultural Specialities Limited, UK), Isaria fumosorosea Apopka-97 isolated from mealy bug on gynura in Apopka, Florida, U.S.A. (ATCC 20874; Biocontrol Science Technol. 22(7), 747-761, 2012; e.g. PFR-97^{™} or PreFeRal^{®} from Certis LLC, USA), Metarhizium anisopliae var. anisopliae F52 also called 275 or V275 isolated from codling moth in Austria (DSM 3884, ATCC 90448; e.g. Met52^{®} Novozymes Biologicals BioAg Group, Canada), Metschnikowia fructicola 277 isolated from grapes in the central part of Israel (US 6,994,849; NRRL Y-30752; e.g. formerly Shemer^{®} from Agrogreen, Israel), Paecilomyces ilacinus 251 isolated from infected nematode eggs in the Philippines (AGAL 89/030550; WO1991/02051; Crop Protection 27, 352-361, 2008; e.g. BioAct^{®}from Bayer CropScience AG, Germany and MeloCon^{®} from Certis, USA), Paenibacillus alvei NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), Paenibacillus strains isolated from soil samples from a variety of European locations including Germany: P. epiphyticus Lu17015 (WO 2016/020371; DSM 26971), P. polymyxa ssp. plantarum Lu16774 (WO 2016/020371; DSM 26969), P. p. ssp. plantarum strain Lu17007 (WO 2016/020371; DSM 26970); Pasteuria nishizawae Pn1 isolated from a soybean field in the mid-2000s in Illinois, U.S.A. (ATCC SD 5833; Federal Register 76(22), 5808, February 2, 2011; e.g. Clariva^{™} PN from Syngenta Crop Protection, LLC, USA), Penicillium bilaiae (also called P. bilaii) strains ATCC 18309 (= ATCC 74319), ATCC 20851 and/or ATCC 22348 (=ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e.g. Jump Start^{®}, Provide^{®} from Novozymes Biologicals BioAg Group, Canada), Reynoutria sachalinensis extract (EP 0307510 B1; e.g. Regalia^{®} SC from Marrone Biolnnovations, Davis, CA, USA or Milsana^{®} from BioFa AG, Germany), Steinernema carpocapsae (e.g. Millenium^{®} from BASF Agricultural Specialities Limited, UK), S. feltiae (e.g. Nemashield^{®} from BioWorks, Inc., USA; Nemasys^{®} from BASF Agricultural Specialities Limited, UK), Streptomyces microflavus NRRL B-50550 (WO 2014/124369; Bayer CropScience, Germany), Trichoderma asperelloides JM41R isolated in South Africa (NRRL 50759; also referred to as T. fertile; e.g. Trichoplus^{®} from BASF Agricultural Specialities (Pty) Ltd., South Africa), T. harzianum T-22 also called KRL-AG2 (ATCC 20847; BioControl 57, 687-696, 2012; e.g. Plantshield^{®} from BioWorks Inc., USA or SabrEx^{™} from Advanced Biological Marketing Inc., Van Wert, OH, USA).

According to the invention, the solid material (dry matter) of the biopesticides (with the ex-ception of oils e.g. Neem oil) are considered as active components (e.g. to be obtained after drying or evaporation of the extraction or suspension medium in case of liquid formulations of the microbial pesticides).

In accordance with the invention, the weight ratios and percentages used herein for a biological extract e.g. Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1x1010 CFU equals one gram of total weight of the respective active component. Colony forming unit is measure of viable microbial cells, in particular fungal and bacterial cells. In addition, here "CFU" may also be understood as the number of (juvenile) individual nematodes in case of (entomo¬pathogenic) nematode biopesticides, e.g. Steinernema feltiae.

When mixtures comprising microbial pesticides are employed in crop protection, the application rates range from 1x106 to 5x1016 (or more) CFU/ha, preferably from 1x108 to 1x1013 CFU/ha, and even more preferably from 1x109 to 5x1015 CFU/ha and in particular from 1x1012 to 5x1014 CFU/ha. In the case of nematodes as microbial pesticides (e.g. Steinernema feltiae), the application rates regularly range from 1x105 to 1x1012 (or more), preferably from 1x108 to 1x1011, more preferably from 5x108 to 1x1010 individuals (e.g. in the form of eggs, juvenile or any other live stages, preferably in an infetive juvenile stage) per ha.

When mixtures comprising microbial pesticides are employed in seed treatment, the application rates generally range from 1x106 to 1x1012 (or more) CFU/seed, preferably from 1x106 to 1x109 CFU/seed. Furthermore, the application rates with respect to seed treatment generally range from 1x107 to 1x1014 (or more) CFU per 100 kg of seed, preferably from 1x109 to 1x1012 CFU per 100 kg of seed.

The invention also relates to agrochemical compositions comprising an auxiliary and at least one compound of the invention or a mixture thereof.

An agrochemical composition comprises a pesticidally effective amount of a compound of the invention or a mixture thereof.

The compounds of the invention or the mixtures thereof can be converted into customary types of agro-chemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials e.g. seeds (e.g. GF). These and further compositions types are defined in the "Catalogue of pesticide formulation types and international coding system", Technical Monograph No. 2, 6th Ed. May 2008, CropLife International.

The compositions are prepared in a known manner, e.g. described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, e.g. mineral oil fractions of medium to high boiling point, e.g. kerosene, diesel oil; oils of vegetable or animal origin; hydrocarbons, e.g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, CaSO₄, MgSO₄, MgO; polysaccharide powders, e.g. cellulose, starch; fertilizers, e.g. (NH₄)2SO₄, (NH₄)3PO₄, NH₄NO₃, ureas; products of vegetable origin, e.g. cereal meal, tree bark meal, wood meal, nutshell meal, and mixtures thereof.

Suitable surfactants are surface-active compounds, e.g. anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds e.g. alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are homo- or copolymers of vinylpyrrolidone, vinylalcohols, or vinylacetate.

Suitable cationic surfactants are quaternary surfactants, e.g. quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines, or polyethyleneamines.

Suitable adjuvants are compounds, which have a neglectable or even no pesticidal activity themselves, and which improve the biological performance of the compounds of the invention on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives e.g. alkylisothiazolinones and benzisothiazolinones.

Suitable anti-freezing agents are ethylene glycol, propylene glycol, urea, and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo-, and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.
ii) Dispersible concentrates (DC)
   5-25 wt% of a compound I according to the invention and 1-10 wt% dispersant (e.g. polyvinylpyrrolidone) are dissolved in up to 100 wt% organic solvent (e.g. cyclohexanone). Dilution with water gives a dispersion.
iii) Emulsifiable concentrates (EC)
   15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.
iv) Emulsions (EW, EO, ES)
   5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.
v) Suspensions (SC, OD, FS)
   In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e.g. extrusion, spray tower, fluidized bed). Dilution with water gives a stable dispersion or solution of the active substance.
vii) Water-dispersible powders and water-soluble powders (WP, SP, WS)
   50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.
viii) Gel (GW, GF)
   In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active substance. Dilution with water gives a stable suspension of the active substance.
ix) Microemulsion (ME)
   5-20 wt% of a compound I according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water up to 100 %. This mixture is stirred for 1 h to produce spontaneously a thermodynamically stable microemulsion.
x) Microcapsules (CS)
   An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. di-phenylme-thene-4,4'-diisocyanatae) are dispersed into an aqueous solution of a protective colloid (e.g. polyvinyl alcohol). The addition of a polyamine (e.g. hexamethylenediamine) results in the foration of a polyurea microcapsule. The monomers amount to 1-10 wt%. The wt% relate to the total CS composition.
xi) Dustable powders (DP, DS)
   1-10 wt% of a compound I according to the invention are ground finely and mixed intimately with up to 100 wt% solid carrier, e.g. finely divided kaolin.
xii) Granules (GR, FG)
   0.5-30 wt% of a compound I according to the invention is ground finely and associated with up to 100 wt% solid carrier (e.g. silicate). Granulation is achieved by extrusion, spray-drying or the fluidized bed.
xiii) Ultra-low volume liquids (UL)
   1-50 wt% of a compound I according to the invention are dissolved in up to 100 wt% organic solvent, e.g. aromatic hydrocarbon.

The compositions types i) to xi) may optionally comprise further auxiliaries, e.g. 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and most preferably between 0.5 and 75%, by weight of active substance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition of the invention e.g. parts of a kit or parts of a binary or ternary mixture may be mixed by the user himself in a spray tank and further auxiliaries may be added, if appropriate.

In a further embodiment, either individual components of the composition according to the invention or partially premixed components, e.g. components comprising compounds of the invention and/or mixing partners as defined above, may be mixed by the user in a spray tank and further auxiliaries and additives may be added.

In a further embodiment, either individual components of the composition according to the invention or partially premixed components, e.g. components comprising compounds of the invention and/or mixing partners as defined above, can be applied jointly (e.g. after tank mix) or consecutively.

The compounds of the invention are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound of the invention.

The compounds of the invention are also suitable for use in combating or controlling animal pests. Therefore, the invention also relates to a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound of the invention.

The compounds of the invention are effective through both contact and ingestion. Furthermore, the compounds of the invention can be applied to any and all developmental stages, e.g. egg, larva, pupa, and adult.

The compounds of the invention can be applied as such or in form of compositions comprising them as defined above. Furthermore, the compounds of the invention can be applied together with a mixing partner or in form of compositions comprising said mixtures. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, e.g. seeds, soil, or the area, material or environment by the pests. Suitable application methods include i.a. soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the invention. Suitable pheromones for specific crops and pests are known and publicly available from databases of pheromones and semiochemicals, e.g. http://www.pherobase.com.

As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material, or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grapefruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, shrubs, broad-leaved trees or evergreens, eucalyptus; turf; lawn; grass. Preferred plants include potatoes sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "cultivated plants" is to be understood as including plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to ALS inhibitor herbicides by conventional methods of mutagenesis and breeding comprise plant varieties commercially available under the name Clearfield^{®}.

Herbicide tolerance has been created to glyphosate, glufosinate, 2,4-D, dicamba, oxynil herbicides, like bromoxynil and ioxynil, sulfonylurea herbicides, ALS inhibitor herbicides and HPPD inhibitors, like isoxaflutole and mesotrione.

Transgenes which have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are e.g., but not excluding others, DAS40278, MON801, MON802, MON809, MON810, MON832, MON87411, MON87419, MON87427, MON88017, MON89034, NK603, GA21, MZHG0JG, HCEM485, VCO-∅1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are e.g., but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHT∅H2, W62, W98, FG72 and CV127.

Transgenic cotton events comprising herbicide tolerance genes are e.g., but not excluding others, 19-51 a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are e.g., but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of Bacillus spec. and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate in-sect genes. An example for such a transgene is dvsnf7.

Transgenic corn events comprising genes for insecticidal proteins or double stranded RNA are e.g., but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are e.g., but not excluding others, MON87701, MON87751 and DAS-81419.

Transgenic cotton events comprising genes for insecticidal proteins are e.g., but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Cultivated plants comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are known (http://www.isaaa.org/gmapprovaldatabase) and (http://ceragmc. org/GMCropDatabase).

Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531(MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527, for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571, for potato events E12, F10, J3, J55, V11, X17, Y9 in WO14/178910, WO14/178913, WO14/178941, WO14/179276, WO16/183445, WO17/062831, WO17/062825, for rice events LLRICE06, LLRICE601, LLRICE62 in WO00/026345, WO00/026356, WO00/026345 for soybean events H7-1, MON89788, A2704-12, A5547-127, DP305423, DP356043, MON87701, MON87769, CV127, MON87705, DAS68416-4, MON87708, MON87712, SYHTOH2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compositions according to the invention on cultivated plants may result in effects which are specific to a cultivated plant comprising a certain gene or event. These effects may comprise enhanced yield, enhanced resistance or tolerance to insects, nematodes, fungal, bacterial, mycoplasma, viral or viroid pathogens as well as early vigor, early or delayed ripening, cold or heat tolerance as well as changed amino acid or fatty acid spectrum or content.

It has been found that the pesticidal activity of the compounds of the invention may be enhanced by the insecticidal trait of a modified plant. Furthermore, it has been found that the compounds of the invention are suitable for preventing insects to become resistant to the insecticidal trait or for combating pests, which already have become resistant to the insecticidal trait of a modified plant. Moreover, the compounds of the invention are suitable for combating pests, against which the insecticidal trait is not effective, so that a complementary insecticidal activity can advantageously be used.

The term "plant propagation material" refers to all the generative parts of the plant e.g. seeds and vegetative plant material e.g. cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be trans-planted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises e.g. seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is e.g. seed of cereals, root crops, oil crops, vegetables, spices, ornamentals, e.g. seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants e.g. potatoes, sugar cane, tobacco, grapes, petunias, geranium/pelargoniums, pansies and impatiens.

In addition, the active compound may also be used for the treatment of seeds from plants, which have been modified by mutagenisis or genetic engineering, and which e.g. tolerate the action of herbicides or fungicides or insecticides.

Conventional seed treatment formulations include e.g. flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40% by weight.

In a preferred embodiment a FS formulation is used for seed treatment. Typically, a FS formulation may comprise 1-800g/l of active ingredient, 1-200g/l Surfactant, 0 to 200g/l anti-freezing agent, 0 to 400g/l of binder, 0 to 200g/l of a pigment and up to 1 liter of a solvent, preferably water.

Especially preferred FS formulations of the compounds of the invention for seed treatment usually comprise from 0.1 to 80% by weight (1 to 800g/l) of the active ingredient, from 0.1 to 20% by weight (1 to 200g/l) of at least one surfactant, e.g. 0.05 to 5% by weight of a wetter and from 0.5 to 15% by weight of a dispersing agent, up to 20% by weight, e.g. from 5 to 20% of an anti-freeze agent, from 0 to 15% by weight, e.g. 1 to 15% by weight of a pigment and/or a dye, from 0 to 40% by weight, e.g. 1 to 40% by weight of a binder (sticker/adhesion agent), optionally up to 5% by weight, e.g. from 0.1 to 5% by weight of a thickener, optionally from 0.1 to 2% of an anti-foam agent, and optionally a preservative e.g. a biocide, antioxidant or the like, e.g. in an amount from 0.01 to 1% by weight and a filler/vehicle up to 100% by weight.

In the treatment of seed, the application rates of the compounds of the invention are generally from 0.1 g to 10kg per 100kg of seed, preferably from 1 g to 5kg per 100kg of seed, more preferably from 1 g to 1000g per 100kg of seed and in particular from 1g to 200g per 100kg of seed, e.g. from 1 g to 100g or from 5g to 100g per 100kg of seed.

The invention therefore also relates to seed comprising a compound of the invention, or an agriculturally useful salt thereof, as defined herein. The amount of the compound of the invention or the agriculturally useful salt thereof will in general vary from 0.1g to 10kg per 100kg of seed, preferably from 1g to 5kg per 100kg of seed, in particular from 1g to 1000g per 100kg of seed. For specific crops e.g. lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect but which does not give rise to any phytotoxic symptom on the treated plant or on the plant grown from the treated propagule or treated soil.

"Plant health" is defined as a condition of the plant and/or its products which is determined by several aspects alone or in combination with each other e.g. yield (e.g. increased biomass and/or increased content of valuable ingredients), quality (e.g. improved content or composition of certain ingredients or shelf life), plant vigor (e.g. improved plant growth and/or greener leaves ("greening effect"), tolerance to abiotic (e.g. drought) and/or biotic stress (e.g. disease) and production efficiency (e.g., harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, e.g. ants, termites, wasps, flies, ticks, mosquitoes, bed bugs, crickets, or cockroaches.

The bait can be a liquid, a solid or a semisolid preparation (e.g. a gel). The bait employed in the composition is a product, which is sufficiently attractive to incite insects e.g. ants, termites, wasps, flies, mosquitoes, crickets etc. or cockroaches to eat it. The attractiveness can be manipulated by using feeding stimulants or sex pheromones. Food stimulants are preferably chosen from animal and/or plant proteins (meat-, fish- or blood meal, insect parts, egg yolk), from fats and oils of animal and/or plant origin, or mono-, oligo- or polyorgano¬saccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are known (http://www.pherobase.com).

For use in bait compositions, the typical content of active ingredient is from 0.001wt% to 15wt%, desirably from 0.001wt% to 5wt% of active compound.

Formulations of the compounds of the invention as aerosols (e.g. in spray cans), oil sprays or pump sprays are highly suitable for professional or non-professional users for controlling pests e.g. flies, fleas, ticks, bed bugs, mosquitoes or cockroaches. Aerosol recipes are preferably composed of the active compound, solvents, furthermore auxiliaries e.g. emulsifiers, perfume oils, if appropriate stabilizers, and, if required, propellants.

The oil spray formulations differ from the aerosol recipes in that no propellants are used.

For use in spray compositions, the content of active ingredient is from 0.001 to 80wt%, preferably from 0.01 to 50wt% and most preferably from 0.01 to 15wt%.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the invention and its respective compositions also comprise treating surfaces of huts and houses, air spraying and impregnation of curtains, tents, clothing items, bed nets, tsetse-fly trap. Insecticidal compositions for application to fibers, fabric, knitgoods, nonwovens, netting material or foils and tarpaulins preferably comprise a mixture including the insecticide, optionally a repellent and at least one binder.

The compounds of the invention and its compositions can be used for protecting wooden materials e.g. trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000g or from 0.01g to 1000g of active compound per m² treated material, desirably from 0.1g to 50g per m²_{.}

Insecticidal compositions for use in the impregnation of materials typically contain from 0.001 to 95wt%, preferably from 0.1 to 45wt%, and more preferably from 1 to 25wt% of at least one repellent and/or insecticide.

The compounds of the invention are especially suitable for efficiently combating animal pests e.g. arthropods, gastropods and nematodes including:
insects from the order of Lepidoptera, e.g. Achroia grisella, Acleris spp. e.g. A. fimbriana, A. gloverana, A. variana; Acrolepiopsis assectella, Acronicta major, Adoxophyes spp. e.g. A. cyrtosema, A. orana; Aedia leucomelas, Agrotis spp. e.g. A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Ther¬me¬sia) spp. e.g. A. gemmatalis; Apamea spp., Aproaerema modicella, Archips spp. e.g. A. argyrospila, A. fuscocupreanus, A. rosana, A. xyloseanus; Argyresthia conjugella, Argyroplo¬ce spp., Argyrotaenia spp. e.g. A. velutinana; Athetis mindara, Austroasca virid¬gri¬sea, Autographa gamma, Autographa nigrisigna, Barathra brassicae, Bedellia spp., Bonagota salubricola, Borbo cinnara, Bucculatrix thurberiella, Bupalus piniarius, Busseola spp., Cacoecia spp. e.g. C. murinana, C. podana; Cactoblastis cactorum, Cadra cautella, Calingo brazilien¬sis, Caloptilis theivora, Capua reticulana, Carposina spp. e.g. C. niponensis, C. sasakii; Cephus spp., Chaetocnema aridula, Cheimatobia brumata, Chilo spp. e.g. C. Indicus, C. suppressalis, C. partellus; Choreutis pariana, Choristoneura spp. e.g. C. conflictana, C. fumife¬rana, C. longicellana, C. murinana, C. occidentalis, C. rosaceana; Chrysodeixis (=Pseudoplusia) spp., e.g. C. eriosoma, C. includens; Cirphis unipuncta, Clysia ambiguella, Cnaphalocerus spp., Cna¬phalocrocis medinalis, Cnephasia spp., Cochylis hospes, Coleophora spp., Colias eurytheme, Conopomorpha spp., Conotrachelus spp., Copitarsia spp., Corcyra cephalonica, Crambus caliginosellus, Crambus teterrellus, Crocidosema (=Epinotia) aporema, Cydalima (=Diaphania) perspectalis, Cydia (=Carpocapsa) spp., e.g. C. pomonella, C. latiferreana; Dalaca noctuides, Datana integerrima, Dasychira pinicola, Dendrolimus spp., e.g. D. pini, D. spectabilis, D. sibiricus; Desmia funeralis, Diaphania spp., e.g. D. nitidalis, D. hyalinata; Diatraea grandiosella, Diatraea saccharalis, Diphthera festiva, Earias spp. e.g. E. insulana, E. vittella; Ecdytolopha aurantianu, Egira (=Xylomyges) curialis, Elasmopalpus lignosellus, Eldana saccharina, Endo¬pi¬za viteana, Ennomos subsignaria, Eoreuma loftini, Ephestia spp., e.g. E. cautella, E. elutella, E. kuehniella; Epinotia aporema, Epi¬phy¬as postvittana, Erannis tiliaria, Erio¬nota thrax, Etiella spp., Eulia spp., Eupoecilia ambiguella, Euproctis chrysorrhoea, Euxoa spp., Eve¬tria bouliana, Faronta albilinea, Feltia spp. e.g. F. subterranean; Galleria mellonella, Gra¬cillaria spp., Gra¬pholita spp. e.g. G. funebrana, G. molesta, G. inopinata; Halysidota spp., Harrisina americana, Hedylepta spp., Helicoverpa spp. e.g. H. armigera (=Heliothis armigera), H. zea (=Heliothis zea); Heliothis spp. e.g. H. assulta, H. subflexa, H. virescens; Hellula spp. e.g. H. undalis, H. rogatalis; Helocoverpa gelotopoeon, Hemileuca oliviae, Herpetogramma licarsisalis, Hibernia defoliaria, Hofmannophila pseudospretella, Homo¬eosoma electellum, Homona magna¬nima, Hypena scabra, Hyphantria cunea, Hyponomeuta padella, Hypono¬meuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora gly¬cinivorella, Lerodea eufala, Leucinodes orbonalis, Leucoma salicis, Leucoptera spp. e.g. L. coffeella, L. scitella; Leuminivora lycinivorella, Lithocolletis blancardella, Lithophane antennata, Llattia octo (=Amyna axis), Lobesia botrana, Lophocampa spp., Loxagrotis albicosta, Loxostege spp. e.g. L. sticticalis, L. cereralis; Lymantria spp., e.g. L. dispar, L. monacha; Lyone¬tia clerkella, Lyonetia prunifoliella, Malacosoma spp., e.g. M. americanum, M. californicum, M. constrictum, M. neu-stria; Mamestra spp., e.g. M. brassicae, M. configurata; Mamstra brassicae, Manduca spp. e.g. M. quinquemaculata, M. sexta; Marasmia spp, Marmara spp., Maruca testulalis, Megalopyge lanata, Melanchra picta, Melanitis leda, Mocis spp., e.g. M. lapites, M. repan¬da; Mocis latipes, Monochroa fragariae, Mythimna separata, Nemapogon cloacella, Neoleucino¬des elegantalis, Nepytia spp., Nymphula spp., Oiketicus spp., Omiodes indicata, Omphisa anastomosalis, Operophtera brumata, Orgyia pseudotsugata, Oria spp., Ortha¬ga thyrisalis, Ostrinia spp. e.g. O. nubilalis; Oulema oryzae, Paleacrita vernata, Panolis flammea, Parnara spp., Papa¬ipema nebris, Papilio cresphontes, Paramyelois transitella, Paranthrene regalis, Paysan¬disia archon, Pectinophora spp. e.g. P. gossypiella; Peridroma saucia, Perileucoptera spp., e.g. P. coffeella; Phalera bucephala, Phryganidia californica, Phtho¬rimaea spp. e.g. P. operculella; Phyllocnistis citrella, Phyllonorycter spp. e.g. P. blancardella, P. crataegella, P. issikii, P. ringoniella; Pieris spp. e.g. P. brassicae, P. rapae, P. napi; Pilocrocis tripunctata, Plathypena scabra, Pla¬tynota spp. e.g. P. flavedana, P. idaeusalis, P. stultana; Platyptilia carduidactyla, Plebejus argus, Plodia interpunctella, Plusia spp, Plutella maculipennis, Plutella xylostella, Pontia proto¬di¬ca, Prays spp., Prodenia spp., Proxenus lepigone, Pseudaletia spp. e.g. P. sequax, P. un¬punc¬ta; Pyrausta nubilalis, Rachiplusia nu, Richia albicosta, Rhizobius ventralis, Rhyacionia frustra¬na, Sabulodes aegrotata, Schizura concinna, Schoenobius spp., Schreckensteinia festaliella, Scirpophaga spp. e.g. S. incertulas, S. innotata; Scotia sege¬tum, Sesamia spp. e.g. S. inferens, Seudyra subflava, Sitotroga cerealella, Sparganothis pilleriana, Spilonota lechriaspis, S. ocelli-na, Spodoptera (=Lamphygma) spp. e.g. S. cosmoides, S. eridania, S. exigua, S. frugiperda, S. latisfascia, S. littoralis, S. litura, S. omithogalli; Stigmella spp., Stomopteryx subsecivella, Strymon bazochii, Sylepta dero¬gata, Synanthedon spp. e.g. S. exitiosa, Tecia solanivora, Telehin licus, Thaumatopoea pityocampa, Thaumatotibia (=Cryptophlebia) leucotreta, Thaumetopoea pityocampa, Thecla spp., Theresimima ampelophaga, Thyrinteina spp, Tildenia inconspi¬cuella, Tinea spp. e.g. T. cloacella, T. pellionella; Tineola bisselliella, Tortrix spp. e.g. T. virida¬na; Trichophaga tapetzella, Trichoplusia spp. e.g. T. ni; Tuta (=Scrobipalpula) absoluta, Udea spp. e.g. U. rubigalis, U. rubigalis; Virachola spp., Yponomeuta padella, and Zeiraphera canadensis;
insects from the order of Coleoptera, e.g. Acalymma vittatum, Acanthoscehdes obtectus, Ado¬retus spp., Agelastica alni, Agrilus spp. e.g. A. anxius, A. planipennis, A. sinuatus; Agriotes spp. e.g. A. fuscicollis, A. lineatus, A. obscurus; Alphitobius diaperinus, Amphimallus solstitialis, Anisandrus dispar, Anisoplia austriaca, Anobium punctatum, Anomala corpulen¬ta, Anoma¬la rufocuprea, Anoplophora spp. e.g. A. glabripennis; Anthonomus spp. e.g. A. eugenii, A. grandis, A. pomorum; Anthrenus spp., Aphthona euphoridae, Apion spp., Apogonia spp., Atho¬us haemorrhoidalis, Atomaria spp. e.g. A. linearis; Attagenus spp., Aulaco¬phora fe¬moralis, Blastophagus piniperda, Blitophaga undata, Bruchidius obtectus, Bruchus spp. e.g. B. lentis, B. pisorum, B. rufimanus; Byctiscus betulae, Callidiellum rufipenne, Callopistria floriden¬sis, Callosobruchus chinensis, Cameraria ohridella, Cassida nebulosa, Cerotoma trifurcata, Cetonia aurata, Ceuthorhynchus spp. e.g. C. assimilis, C. napi; Chaetocnema tibialis, Cleo¬nus mendicus, Conoderus spp. e.g. C. vespertinus; Conotrachelus nenuphar, Cosmopolites spp., Costelytra zealandica, Crioceris asparagi, Cryptolestes ferrugineus, Cryptorhynchus lapathi, Ctenicera spp. e.g. C. destructor; Curculio spp., Cylindrocopturus spp., Cyclo¬cephala spp., Dac-tylispa balyi, Dectes texanus, Dermestes spp., Diabrotica spp. e.g. D. undeci¬punctata, D. speciosa, D. longicornis, D. semipunctata, D. virgifera; Diaprepes abbreviates, Dichocrocis spp., Dicladispa armigera, Diloboderus abderus, Diocalandra frumenti (Diocalan¬dra stigmaticollis), Enaphalodes rufulus, Epilachna spp. e.g. E. varivestis, E. vigintioctomaculata; Epitrix spp. e.g. E. hirtipennis, E. similaris; Eutheola humilis, Eutinobothrus bra¬silien¬sis, Faustinus cubae, Gibbium psylloides, Gnathocerus cornutus, Hellula undalis, Heteronychus ara¬tor, Hylamorpha elegans, Hylobius abietis, Hylo¬trupes bajulus, Hypera spp., e.g. H. brunneipennis, H. postica; Hypomeces squamosus, Hypothenemus spp., Ips typographus, Lachno¬sterna consanguinea, Lasioderma serricorne, Latheticus oryzae, Lathridius spp., Lema spp. e.g. L. bilineata, L. melanopus; Leptinotarsa spp. e.g. L. decemlineata; Leptispa pygma¬ea, Limonius californi-cus, Lissorhoptrus oryzophilus, Lixus spp., Luperodes spp., Lyctus spp. e.g. L. bruneus; Liogenys fuscus, Macrodactylus spp. e.g. M. subspinosus; Mala¬de¬ra matrida, Megaplatypus mutates, Megascelis spp., Melanotus communis, Meligethes spp. e.g. M. aeneus; Melolontha spp. e.g. M. hippocastani, M. melolontha; Metamasius hemipterus, Microtheca spp., Migdolus spp. e.g. M. fryanus, Monochamus spp. e.g. M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon spp. e.g. P. brassicae, P. cochleariae; Phoracantha recurva, Phyllobius pyri, Phyllopertha horticola, Phyllophaga spp. e.g. P. helleri; Phyllotreta spp. e.g. P. chrysocephala, P. nemorum, P. striolata, P. vittula; Phyllopertha horticola, Popillia japonica, Premnotrypes spp., Psacothea hilaris, Psylliodes chrysocephala, Prostephanus truncates, Psylliodes spp., Ptinus spp., Pulga saltona, Rhizopertha dominica, Rhynchophorus spp. e.g. R. billineatus, R. ferrugineus, R. palmarum, R. phoenicis, R. vulneratus; Saperda candida, Scolytus schevyrewi, Scyphophorus acupunctatus, Sitona lineatus, Sitophilus spp. e.g. S. granaria, S. oryzae, S. zea¬mais; Sphenophorus spp. e.g. S. levis; Stegobium paniceum, Sternechus spp. e.g. S. subsignatus; Strophomorphus ctenotus, Symphyletes spp., Tanymecus spp., Tenebrio moli¬tor, Tenebrioides mauretanicus, Tribolium spp. e.g. T. castaneum; Trogoderma spp., Tychius spp., Xylo¬trechus spp. e.g. X. pyrrhoderus; and, Zabrus spp. e.g. Z. tenebrioides;
insects from the order of Diptera e.g. Aedes spp. e.g. A. aegypti, A. albopictus, A. vexans; Anastrepha ludens, Anopheles spp. e.g. A. albimanus, A. crucians, A. freeborni, A. gambiae, A. leucosphyrus, A. maculipennis, A. minimus, A. quadrimaculatus, A. sinensis; Bactro¬ce¬ra invadens, Bibio hortulanus, Calliphora erythrocephala, Calliphora vicina, Ceratitis capitata, Chrysomyia spp. e.g. C. bezziana, C. hominivorax, C. macellaria; Chrysops atlanticus, Chrysops discalis, Chrysops silacea, Cochliomyia spp. e.g. C. hominivorax; Contarinia spp. e.g. C. sorghicola; Cordylobia anthropophaga, Culex spp. e.g. C. nigripalpus, C. pipiens, C. quinquefasciatus, C. tarsalis, C. tritaeniorhynchus; Culicoides furens, Culiseta inornata, Culiseta melanura, Cuterebra spp., Dacus cucurbitae, Dacus oleae, Dasineura brassicae, Dasineura oxycoccana, Delia spp. e.g. D. antique, D. coarctata, D. platura, D. radicum; Dermatobia hominis, Drosophila spp. e.g. D. suzukii, Fannia spp. e.g. F. canicularis; Gastraphilus spp. e.g. G. intestinalis; Geomyza tipunctata, Glossina spp. e.g. G. fuscipes, G. morsitans, G. palpalis, G. tachinoides; Haematobia irritans, Haplodiplosis equestris, Hippelates spp., Hylemyia spp. e.g. H. platura; Hypoderma spp. e.g. H. lineata; Hyppobosca spp., Hydrellia philippina, Leptoconops torrens, Liriomyza spp. e.g. L. sativae, L. trifolii; Lucilia spp. e.g. L. caprina, L. cuprina, L. sericata; Lycoria pectoralis, Mansonia titillanus, Mayetiola spp. e.g. M. destructor; Musca spp. e.g. M. autumnalis, M. domestica; Muscina stabulans, Oestrus spp. e.g. O. ovis; Opomyza florum, Oscinella spp. e.g. O. frit; Orseolia oryzae, Pegomya hysocyami, Phlebotomus argentipes, Phorbia spp. e.g. P. antiqua, P. brassicae, P. coarctata; Phytomyza gymnostoma, Prosimulium mixtum, Psila rosae, Psorophora columbiae, Psorophora discolor, Rhagoletis spp. e.g. R. cerasi, R. cingulate, R. indifferens, R. mendax, R. pomonella; Rivellia quadrifasciata, Sarcophaga spp. e.g. S. haemorrhoidalis; Simulium vittatum, Sitodiplosis mosellana, Stomoxys spp. e.g. S. calcitrans; Tabanus spp. e.g. T. atratus, T. bovinus, T. lineola, T. similis; Tannia spp., Thecodiplosis japonensis, Tipula oleracea, Tipula paludosa, and Wohlfahrtia spp;
insects from the order of Thysanoptera e.g., Baliothrips biformis, Dichromothrips corbetti, Dichromothrips ssp., Echinothrips americanus, Enneothrips flavens, Frankliniella spp. e.g. F. fusca, F. occidentalis, F. tritici; Heliothrips spp., Hercinothrips femoralis, Kakothrips spp., Microcephalothrips abdominalis, Neohydatothrips samayunkur, Pezothrips kellyanus, Rhipiphorothrips cruentatus, Scirtothrips spp. e.g. S. citri, S. dorsalis, S. perseae; Stenchaetothrips spp, Taeniothrips cardamoni, Taeniothrips inconsequens, Thrips spp. e.g. T. imagines, T. hawaiiensis, T. oryzae, T. palmi, T. parvispinus, T. tabaci;
insects from the order of Hemiptera e.g., Acizzia jamatonica, Acrosternum spp., e.g. A. hilare; Acyrthosipon spp., e.g. A. onobrychis, A. pisum; Adelges laricis, Adelges tsugae, Adel¬phocoris spp., e.g. A. rapidus, A. superbus; Aeneolamia spp., Agonoscena spp., Aula¬cor¬thum solani, Aleurocanthus woglumi, Aleurodes spp., Aleurodicus disperses, Aleurolobus ba¬ro¬densis, Aleurothrixus spp., Amrasca spp., Anasa tristis, Antestiopsis spp., Anuraphis cardui, Aoni¬diella spp., Aphanostigma piri, Aphidula nasturtii, Aphis spp. e.g. A. craccivora, A. fabae, A. forbesi, A. gossypii, A. grossulariae, A. maidiradicis, A. pomi, A. sambuci, A. schnei¬deri, A. spiraecola; Arboridia apicalis, Arilus critatus, Aspidiella spp., Aspidiotus spp., Atanus spp., Aulacaspis yasumatsui, Aulacorthum solani, Bactericera cockerelli (Paratrioza cockerelli), Bemisia spp. e.g. B. argentifolii, B. tabaci (Aleurodes tabaci); Blissus spp. e.g. B. leucop¬terus; Brachycaudus spp. e.g. B. cardui, B. helichrysi, B. persicae, B. prunicola; Brachyco¬lus spp., Brachycorynella asparagi, Brevicoryne brassicae, Cacopsylla spp. e.g. C. fulgura¬lis, C. pyricola (Psylla piri); Calligy¬pona marginata, Calocoris spp., Campylomma livida, Capito¬phorus horni, Carneocephala fulgida, Cavelerius spp., Ceraplastes spp., Ceratovacuna lanigera, Ceroplastes ceriferus, Cerosi¬pha gossypii, Chaetosiphon fragaefolii, Chionaspis tega¬lensis, Chlorita onukii, Chromaphis juglandicola, Chrysomphalus ficus, Cicadulina mbila, Cimex spp. e.g. C. hemipterus, C. lectularius; Coccomytilus halli, Coccus spp. e.g. C. hesperi¬dum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus spp., Dasynus piperis, Dialeurodes spp. e.g. D. citrifolii; Dalbulus maidis, Diaphorina spp. e.g. D. citri; Diaspis spp. e.g. D. bromeliae; Dichelops furcatus, Diconocoris hewetti, Doralis spp., Dreyfusia nordmannianae, Dreyfusia piceae, Drosicha spp., Dysaphis spp. e.g. D. plan¬taginea, D. pyri, D. radicola; Dysaulacorthum pseudosolani, Dysdercus spp. e.g. D. cingulatus, D. intermedius; Dysmicoccus spp., Edessa spp., Geocoris spp., Empo¬asca spp. e.g. E. fabae, E. solana; Epidiaspis leperii, Eriosoma spp. e.g. E. lanigerum, E. pyricola; Erythroneura spp., Eurygaster spp. e.g. E. integriceps; Euscelis bilobatus, Euschistus spp. e.g. E. heros, E. impictiventris, E. servus; Fiorinia theae, Geococcus coffeae, Glycaspis brimblecombei, Halyomorpha spp. e.g. H. halys; Heliopeltis spp., Homalodisca vitripennis (=H. coagulata), Horcias nobilellus, Hyalopterus pruni, Hyperomyzus lactucae, Icerya spp. e.g. I. purchase; Idiocerus spp., Idioscopus spp., Laodelphax striatellus, Lecanium spp., Leca¬nio¬deus floccissimus, Lepidosaphes spp. e.g. L. ulmi; Leptocorisa spp., Leptoglossus phyllo¬pus, Lipaphis erysimi, Lygus spp. e.g. L. hesperus, L. lineolaris, L. praten-sis; Maconellicoccus hirsutus, Marchalina hellenica, Macropes excavatus, Macrosiphum spp. e.g. M. rosae, M. avenae, M. euphorbiae; Macrosteles quadrilineatus, Mahanarva fimbriolata, Megacopta cr¬braria, Megoura viciae, Melanaphis pyrarius, Melanaphis sacchari, Melanocallis (=Tinocallis) caryaefoliae, Metcafiella spp., Metopolophium dirhodum, Monellia costalis, Mo-nelliopsis peca¬nis, Myzocallis coryli, Murgantia spp., Myzus spp. e.g. M. ascalonicus, M. cerasi, M. nicotianae, M. persicae, M. varians; Nasonovia ribisnigri, Neotoxoptera formosana, Neomegalotomus spp, Nephotettix spp. e.g. N. malayanus, N. nigropictus, N. parvus, N. vires-cens; Ne¬zara spp. e.g. N. viridula; Nilaparvata lugens, Nysius huttoni, Oebalus spp. e.g. O. pug¬nax; Oncometopia spp., Orthezia praelonga, Oxycaraenus hyalinipennis, Parabemisia myricae, Parlatoria spp., Parthe¬nolecanium spp. e.g. P. corni, P. persicae; Pemphigus spp. e.g. P. bursarius, P. populivenae; Peregrinus maidis, Perkinsiella saccharicida, Phenacoc¬cus spp. e.g. P. aceris, P. gossypii; Phloeomyzus passerinii, Phorodon humuli, Phylloxera spp. e.g. P. devastatrix, Piesma quadrata, Piezodorus spp. e.g. P. guildinii; Pinnaspis aspidistrae, Planococcus spp. e.g. P. citri, P. ficus; Prosapia bicincta, Protopulvinaria pyriformis, Psallus seriatus, Pseudacysta persea, Pseu¬daulacaspis pentagona, Pseudococcus spp. e.g. P. com¬stocki; Psylla spp. e.g. P. mali; Pteromalus spp., Pulvinaria amygdali, Pyrilla spp., Qua¬draspidiotus spp., e.g. Q. perniciosus; Que¬sada gigas, Rastrococcus spp., Reduvius senilis, Rhizoecus americanus, Rhodnius spp., Rho¬palomyzus ascalonicus, Rhopalo¬si¬phum spp. e.g. R. pseudobrassicas, R. insertum, R. maidis, R. padi; Sagatodes spp., Sahlbergella singula¬ris, Saissetia spp., Sappaphis mala, Sappaphis mali, Scaptocoris spp., Scaphoides titanus, Schi¬zaphis graminum, Schizoneura lanuginosa, Scotinophora spp., Selenaspidus articulatus, Sitobion avenae, Sogata spp., Sogatella furcifera, Solubea insularis, Spissistilus festinus (=Stictocephala festina), Stephanitis nashi, Stephanitis pyrioides, Stephanitis takeyai, Tenala¬phara malayensis, Tetraleurodes perseae, Therioaphis maculate, Thyanta spp. e.g. T. accerra, T. perditor; Tibraca spp., Tomaspis spp., Toxoptera spp. e.g. T. aurantii; Trialeurodes spp. e.g. T. abutilonea, T. ricini, T. vaporariorum; Triatoma spp., Trioza spp., Typhlocyba spp., Unaspis spp. e.g. U. citri, U. yanonensis; and Viteus vitifolii,
   Insects from the order Hymenoptera e.g. Acanthomyops interjectus, Athalia rosae, Atta spp. e.g. A. capiguara, A. cephalotes, A. cephalotes, A. laevigata, A. robusta, A. sex¬dens, A. texana, Bombus spp., Brachymyrmex spp., Camponotus spp. e.g. C. floridanus, C. pennsylvanicus, C. modoc; Cardiocondyla nuda, Chalibion sp, Crematogaster spp., Dasymutilla occidentalis, Diprio¬n spp., Dolichovespula maculata, Dorymyrmex spp., Dryocosmus kuriphilus, Formica spp., Hoplocampa spp. e.g. H. minuta, H. testudinea; Iridomyrmex humilis, Lasius spp. e.g. L. niger, Linepithema humile, Liometopum spp., Leptocybe invasa, Monomorium spp. e.g. M. pharaonis, Monomorium, Nylandria fulva, Pachycondyla chinensis, Paratre¬chi¬na longicornis, Paravespula spp., e.g. P. germanica, P. pennsylvanica, P. vulgaris; Pheidole spp. e.g. P. megacephala; Po¬go¬nomyrmex spp. e.g. P. barbatus, P. californicus, Polistes rubiginosa, Prenolepis impairs, Pseu¬domyrmex gracilis, Schelipron spp., Sirex cyaneus, Solenopsis spp. e.g. S. geminata, S.invicta, S. molesta, S. richteri, S. xyloni, Sphecius speciosus, Sphex spp., Tapinoma spp. e.g. T. melanocephalum, T. sessile; Tetramorium spp., e.g. T. caespitum, T. bicarinatum, Vespa spp., e.g. V. crabro; Vespula spp., e.g. V. squamosal; Wasmannia auropunctata, Xylocopa sp;
Insects from the order Orthoptera e.g. Acheta domesticus, Calliptamus italicus, Chortoicetes terminifera, Ceuthophilus spp., Diastrammena asynamora, Dociostaurus maroccanus, Gryllotalpa spp. e.g. G. africana, G. gryllotalpa; Gryllus spp., Hieroglyphus daganensis, Kraussa-ria angulifera, Locusta spp. e.g. L. migratoria, L. pardalina; Melanoplus spp. e.g. M. bivittatus, M. femurrubrum, M. mexicanus, M. sanguinipes, M. spretus; Nomadacris septemfasciata, Oedaleus senegalensis, Scapteriscus spp., Schistocerca spp. e.g. S. americana, S. gregaria, Stemopelmatus spp., Tachycines asynamorus, and Zonozerus variegatus;
Pests from the Class Arachnida e.g. Acari,e.g. of the families Argasidae, Ixodidae and Sarcop¬tidae, e.g. Amblyomma spp. (e.g. A. americanum, A. variegatum, A. maculatum), Argas spp. e.g. A. persicu), Boophilus spp. e.g. B. annulatus, B. decoloratus, B. micro¬plus, Dermacentor spp. e.g. D.silvarum, D. andersoni, D. variabilis, Hyalomma spp. e.g. H. truncatum, Ixodes spp. e.g. I. ricinus, I. rubicundus, I. scapularis, I. holocyclus, I. pacificus, Rhipicephalus sanguineus, Ornithodorus spp. e.g. O. moubata, O. hermsi, O. turicata, Ornithonyssus bacoti, Otobius meg¬nini, Dermanyssus gallinae, Psoroptes spp. e.g. P. ovis, Rhi¬picephalus spp. e.g. R. sanguineus, R. appendiculatus, Rhipicephalus evertsi, Rhizogly¬phus spp., Sarcoptes spp. e.g. S. Scabiei; and Family Eriophyidae including Aceria spp. e.g. A. sheldoni, A. anthocoptes, Acallitus spp., Aculops spp. e.g. A. lycopersici, A. pelekassi; Aculus spp. e.g. A. schlechtendali; Colomerus vitis, Epitrimerus pyri, Phyllocoptruta oleivora; Eriophytes ribis and Eriophyes spp. e.g. Eriophyes sheldoni; Family Tarsonemidae including Hemitarsonemus spp., Phytonemus pallidus and Polyphagotarsonemus latus, Steno¬tar¬so¬nemus spp. Steneotarsonemus spinki; Family Tenuipalpidae including Brevipalpus spp. e.g. B. phoenicis; Family Tetranychidae including Eotetranychus spp., Eutetranychus spp., Oligonychus spp., Petrobia latens, Tetranychus spp. e.g. T. cinnabarinus, T. evansi, T. kan¬zawai, T, pacificus, T. phaseulus, T. telarius and T. urticae; Bryobia praetiosa; Panonychus spp. e.g. P. ulmi, P. citri; Metatetranychus spp. and Oligonychus spp. e.g. O. pratensis, O. perseae, Vasates lycopersici; Raoiella indica, Family Carpoglyphidae including Car¬poglyphus spp.; Penthaleidae spp. e.g. Halotydeus destructor; Family Demodicidae with species e.g. Demodex spp.; Family Trombicidea including Trombicula spp.; Family Macronyssidae including Ornothonyssus spp.; Family Pyemotidae including Pyemotes tritici; Tyrophagus putrescen¬tiae; Family Acaridae includ-ing Acarus siro; Family Araneida including Latrodectus mactans, Tegenaria agrestis, Chi-racanthium sp, Lycosa sp Achaearanea tepidariorum and Loxosceles reclusa;
Pests from the Phylum Nematoda, e.g. plant parasitic nematodes e.g. root-knot nematodes, Me¬loidogyne spp. e.g. M. hapla, M. incognita, M. javanica; cyst-forming nematodes, Globodera spp. e.g. G. rostochiensis; Heterodera spp. e.g. H. avenae, H. glyci¬nes, H. schachtii, H. trifolii; Seed gall nematodes, Anguina spp.; Stem and foliar nematodes, Aphelenchoides spp. e.g. A. besseyi; Sting nematodes, Belonolaimus spp. e.g. B. longicaudatus; Pine nematodes, Bursaphelenchus spp. e.g. B. lignicolus, B. xylophilus; Ring nematodes, Criconema spp., Criconemella spp. e.g. C. xenoplax and C. ornata; and, Criconemoides spp. e.g. Criconemoides informis; Mesocriconema spp.; Stem and bulb nematodes, Ditylenchus spp. e.g. D. destructor, D. dipsaci; Awl nematodes, Dolichodorus spp.; Spiral nematodes, Heliocotylenchus multicinctus; Sheath and sheathoid nematodes, Hemicycliophora spp. and Hemicriconemoides spp.; Hirshmanniella spp.; Lance nematodes, Hoploaimus spp.; False rootknot nematodes, Nacobbus spp.; Needle nematodes, Longidorus spp. e.g. L. elongatus; Lesion nematodes, Pratylenchus spp. e.g. P. brachyurus, P. neglectus, P. penetrans, P. curvitatus, P. goodeyi; Burrowing nematodes, Radopholus spp. e.g. R. similis; Rhadopholus spp.; Rhodopholus spp.; Reniform nematodes, Rotylenchus spp. e.g. R. robustus, R. reniformis; Scutellonema spp.; Stubby-root nematode, Trichodorus spp. e.g. T. obtusus, T. primitivus; Paratrichodorus spp. e.g. P. minor; Stunt nematodes, Tylenchorhynchus spp. e.g. T. claytoni, T. dubius; Citrus nematodes, Tylenchulus spp. e.g. T. semipenetrans; Dagger nematodes, Xiphinema spp.; and other plant parasitic nematode species;
Insects from the order Blattodea e.g. Macrotermes spp. e.g. M. natalensis; Cornitermes cu-mulans, Procornitermes spp., Globitermes sulfureus, Neocapritermes spp. e.g. N. opa¬cus, N. parvus; Odontotermes spp., Nasutitermes spp. e.g. N. corniger; Coptotermes spp. e.g. C. formo¬sanus, C. gestroi, C. acinaciformis; Reticulitermes spp. e.g. R. hesperus, R. tibialis, R. speratus, R. flavipes, R. grassei, R. lucifugus, R. virginicus; Heterotermes spp. e.g. H. aureus, H. longiceps, H. tenuis; Cryptotermes spp. e.g. C. brevis, C. cavifrons; Incisitermes spp. e.g. I. minor, I. snyderi; Marginitermes hubbardi, Kalotermes flavicollis, Neotermes spp. e.g. N. castaneus, Zootermopsis spp. e.g. Z. angusticollis, Z. nevadensis, Mastotermes spp. e.g. M. darwiniensis; Blatta spp. e.g. B. orientalis, B. lateralis; Blattella spp. e.g. B. asahinae, B. germanica; Rhyparobia maderae, Panchlora nivea, Periplaneta spp. e.g. P. americana, P. australasiae, P. brunnea, P. fuliginosa, P. japonica; Supella longipalpa, Parcoblatta pennsylvanica, Eurycotis floridana, Pycnoscelus surinamensis,
Insects from the order Siphonoptera e.g. Cediopsylla simples, Ceratophyllus spp., Ctenocephalides spp. e.g. C. felis, C. canis, Xenopsylla cheopis, Pulex irritans, Trichodectes canis, Tunga penetrans, and Nosopsyllus fasciatus,
Insects from the order Thysanura e.g. Lepisma saccharina, Ctenolepisma urbana, and Thermobia domestica,
Pests from the class Chilopoda e.g. Geophilus spp., Scutigera spp. e.g. Scutigera coleoptrata;
Pests from the class Diplopoda e.g. Blaniulus guttulatus, Julus spp., Narceus spp.,
Pests from the class Symphyla e.g. Scutigerella immaculata,
Insects from the order Dermaptera, e.g. Forficula auricularia,
Insects from the order Collembola, e.g. Onychiurus spp., e.g. Onychiurus armatus,
Pests from the order Isopoda, e.g. Armadillidium vulgare, Oniscus asellus, Porcellio scaber,

Insects from the order Phthiraptera, e.g. Damalinia spp., Pediculus spp. e.g. Pediculus hu-manus capitis, Pediculus humanus corporis, Pediculus humanus humanus; Pthirus pubis, Haematopinus spp. e.g. Haematopinus eurysternus, Haematopinus suis; Linognathus spp. e.g. Linognathus vituli; Bovicola bovis, Menopon gallinae, Menacanthus stramineus and Solenopotes capillatus, Trichodectes spp.,

Further pest species which may be controlled by compounds I include: from the Phylum Mollusca, class Bivalvia, e.g., Dreissena spp.; class Gastropoda, e.g., Arion spp., Biomphalaria spp., Bulinus spp., Deroceras spp., Galba spp., Lymnaea spp., Oncomelania spp., Pomacea canaliclata, Succinea spp.; from the class of the helminths, e.g., Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp. e.g. Haemonchus contortus; Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuel-leborni, Strongyloides stercora lis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

The compounds of the invention are particularly suitable for efficiently combating
insects from the sub-order of Auchenorrhyncha, e.g. Amrasca biguttula, Empoasca spp., Nephotettix virescens, Sogatella furcifera, Mahanarva spp., Laodelphax striatellus, Nilapar-vata lugens, Diaphorina citri;
Lepidoptera, e.g. Helicoverpa spp., Heliothis virescens, Lobesia botrana, Ostrinia nubilalis, Plutella xylostella, Pseudoplusia includens, Scirpophaga incertulas, Spodoptera spp., Trichoplusia ni, Tuta absoluta, Cnaphalocrocis medialis, Cydia pomonella, Chilo suppressalis, Anticarsia gemmatalis, Agrotis ipsilon, Chrysodeixis includens;
True bugs, e.g. Lygus spp., Stink bugs such as Euschistus spp., Halyomorpha halys, Nezara viridula, Piezodorus guildinii, Dichelops furcatus;
Thrips, e.g. Frankliniella spp., Thrips spp., Dichromothrips corbettii;
Aphids, e.g. Acyrthosiphon pisum, Aphis spp., Myzus persicae, Rhopalosiphum spp., Schizaphis graminum, Megoura viciae;
Whiteflies, e.g. Trialeurodes vaporariorum, Bemisia spp.;
Coleoptera, e.g. Phyllotreta spp., Melanotus spp., Meligethes aeneus, Leptinotarsa decimlineata, Ceutorhynchus spp., Diabrotica spp., Anthonomus grandis, Atomaria linearia, Agriotes spp., Epilachna spp.;
Flies, e.g. Delia spp., Ceratitis capitate, Bactrocera spp., Liriomyza spp.;
Coccoidea, e.g. Aonidiella aurantia, Ferrisia virgate;
Anthropods of class Arachnida (Mites), e.g. Penthaleus major, Tetranychus spp.;
Nematodes, e.g. Heterodera glycines, Meloidogyne sp., Pratylenchus spp., Caenorhabditis elegans.

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound of the invention.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or controlling parasites in and on animals, which comprises contacting the parasites with a parasitically effective amount of a compound of the invention.

The invention also relates to the non-therapeutic use of compounds of the invention for controlling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyetherantibiotics e.g. Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at its locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas.

The compounds of the invention are especially useful for combating parasites of the following orders and species, respectively:
fleas (Siphonaptera), e.g. Ctenocephalides felis, C. canis, Xenopsylla cheopis, Pulex irri-tans, Tunga penetrans, and Nosopsyllus fasciatus; cockroaches (Blattaria - Blattodea), e.g. Blattella germanica, B. asahinae, Periplaneta americana, P. japonica, P. brunnea, P. fuligginosa, P. australasiae, and Blatta orientalis; flies, mosquitoes (Diptera), e.g. Aedes aegypti, A. albopictus, A. vexans, Anastrepha ludens, Anopheles maculipennis, A. crucians, A. albimanus, A. gambiae, A. freeborni, A. leucosphyrus, A. minimus, A. quadrimaculatus, Calliphora vicina, Chrysomya bezziana, C. hominivorax, C. macellaria, Chrysops discalis, C. silacea, C. atlanticus, Cochliomyia hominivorax, Cordylobia anthropophaga, Culicoides furens, Culex pipiens, C. nigripalpus, C. quinquefasciatus, C. tarsalis, Culiseta inornata, C. melanura, Dermatobia hominis, Fannia canicularis, Gasterophilus intestinalis, Glossina morsitans, G. palpalis, G. fuscipes, G. tachinoides, Haematobia irritans, Haplodiplosis equestris, Hippelates spp., Hypoderma line-ata, Leptoconops torrens, Lucilia caprina, L. cuprina, L. sericata, Lycoria pectoralis, Mansonia spp., Musca domestica, M. stabulans, Oestrus ovis, Phlebotomus argentipes, Psorophora columbiae, P. discolor, Prosimulium mixtum, Sarcophaga spp., S. haemorrhoidalis, Simulium vittatum, Stomoxys calcitrans, Tabanus bovinus, T. atratus, T. lineola, and T. similis; lice (Phthiraptera), e.g. Pediculus humanus capitis, P. humanus humanus, Pthirus pubis, Haematopinus eurysternus, H. suis, Linognathus vituli, Bovicola bovis, Menopon gallinae, Menacanthus stramineus, and Solenopotes capillatus; ticks and parasitic mites (Parasitiformes): ticks (Ixodida), e.g. Ixodes scapularis, I. holocyclus, I. pacificus, Rhiphicephalus sanguineus, Dermacentor andersoni, D. variabilis, Amblyomma americanum, A. maculatum, Ornithodorus hermsi, O. turicata and parasitic mites (Mesostigmata), e.g. Ornithonyssus bacoti, Dermanyssus gallinae; Actinedida (Prostigmata) and Acaridida (Astigmata), e.g. Acarapis spp., Cheyletiella spp., Ornithocheyletia spp., Myobia spp., Psorergates spp., Demo-dex spp., Trombicula spp., Listro¬phorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., and Laminosioptes spp; Bugs (Het-eropterida): Cimex lectularius, C. hemipterus, Reduvius senilis, Triatoma spp., Rhodnius ssp., Panstrongylus ssp., and Arilus critatus; Anoplurida, e.g. Haematopinus spp., Linognathus spp., Pediculus spp., Phtirus spp., and Solenopotes spp.; Mallophagida (suborders Arnblycerina and Ischnocerina), e.g. Tri¬menopon spp., Menopon spp., Trinoton spp., Bovicola spp., Werneckiella spp., Lepikentron spp., Trichodectes spp., and Felicola spp.; Roundworms Nematoda: Wipeworms and Trichi¬nosis (Trichosyringida), e.g. Tri¬chinellidae (Trichinella spp.), (Trichuridae) Trichuris spp., Capillaria spp.; Rhabditida, e.g. Rhabditis spp., Strongyloides spp., Helicephalobus spp.; Strongylida, e.g. Strongylus spp., Ancylo¬sto¬ma spp., Necator americanus, Bunostomum spp. (Hookworm), Trichostrongylus spp., Haemonchus contortus, Ostertagia spp., Cooperia spp., Nematodirus spp., Dictyocaulus spp., Cyatho¬sto¬ma spp., Oesophagostomum spp., Stephanurus dentatus, Ollula¬nus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp., Aleurostrongylus abstrusus, and Dioctophyma renale; Intestinal roundworms (Ascaridida), e.g. Ascaris lumbricoides, Ascaris suum, Ascaridia galli, Parascaris equorum, Enterobius vermicularis (Threadworm), Toxocara canis, Toxascaris leonine, Skrjabinema spp., and Oxyuris equi; Camallanida, e.g. Dracunculus medinensis (gui¬nea worm); Spirurida, e.g. Thelazia spp., Wuchereria spp., Brugia spp., Onchocerca spp., Dirofilari spp.a, Dipetalonema spp., Setaria spp., Elaeophora spp., Spirocerca lupi, and Habronema spp.; Thorny headed worms (Acanthocephala), e.g. Acanthocephalus spp., Macracanthorhynchus hirudinaceus and Oncicola spp.; Planarians (Plathelminthes): Flukes (Trematoda), e.g. Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp., and Nanocyetes spp.; Cercomeromorpha, in particular Cestoda (Tapeworms), e.g. Diphyllo-bothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp., and Hymenolepis spp..

The term "animal" includes warm-blooded animals (including humans) and fish. Preferred are mammals, e.g. cattle, sheep, swine, camels, deer, horses, pigs, poultry, rabbits, goats, dogs and cats, water buffalo, donkeys, fallow deer and reindeer, and also in fur-bearing animals e.g. mink, chinchilla and raccoon, birds e.g. hens, geese, turkeys and ducks and fish e.g. fresh- and salt-water fish e.g. trout, carp and eels. Particularly preferred are domestic animals, e.g. dogs or cats.

Generally, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions e.g. desired parasiticidal effect and duration, target species, mode of application.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. In addition, the compounds I may be administered to the animals in their drinking water. For oral administration, the dosage form chosen should provide the animal with 0.01 mg/kg to 100 mg/kg of animal body weight per day of the compounds I, preferably with 0.5 mg/kg to 100 mg/kg of animal body weight per day. Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition the compounds I may be transdermally administered to animals. For parenteral administration, the dosage form chosen should provide the animal with 0.01 mg/kg to 100 mg/kg of animal body weight per day of the compounds I.

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds I. In addition, the compounds I may be formulated as ear tags for animals, particularly quadrupeds e.g. cattle and sheep.

Suitable preparations are:
- Solutions e.g. oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations e.g. powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further auxiliaries e.g. acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on. Solutions for use on the skin are prepared according to the state of the art and according to what is described above for injection solutions, sterile procedures not being necessary.

Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dissolving, suspending or emulsifying the active compound in suitable skin-compatible solvents or solvent mixtures. If appropriate, other auxiliaries e.g. colorants, bioabsorption-promoting substances, antioxidants, light stabilizers, adhesives are added. Suitable such auxiliaries are known in the art.

Emulsions can be administered orally, dermally or as injections. Emulsions are either of the water-in-oil type or of the oil-in-water type. They are prepared by dissolving the active compound either in the hydrophobic or in the hydrophilic phase and homogenizing this with the solvent of the other phase with the aid of suitable emulsifiers and, if appropriate, other auxiliaries e.g. colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art. Suspensions can be administered orally or topically/dermally. They are prepared by suspending the active compound in a suspending agent, if appropriate with addition of other auxiliaries e.g. wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight. Preparations which are diluted before use contain the compounds acting against ectoparasites in concentrations of 0.5 to 90% by weight, preferably of 1 to 50% by weight.

Furthermore, the preparations comprise the compounds of formula I against endoparasites in concentrations of 10 ppm to 2% by weight, preferably of 0.05 to 0.9% by weight, very particularly preferably of 0.005 to 0.25% by weight.

Topical application may be conducted with compound-containing shaped articles e.g. collars, medallions, ear tags, bands for fixing at body parts, and adhesive strips and foils.

Generally it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

### A. Preparation examples

With appropriate modification of the starting materials, the procedures given in the synthesis description were used to obtain further compounds I. The compounds obtained in this manner are listed in the table that follows, together with physical data.

The products shown below were characterized by melting point determination, by NMR spectroscopy or by the masses ([m/z]) or retention time (RT; [min.]) determined by HPLC-MS or HPLC spectrometry.

HPLC-MS = high performance liquid chromatography-coupled mass spectrometry;
HPLC method A: Shimadzu LC2010, Column: Waters XBridge C18 ,150mm x 4.6mm ID x 5µ; Mobile Phase: A: water + 0,1% TFA; B: acetonitrile + 0,1% TFA; Temperature: 40°C; Gradient: 10% B to 100% B in 5min; 100% B 2min; 10% B 3min; Flow: 1,4ml/min; Run Time: 10 min; PDA detector.

### Example 1: 2-chloro-N-(2,4-difluorophenyl)-5-[[5-(trifluoromethyl)-5-[3-(trifluoromethyl)phenyl]-4H-isoxazol-3-yl]amino]benzamide [I-20].

Step 1: To a solution of i-PrMgCl (99mL, 0.20mol) in THF (600mL) at 0°C was added n-BuLi (87.3 mL, 0.22 mol), followed by the dropwise addition of 1-bromo-3-(trifluoromethyl)benzene (45g, 0. 20mol) in THF (50mL), and the resulting mixture was stirred at 0°C for 30min, after which HPLC analysis showed the reaction was completed. 2,2,2-trifluoro-N-methoxy-N-methylacetamide (94.5g, 0.60mol) was added dropwise at 0°C, the reaction was allowed to warm to ambient temperature (20-25°C; RT) and stirred for 3h, after which HPLC showed the reaction was completed. The resulting reaction mixture was poured into NH₄Cl solution (600mL, sat. aq.), extracted with ethyl acetate (EtOAc; 3x400mL), and the combined organic extracts were washed with NaCl solution (400 mL, sat. aq.), dried over Na₂SO₄, filtered and concentrated to give 2,2,2-trifluoro-1-[3-(trifluoromethyl)phenyl]ethanone (37g, yield 77%) as a yellow oil which could be directly used in next step without further purification.

Step 2: To a solution of 2,2,2-trifluoro-1-[3-(trifluoromethyl)phenyl]ethanone (37.0g, 0.153mol) in THF (400mL) at ambient temperature was added 2-diethoxyphosphorylacetonitrile (26.6 g, 0.153mol), LiBr (13.3g, 0.153mol), and Et₃N (31.0g, 0.306mol), before the resulting reaction mixture was heated to 70°C and stirred at that temperature for 16 hours. After that time, TLC analysis (petroleum ether/ EtOAc = 5:1) showed that the reaction was completed. The reaction mixture was allowed to cool to ambient temperature, filtered, and the filtrate was extracted with EtOAc (3x300mL). The combined organic extracts were washed with NaCl solution (200 mL, sat. aq.), dried over Na₂SO₄, filtered and concentrated. the residue was purified by column (petroleum ether/EtOAc= 5:1) to give (Z)-4,4,4-trifluoro-3-[3-(trifluoromethyl)phenyl]but-2-enenitrile (30 g, yield 75%) as a colorless oil.
¹H-NMR (400MHz, CDC13, RT): δ 7.82 (br d, J=7.9Hz, 1 H) 7.77 (s, 1 H) 7.67 - 7.74 (m, 1 H) 7.59 - 7.66 (m, 1 H) 5.91 - 6.42 (m, 1 H).

Step 3: To a solution of (Z)-4,4,4-trifluoro-3-[3-(trifluoromethyl)phenyl]but-2-enenitrile (30g, 0.11mol) in MeOH (300 mL) was added hydroxyurea (12.0g, 0.159mol) and NaOMe (10.7 g, 0.204mol) in portions at 25°C, and the resulting mixture was then heated to 80°C and stirred at that temperature for 16h. After that time, TLC analysis (petroleum ether/EtOAc = 5:1) showed that the reaction was completed. The reaction mixture was poured into water (100 mL), extracted with EtOAc (3x100mL), and the combined organic extracts were washed with NaCl solution (100mL, sat. aq.), dried over Na₂SO₄, filtered and concentrated. Purification by column chromatography (petroleum ether/EtOAc = 5:1) afforded 5-(trifluoromethyl)-5-[3-(trifluoromethyl)phenyl]-4H-isoxazol-3-amine (16 g, 48%) as a white solid.
¹H-NMR (400MHz, DMSO-d₆, RT): δ 7.82 (br d, J=7.9Hz, 1 H) 7.77 (s, 1 H) 7.67 - 7.74 (m, 1 H) 7.59 - 7.66 (m, 1 H) 5.91 - 6.42 (m, 1 H).

Step 4: To a solution of 5-(trifluoromethyl)-5-[3-(trifluoromethyl)phenyl]-4H-isoxazol-3-amine (5.00 g, 16.8mmol) in 1,4-dioxane (50mL) at ambient temperature was added Pd₂(dba)₃ (1.54 g, 1.68 mmol, CAS-No 51364-51-3), Xantphos (1.45g, 2.51mmol, CAS-No 161265-03-8), Cs₂CO₃ (8.21g, 25.2mmol) and tert-butyl 5-bromo-2-chloro-benzoate (5.38g, 18.5mmol), before the reaction mixture was heated to 80°C and stirred at that temperature for 16h. Then LCMS and TLC analysis showed that the reaction was completed. The reaction mixture was poured into water (50mL), extracted with EtOAc (3x40mL), and the combined organic extracts were washed with NaCl solution (40 mL), dried over Na₂SO₄, filtered, and concentrated. Purification by column chromatography (petroleum ether/EtOAc=3:1) to give tert-butyl 2-chloro-5-[[5-(trifluoromethyl)-5-[3-(trifluoromethyl)phenyl]-4H-isoxazol-3-yl]amino]benzoate (7.0 g, 88%) as a brown solid.
¹H-NMR (400MHz, DMSO-d₆, RT): δ 7.79 (br d, J=7.9 Hz, 1 H), 7.82 (s,1 H), 7.70 (d, J=7.8 Hz, 1 H), 7.53 - 7.62 (m, 3 H), 7.34 (d, J=8.5 Hz, 1 H), 3.93 (d, J=16.1 Hz, 1 H), 3.58 (d, J=16.1 Hz, 1 H), 1.61 (s, 9 H).

Step 5: To a solution of tert-butyl 2-chloro-5-[[5-(trifluoromethyl)-5-[3-(trifluoromethyl)phenyl]-4H-isoxazol-3-yl]amino]benzoate (7.0g, 13.3mmol) in a mixture of MeCN (80mL) and H₂O (1 mL) at ambient temperature was added I₂ (3.37g, 13.3mmol), before the reaction was heated to 80°C and stirred at that temperature for 16 hours. Then LCMS and TLC analysis (petroleum ether/EtOAc = 3:1) showed that the reaction was completed. The reaction mixture was poured into water (100 mL) and the pH adjusted to 3 with aq. HCI (2N), the aqueous phase was extracted with EtOAc (3x80mL), and the combined organic extracts were washed with NaCl (80 mL, sat. aqueous), dried over Na₂SO₄, filtered and concentrated to give 2-chloro-5-[[5-(trifluoromethyl)-5-[3-(trifluoromethyl)phenyl]-4H-isoxazol-3-yl]amino]benzoic acid (4.0g, yield 64%) as a brown solid.
¹H-NMR (400MHz, DMSO-d₆, RT): δ 10.3 (s, 1 H) 9.58 (s, 1 H) 7.83 - 7.97 (m, 3 H) 7.69 - 7.80 (m, 2 H) 7.47 (s, 2 H) 7.63 (s, 1 H) 3.79 - 4.18 (m, 2 H).

Step 6: To a solution of 2-chloro-5-[[5-(trifluoromethyl)-5-[3-(trifluoromethyl)phenyl]-4H-isoxazol-3-yl]amino]benzoic acid (1.0g, 2.2mmol) in DMF (10mL) at ambient temperature was added HATU (1.26g, 3.31 mmol, CAS-No 148893-10-1) and Et₃N (672mg, 6.63mmol), and 2,4-difluoroaniline (200mg, 2.43mmol), and the reaction mixture was stirred at that temperature for 16 hours. Then LCMS and TLC analysis (petroleum ether/EtOAc = 3:1) showed that the reaction was completed. The mixture was poured into water (10mL), extracted with EtOAc (3x20mL), and the combined organic extracts were washed with NaCl solution (2×10 mL), dried over Na₂SO₄, filtered and concentrated. Purification by column chromatography (petroleum ether/EtOAc = 3:1) afforded the title compound 2-chloro-N-(2,4-difluorophenyl)-5-[[5-(trifluoromethyl)-5-[3-(trifluoromethyl)phenyl]-4H-isoxazol-3-yl]amino]benzamide [l-20] (200mg, 16%) as a yello-w solid.
¹H NMR (400MHz, CDCl₃, RT): δ 10.3 (s, 1 H) 9.59 (s, 1 H) 7.85 - 7.98 (m, 3 H) 7.70 - 7.82 (m, 2 H) 7.64 (s, 1 H) 7.48 (s, 2 H) 7.32 - 7.41 (m, 1 H) 7.14 (br t, J=7.8 Hz, 1 H) 3.78 - 4.16 (m, 2 H).

### Example 2: N-(1-cyanocyclopropyl)-3-[[5-(3,5-dichlorophenyl)-5-(trifluoromethyl)-4H-isoxazol-3-yl]amino]-2,6-difluoro-benzamide [I-11].

Step 1: To a suspension of Mg(0) turnings (27.7g, 1.15mol) in THF (300mL) at ambient temperature was added a solution of 1-bromo-3,5-dichloro-benzene (250 g, 0.960 mol) in THF (1.0 L) dropwise, before the solution was heated to 70°C and stirred at that temperature for 1h. After that time, the reaction mixture was cooled to 0°C, and 2,2,2-trifluoro-N-methoxy-N-methylacetamide was added dropwise into the reaction at 0°C and the mixture was stirred at that temperature for 1h. The resulting solution was quenched with NH₄Cl (1.2L, sat. aq.), extracted with EtOAc (3x500mL), and the combined organic extracts were washed with NaCl solution (1.2L, sat. aq.), filtered, and concentrated. Purification by distillation at 110 °C with reduced pressure afforded 1-(3,5-dichlorophenyl)-2,2,2-trifluoro-ethanone (300g, 64%) as a faintly yellowish oil.
¹H-NMR (400MHz, CDC13, RT): δ = 7.92 (d, J = 1.0 Hz, 2H), 7.70 (t, J = 1.9 Hz, 1H).

Step 2: To the solution of 1-(3,5-dichlorophenyl)-2,2,2-trifluoro-ethanone (25g, 0.11mol) and Ph₃PMeBr (1.3g, 12mmol) in THF (400mL) at ambient temperature was added t-BuOK, 1M in THF (123mL, 0.139mol) dropwise, and the mixture was stirred at that temperature for 2h. After that time, HPLC analysis showed that the reaction was completed. The mixture was quenched with H₂O (1L), extracted with EtOAc (3x300mL), washed with NaCl solution (1L, sat. aq.), dried over Na₂SO₄, filtered and concentrated. Purification by column chromatography (petrol ether [PE]) afforded 1,3-dichloro-5-[1-(trifluoromethyl)vinyl]benzene (70 g, 73%) as a yellow oil.
¹H-NMR (400MHz, CDC13, RT): δ = 7.41 - 7.39 (m, 1H), 7.36 - 7.34 (m, 2H), 6.07 - 6.03 (m, 1H), 5.84 - 5.81 (m, 1H).

Step 3: To a solution of 1,3-dichloro-5-[1-(trifluoromethyl)vinyl]benzene (22.0g, 92.8mmol) in EtOAc (220mL) at ambient temperature was added dibromomethanone oxime (22.5g, 113 mmol), and NaHCO₃ (78.1 g, 0.928mol), before the mixture was heated to 50°C and stirred at that temperature for 12h. Then TLC analysis (petroleum ether/EtOAc=10:1) showed that the reaction was completed. The reaction mixture was quenched with H₂O (300 mL), extracted with EtOAc (3x100mL), and the combined organic extracts were washed with NaCl solution (300mL, sat. aq.), dried over Na₂SO₄, filtered and concentrated. Purification by trituration (petroleum ether) afforded 3-bromo-5-(3,5-dichlorophenyl)-5-(trifluoromethyl)-4H-isoxazole (64g, 63%) as a white solid.
¹H-NMR (400MHz, CDC13, RT): δ = 7.48 - 7.44 (m, 1H), 7.44 - 7.38 (m, 2H), 3.92 (d, J = 17.8 Hz, 1H), 3.57 (dd, J = 0.6, 17.8 Hz, 1H).

Step 4: To a solution of 3-bromo-5-(3,5-dichlorophenyl)-5-(trifluoromethyl)-4H-isoxazole (0.50 g, 1.38 mmol) in 2,4-dimethylpentan-3-ol (5 mL) at ambient temperature was added methyl 3-amino-2,6-difluoro-benzoate (0.257g, 1.377mmol), and TsOH (0.183g, 0.964mmol), before the mixture was heated to 130°C and stirred at that temperature for 16hrs. After that time, LCMS showed ca. 30% of the desired compound, and the reaction mixture was quenched with H₂O (100mL), the aqueous phase was extracted with EtOAc (3x30mL), and the combined organic extracts were washed with NaCl solution (100mL, sat. aq.), dried over Na₂SO₄, filtered and concentrated. The residue was purified by trituration (petroleum ether) to give methyl 3-[[5-(3,5-dichlorophenyl)-5-(trifluoromethyl)-4H-isoxazol-3-yl]amino]-2,6-difluoro-benzoate (0.24 g, 38%) as a yellow solid.
¹H-NMR (400MHz, CDC13, RT): δ = 8.21 (dt, J = 5.4, 9.4 Hz, 1H), 7.48 (s, 2H), 7.46 - 7.42 (m, 1H), 6.98 (dt, J = 1.8, 9.2 Hz, 1H), 6.11 (br s, 1H), 3.94 (s, 1H), 3.59 (d, J = 16.0 Hz, 1H), 1.56 (s, 3H).

Step 5: To the solution of methyl 3-[[5-(3,5-dichlorophenyl)-5-(trifluoromethyl)-4H-isoxazol-3-yl]amino]-2,6-difluoro-benzoate (1.10g, 2.34mmol) in THF (220mL) at ambient temperature was added LiOH·H₂O (0.148g, 3.52mmol), before the mixture was heated to 70°C and stirred at that temperature for 5 days. Then LCMS analysis showed that the reaction was completed. The reaction mixture was quenched with H₂O (200mL), the aqueous phase was adjusted to pH=5 with aq. HCl, and then extracted with EtOAc (3x60mL). The combined organic extracts were washed with NaCl solution (200mL), dried over Na₂SO₄, filtered and concentrated. The residue was purified by Prep-HPLC (NH₄HCO₃) to give 3-[[5-(3,5-dichlorophenyl)-5-(trifluoromethyl)-4H-isoxazol-3-yl]amino]-2,6-difluoro-benzoic acid (0.65g, 61%) as a white solid.
¹H NMR (400MHz, CDC13, RT): δ = 7.47 (s, 1H), 7.38 (s, 2H), 4.68 (br s, 2H), 3.48 (d, J = 17.2 Hz, 1H), 3.19 (br d, J = 17.2Hz, 1H), 1.26 (s, 1H).

Step 6: To a solution of 3-[[5-(3,5-dichlorophenyl)-5-(trifluoromethyl)-4H-isoxazol-3-yl]amino]-2,6-difluoro-benzoic acid (0.30g, 0.66mmol) in MeCN (4mL) at ambient temperature was added 1-aminocyclopropanecarbonitrile (93.5mg, 0.793 mmol), N,N,N',N'-tetramethylchloroformamidinium hexafluorophosphate ("TCFH") (0.278g, 0.991mmol), and N-methylimidazole ("NMI") (0.217g, 2.64mmol), and the reaction mixture was stirred at that temperature for 16h. Then TLC analysis (petroleum ether/EtOAc = 1:1) showed that the reaction was completed, and the reaction mixture was quenched with H₂O (10mL), filtered and the filtrate was extracted with EtOAc (3x3mL). The combined organic extracts were washed with NaCl solution (10mL), dried over Na₂SO₄, filtered and concentrated. Purification by column chromatography (PE/EtOAc = 100:0 to 65:35) afforded the title compound N-(1-cyanocyclopropyl)-3-[[5-(3,5-dichlorophenyl)-5-(trifluoromethyl)-4H-isoxazol-3-yl]amino]-2,6-difluoro-benzamide [1-11] (0.116g, yield 34%) as a white solid.
¹H-NMR (400MHz, Methanol-d₄, RT): δ = 8.12 (dt, J = 5.7, 9.3Hz, 1H), 7.56 (s, 3H), 7.04 (dt, J = 1.7, 9.0Hz, 1H), 4.86 (s, 47H), 3.98 (d, J = 17.0Hz, 1H), 3.71 (d, J = 17.0Hz, 1H), 1.63 - 1.57 (m, 2H), 1.34 - 1.31 (m, 2H).

### Example 3: 3-[[5-(3,5-dichlorophenyl)-5-(trifluoromethyl)-4H-isoxazol-3-yl]amino]-N-(2,4-difluorophenyl)-2-fluoro-benzamide [I-13].

Step 1: To a solution of 3-bromo-5-(3,5-dichlorophenyl)-5-(trifluoromethyl)-4H-isoxazole (0.50 g, 1.38mmol, synthesized as described in example 2; steps 1-3) in 2,4-dimethylpentan-3-ol (5 mL) at ambient temperature was added 3-bromo-2-fluoro-aniline (0.178g, 1.38mmol), and TsOH (0.183g, 0.964mmol), before the reaction was heated to 120°C and stirred at that temperature for 48h. Then LCMS analysis showed ca. 20% desired product, and the reaction mixture was quenched with H₂O (100mL), filtered and the filtrate extracted with EtOAc (3x30mL). The combined organic extracts were washed with NaCl solution (100mL, sat. aq.), dried over Na₂SO₄, filtered and concentrated. Purification by column chromatography (PE/EtOAc = 100:0 to 90:10) afforded N-(3-bromo-2-fluoro-phenyl)-5-(3,5-dichlorophenyl)-5-(trifluoromethyl)-4H-isoxazol-3-amine (78mg, 12%) as a white solid.
¹H-NMR (400MHz, CDCl₃, RT): δ = 8.04 (dt, J = 1.3, 8.0 Hz, 1H), 7.54 - 7.48 (m, 2H), 7.47 - 7.40 (m, 1H), 7.23 - 7.13 (m, 1H), 7.09 - 7.00 (m, 1H), 6.19 (br s, 1H), 4.03 - 3.92 (m, 1H), 3.60 (br d, J = 16.1 Hz, 1H).

Step 2: To a solution of N-(3-bromo-2-fluoro-phenyl)-5-(3,5-dichlorophenyl)-5-(trifluoromethyl)-4H-isoxazol-3-amine (500mg, 1.06mmol) in toluene (5mL) was added potassium vinyltrifluoroborate (212mg, 1.59mmol), t-Bu₃P-Pd-G2 (54mg, 0.11mmol, CAS-No 1375325-71-5), and K₂CO₃ solution (0.525mL, 2M in H₂O), before the reaction was heated to 80°C and stirred at that temperature for 12h. Then HPLC analysis showed ca. 50% of the desired compound, and the mixture was quenched with H₂O (20mL), filtered and the filtrate was extracted with EtOAc (3x8mL). The combined organic extracts were washed with NaCl solution (20mL, sat. aq.), dried over Na₂SO₄, filtered and concentrated. Purification by column chromatography (PE/EtOAc = 100:0 to 95:5) afforded 5-(3,5-dichlorophenyl)-N-(2-fluoro-3-vinyl-phenyl)-5-(trifluoromethyl)-4H-isoxazol-3-amine (286mg, 65%) as a white solid.
¹H NMR (400MHz, CDCl₃, RT): δ = 7.94 (br t, J = 7.1Hz, 1H), 7.50 (s, 2H), 7.45 - 7.41 (m, 1H), 7.18 - 7.08 (m, 2H), 6.85 (dd, J = 11.2, 17.7Hz, 1H), 6.18 (br s, 1H), 5.83 (dd, J = 0.8, 17.7 Hz, 1H), 5.42 (dd, J = 0.8, 11.2Hz, 1H), 3.97 (d, J = 16.1Hz, 1H), 3.59 (br d, J = 16.1Hz, 1H).

Step 3: To a solution of 5-(3,5-dichlorophenyl)-N-(2-fluoro-3-vinyl-phenyl)-5-(trifluoromethyl)-4H-isoxazol-3-amine (3.0g, 7.2mmol) in a mixture of THF (20mL) and H₂O (10mL) at 0°C was added N-methylmorpholin-N-oxid (2.01g, 17.2mmol), NalO₄ (1.50g, 7.02mmol), and K₂OsO₄ (21mg, 0.006 mmol), and the resulting reaction mixture was stirred at 0-2 °C for 16h. After that time, LCMS showed ca. 50% of the desired compound, and the mixture was quenched with H₂O (30mL), filtered and the filtrate was extracted with EtOAc (3×10mL). The combined organic extracts were washed with NaCl solution (30mL, sat. aq.), dried over Na₂SO₄, filtered and concentrated to give 3-[[5-(3,5-dichlorophenyl)-5-(trifluoromethyl)-4H-isoxazol-3-yl]amino]-2-fluoro-benzaldehyde (3g, crude) as a yellow solid which was used in the next step without further purification.
¹H NMR (400MHz, CDCl₃, RT) δ = 10.33 (s, 1H), 8.37 (br t, J = 8.1 Hz, 1H), 7.54 - 7.45 (m, 4H), 7.46 - 7.40 (m, 1H), 6.36 (br s, 1H), 4.05 - 3.97 (m, 1H), 3.64 (br d, J = 16.3Hz, 1H).

Step 4: To a solution of 3-[[5-(3,5-dichlorophenyl)-5-(trifluoromethyl)-4H-isoxazol-3-yl]amino]-2-fluoro-benzaldehyde (0.50g, 1.2mmol) in a mixture of 1, 4-dioxane (3.6mL) and H₂O (1.2 mL) at ambient temperature was added NaClO₂ (0.214g, 2.4mmol), and NH₃SO₃ (0.0462g, 4.76mmol), and the reaction mixture was stirred at that temperature for 40min. Then LCMS analysis showed that the reaction was completed, and the mixture was quenched with H₂O (10mL), filtered and the filtrate was extracted with EtOAc (3x3mL). The combined organic extracts were washed with NaCl solution (10 mL, sat. aq.), dried over Na₂SO₄, filtered and concentrated. Purification by Prep-HPLC (TFA) afforded 3-[[5-(3,5-dichlorophenyl)-5-(trifluoromethyl)-4H-isoxazol-3-yl]amino]-2-fluoro-benzoic acid (1.33g, 52%) as a yellow solid.
¹H NMR (400MHz, DMSO-d₆, RT) δ = 9.18 (d, J = 1.8Hz, 1H), 8.13 (dt, J = 1.5, 7.9Hz, 1H), 7.78 (t, J = 1.8Hz, 1H), 7.60 (d, J = 1.6Hz, 2H), 7.46 - 7.36 (m, 1H), 7.23 (t, J = 8.0Hz, 1H), 4.08 - 3.97 (m, 1H), 3.93 - 3.85 (m, 1H).

Step 5: To a solution of 3-[[5-(3,5-dichlorophenyl)-5-(trifluoromethyl)-4H-isoxazol-3-yl]amino]-2-fluoro-benzoic acid (0.33g, 0.757mmol) in MeCN (4mL) at ambient temperature was added 2,4-difluoroaniline (0.145g, 1.135mmol), TCFH (0.212g, 1.14mmol), and NMI (0.249g, 3.03 mmol), and the reaction was stirred at that temperature for 16h. Then LCMS analysis showed that the reaction was completed, and the mixture was quenched with H₂O (10 ml), filtered and the filtrate was extracted with EtOAc (3×3mL). The combined organic extracts were washed with NaCl solution (10mL, sat. aq.), dried over Na₂SO₄, filtered and concentrated. Purification by Prep-HPLC (NH₄HCO₃) afforded the 3-[[5-(3,5-dichlorophenyl)-5-(trifluoromethyl)-4H-isoxazol-3-yl]amino]-N-(2,4-difluorophenyl)-2-fluoro-benzamide [l-13] (53 mg, 13%) as a yellow solid.
¹H-NMR (400MHz, CDC13, RT): δ = 8.45 (dt, J = 6.0, 9.1Hz, 1H), 8.36 - 8.29 (m, 1H), 7.78 - 7.67 (m, 1H), 7.51 (d, J = 1.6 Hz, 2H), 7.48 - 7.43 (m, 1H), 7.39 - 7.30 (m, 1H), 6.99 - 6.88 (m, 2H), 6.30 - 6.23 (m, 1H), 3.99 (d, J = 16.1Hz, 1H), 3.62 (d, J = 16.0Hz, 1H).

### Example 4: Synthesis of 2-chloro-5-[[5-(3,5-dichloro-4-fluoro-phenyl)-5-(trifluoromethyl)-4H-isoxazol-3-yl]amino]-N-[(4R)-3-oxo-2-(2,2,2-trifluoroethyl)isoxazolidin-4-yl]benzamide [l-119].

### Synthesis of [(4R)-3-oxo-2-(2,2,2-trifluoroethyl)isoxazolidin-4-yl]ammonium chloride:

Step 1: To a solution of (4R)-4-aminoisoxazolidin-3-one (5.00g, 49.0mmol) in THF (75mL) and water (50mL) at ambient temperature was added triethylamine (5.20g, 51.4mmol). The reaction mixture was cooled to 10°C and a solution of di-tert-butyl carbonate (11.2g, 51.4 mmol) in THF (25mL) was added dropwise. The resulting mixture was stirred for 18h at ambient temperature. Then THF was removed under reduced pressure. The residue was treated with HCI (aqueous, 2N) until pH 2-3 was reached and stirred for 45min. The resulting precipitate was filtered off, washed with water (2x20mL) and cyclohexane (2x10mL), and dried under reduced pressure at 40°C to give tert-butyl N-[(4R)-3-oxoisoxazolidin-4-yl]carbamate (5.32g, 52% yield) as a white solid.
LCMS retention time: 0.659 min, m/z = 285 ([M+H]⁺)

Step 2: To a solution of tert-butyl N-[(4R)-3-oxoisoxazolidin-4-yl]carbamate (2.00g, 9.89mmol) in THF (30mL) at ambient temperature was added triethylamine (3.00g, 29.7 mmol). The reaction mixture was cooled to 0°C and 2,2,2-trifluoroethyl-trifluoromethanesulfonate (2.98g, 12.9mmol) was added. The resulting mixture was stirred for 96h at ambient temperature. The reaction was quenched into water (20ml) and extracted with EtOAc (3× 10ml). The organic phase was washed with water (2x20ml), dried over Na₂SO₄, filtered and concentrated under reduced pressure. The crude residue was dissolved in MTBE (20mL). The precipitate was filtered off and dried under reduced pressure at 30°C to give tert-butyl N-[(4R)-3-oxo-2-(2,2,2-trifluoroethyl)isoxazolidin-4-yl]carbamate (1.32g, 47% yield) as a white solid.
LCMS retention time: 0.948 min, m/z = 229 ([M+H]⁺)

Step 3: To a solution of tert-butyl N-[(4R)-3-oxo-2-(2,2,2-trifluoroethyl)isoxazolidin-4-yl]carbamate (1.32g, 4.64mmol) in dichloromethane (10mL) at 0°C was added HCI (4M in 1,4-dioxane, 4.64mL, 18.6mmol) dropwise. The reaction mixture was stirred at ambient temperature for 19 h, and then concentrated under reduced pressure. The residue was re-dissolved in dichloromethane and concentrated under reduced pressure. The residue was re-dissolved in dichloromethane (3mL), the precipitate was filtered off and dried under reduced pressure at 40°C to give the title [(4R)-3-oxo-2-(2,2,2-trifluoroethyl)isoxazolidin-4-yl]ammonium chloride (906mg, 88%).
¹H-NMR (400MHz, DMSO-d₆, RT) δ 4.70 (d, J = 8.7Hz, 1H), 4.61 (t, J = 8.7Hz, 1H), 4.49 (q, J = 9.1Hz, 2H), 4.34 (t, J = 9.0 Hz, 1H).

### Synthesis of 2-chloro-5-[[5-(3,5-dichloro-4-fluoro-phenyl)-5-(trifluoromethyl)-4H-isoxazol-3-yl]amino]benzoic acid:

Step 1: To a solution of 5-(3,5-dichloro-4-fluoro-phenyl)-5-(trifluoromethyl)-4H-isoxazol-3-amine (1.5g, 4.73mmol, synthesized analogously as described above) and methyl 5-bromo-2-chloro-benzoate (1.18g, 4.73mmol) in 1,4-dioxane (50mL) at ambient temperature was added tris(dibenzylideneacetone)dipalladium(0) (216mg, 0.23mmol), 4,5-bis(diphenylphosphino)-9,9-dimethylxanthene (328mg, 0.57mmol) and Cs₂COs (4.62g, 14.2mmol). The resulting mixture was heated to 80°C and stirred at that temperature for 5h. After cooling to ambient temperature, the reaction mixture was diluted with ethyl acetate (50mL) and washed with ammonium chloride solution (sat. aq., 45mL) and NaCl solution (sat. aq., 45mL). The organic phase was dried over Na₂SO₄ and concentrated under reduced pressure. Purification by column chromatography (silica gel, cyclohexane / ethyl acetate 100:0 to 65:35) afforded methyl 2-chloro-5-[[5-(3,5-dichloro-4-fluoro-phenyl)-5-(trifluoromethyl)-4H-isoxazol-3-yl]amino]benzoate (1.12g, 48% yield).
LCMS retention time: 1.392 min, m/z = 485 ([M+H]⁺); ¹H-NMR (400MHz, CDC13, RT) δ 7.82 (d, J = 2.8Hz, 1H), 7.57 - 7.45 (m, 3H), 7.36 (d, J = 8.8Hz, 1H), 6.28 (s, 1H), 3.92 (s, 3H), 3.89 (d, J = 16.2Hz, 1H), 3.49 (d, J = 16.1 Hz, 1H).

Step 2: To a solution of methyl 2-chloro-5-[[5-(3,5-dichloro-4-fluoro-phenyl)-5-(trifluoromethyl)-4H-isoxazol-3-yl]amino]benzoate (1.12g, 2.31mmol) in THF (5mL) and MeOH (5 mL) at ambient temperature was added a solution of LiOH (968mg, 23.1mmol) in water (5mL) dropwise. After 4h, HPLC-MS analysis showed that the reaction was completed. The reaction mixture was treated with HCI (aqueous, 2N) until pH 1 was reached. The resulting mixture was extracted with EtOAc (3x 5mL). The organic phase was washed with NaCl solution (25mL, sat. aq.), dried over Na₂SO₄, filtered and concentrated under reduced pressure to give 2-chloro-5-[[5-(3,5-dichloro-4-fluoro-phenyl)-5-(trifluoromethyl)-4H-isoxazol-3-yl]amino]-benzoic acid (1.10 g, quantitative). The crude product was used in the next step without further purification.
LCMS retention time: 1.261 min, m/z = 472 ([M+H]+).
¹H-NMR (400MHz, DMSO-d₆, RT) δ 7.92 (d, J = 2.6Hz, 1H), 7.84 (d, J = 6.3Hz, 2H), 7.54 - 7.42 (m, 2H), 3.97 (d, J = 17.1Hz, 1H), 3.86 (d, J = 17.2 Hz, 1H).

### Synthesis of 2-chloro-5-[[5-(3,5-dichloro-4-fluoro-phenyl-5-(trifluoromethyl)-4H-isoxazol-3-yl]amino]-N-[(4R)-3-oxo-2-(2,2,2-trifluoroethyl)isoxazolidin-4-yl]benzamide [1-119]:

Step 1: To a solution of the above 2-chloro-5-[[5-(3,5-dichloro-4-fluoro-phenyl)-5-(trifluoromethyl)-4H-isoxazol-3-yl]amino]-benzoic acid (100mg, 0.21mmol) and [(4R)-3-oxo-2-(2,2,2-trifluoroethyl)isoxazolidin-4-yl]ammonium chloride (56mg, 0.21mmol) in dichloromethane (8ml) at ambient temperature was added 2,4,6-tripropyl-1,3,5,2λ5,4λ5,6λ5-trioxatriphosphinane 2,4,6-trioxide (50% in EtOAc, 809mg, 1.27mmol), iPr₂NEt (82mg, 0.64mmol) and 4-dimethylaminopyridine (26mg, 0.21 mmol), and the resulting reaction mixture was stirred at that temperature for 3h.

Then dichloromethane (5mL) was added and the reaction mixture was washed with water (10 mL). The organic phase was separated, dried over Na₂SO₄, and evaporated under reduced pressure. Purification by column chromatography (silica gel, cyclohexane / EtOAc 100:0 to 30:70) afforded the title 2-chloro-5-[[5-(3,5-dichloro-4-fluoro-phenyl-5-(trifluoromethyl)-4H-isoxazol-3-yl]amino]-N-[(4R)-3-oxo-2-(2,2,2-trifluoroethyl)isoxazolidin-4-yl]benzamide [l-119] (70 mg, 52% yield) as a white wax.
LCMS retention time: 1.303 min, m/z = 637 ([M+H]⁺)
¹H-NMR (500MHz, CDCl₃, RT) δ ppm 7.62 - 7.56 (m, 1H), 7.43 (dd, J = 6.6, 2.9Hz, 2H), 7.25 (t, J = 2.7Hz, 1H), 7.17 (d, J = 8.8Hz, 1H), 5.02 (p, J = 7.7, 6.5Hz, 1H), 4.81 (t, J = 8.6Hz, 1H), 4.34 (t, J = 10.5Hz, 1H), 4.24 (dt, J = 16.3, 8.1Hz, 1H), 4.13 (dt, J = 15.8, 8.1Hz, 1H), 3.85 (d, J = 16.5Hz, 1H), 3.49 (d, J = 16.5Hz, 1H).

### Example 5: Synthesis of 2-chloro-5-[[5-(3,5-dichloro-4-fluoro-phenyl)-5-(trifluoromethyl)-4H-isoxazol-3-yl]-methyl-amino]-N-[(4R)-2-ethyl-3-oxo-isoxazolidin-4-yl]benzamide [I-125].

Step 1: To a solution of methyl 2-chloro-5-[[5-(3,5-dichloro-4-fluoro-phenyl)-5-(trifluoromethyl)-4H-isoxazol-3-yl]amino]benzoate (200mg, 0.41 mmol, prepared as described above) in acetonitrile (5mL) at ambient temperature was added K₂CO₃ (114mg, 0.82mmol) followed by iodomethane (88mg, 0.62mmol). The reaction mixture was stirred for 5 days at ambient temperature. After that time, HPLC-MS analysis showed that the reaction was completed. The reaction mixture was quenched with water (20mL) and extracted with ethyl acetate (3x5ml). The organic phase was dried over Na₂SO₄, filtered and concentrated under reduced pressure. Purification by column chromatography (silica gel, cyclohexane / ethyl acetate 100:0 to 50:50) afforded methyl 2-chloro-5-[[5-(3,5-dichloro-4-fluoro-phenyl)-5-(trifluoromethyl)-4H-isoxazol-3-yl]-methyl-amino]benzoate (100mg, 49% yield) as an oil.
LCMS retention time: 1.426 min, m/z = 500 ([M+H]⁺)
¹H-NMR (400MHz, CDC13, RT) δ 7.62 (d, J = 2.7Hz, 1H), 7.52 - 7.46 (m, 3H), 7.24 (dd, J = 8.6, 2.8Hz, 1H), 3.95 (s, 3H), 3.65 (d, J = 16.5Hz, 1H), 3.30 (s, 3H), 3.20 (d, J = 16.5Hz, 1H).

Step 2: To a solution of methyl 2-chloro-5-[[5-(3,5-dichloro-4-fluoro-phenyl)-5-(trifluoromethyl)-4H-isoxazol-3-yl]-methyl-amino]benzoate (191mg, 0.38mmol) in THF (2mL) and methanol (2mL) at ambient temperature was added a solution of LiOH.H₂O (160mg, 3.80mmol) in water (2mL) dropwise. After 4h, HPLC-MS analysis showed that the reaction was completed. The reaction mixture was treated with HCI (aqueous, 2N) until pH 1 was reached and concentrated under reduced pressure. The residue was dissolved in water (10mL) extracted with ethyl acetate (2x5mL), and the combined organic extracts were washed with water (5mL) and NaCl solution (5mL, sat. aq.), dried over Na₂SO₄, filtered and concentrated under reduced pressure to afford 2-chloro-5-[[5-(3,5-dichloro-4-fluoro-phenyl)-5-(trifluoromethyl)-4H-isoxazol-3-yl]-methyl-amino]benzoic acid (130mg, 70%).
LCMS retention time: 1.267 min, m/z = 485 ([M+H]⁺)
¹H-NMR (500MHz, methanol-d₄, RT) δ 7.71 (d, J = 2.5Hz, 1H), 7.66 (d, J = 6.2Hz, 2H), 7.52 (d, J = 8.6Hz, 1H), 7.41 (dd, J = 8.6, 2.6Hz, 1H), 3.82 (d, J = 17.0Hz, 1H), 3.59 (d, J = 17.0Hz, 1H), 3.28 (s, 3H).

Step 3: To a solution of 2-chloro-5-[[5-(3,5-dichloro-4-fluoro-phenyl)-5-(trifluoromethyl)-4H-isoxazol-3-yl]-methyl-amino]benzoic acid (40mg, 0.08mmol) and (4R)-4-amino-2-ethyl-isoxazolidin-3-one hydrochloride (16mg, 0.1mmol) in dichloromethane (8mL) at ambient temperature was added 2,4,6-tripropyl-1,3,5,2λ5,4λ5,6λ5-trioxatriphosphinane 2,4,6-trioxide (50% in EtOAc, 414mg, 0.49mmol), followed by iPr₂NEt (32mg, 0.25mmol) and 4-dimethylaminopyridine (10mg, 0.08mmol). The resulting reaction mixture was stirred for 2h at RT. Then dichloromethane (5ml) was added and the reaction mixture was washed with water (10ml). The organic phase was dried over Na₂SO₄ and evaporated under reduced pressure. Purification by column chromatography (silica gel, cyclohexane / EtOAc 100:0 to 0:100) afforded 2-chloro-5-[[5-(3,5-dichloro-4-fluoro-phenyl)-5-(trifluoromethyl)-4H-isoxazol-3-yl]-methyl-amino]-N-[(4R)-2-ethyl-3-oxo-isoxazolidin-4-yl]benzamide (34mg, 69%).
LCMS retention time: 1.268 min, m/z = 597 ([M+H]⁺)
¹H-NMR (500MHz, CDC13, RT) δ 7.52 (t, J = 2.6Hz, 1H), 7.49 (d, J = 5.9Hz, 2H), 7.45 (d, J = 8.6Hz, 1H), 7.19 (dd, J = 8.6, 2.7Hz, 1H), 4.96 (t, J = 8.3Hz, 1H), 4.88 (dddd, J = 10.3, 8.0, 4.3, 2.0Hz, 1H), 4.07 (dd, J = 10.6, 8.6Hz, 1H), 3.74 - 3.60 (m, 2H), 3.29 (s, 3H), 3.18 (dd, J = 16.5, 3.3Hz, 1H), 1.27 (t, J = 7.1 Hz, 3H).

### Example 6: Synthesis of 2-chloro-N-(1-cyano-1-methyl-ethyl)-5-[[5-(3,5-dichloro-4-fluoro-phenyl)-5-(trifluoromethyl)-4H-isoxazol-3-yl]amino]benzamide [I-75].

Step 1: To a solution of 2-chloro-5-[[5-(3,5-dichloro-4-fluoro-phenyl)-5-(trifluoromethyl)-4H-isoxazol-3-yl]amino]benzoic acid (100mg, 0.21 mmol, prepared as described above) and 2-amino-2-methyl-propanenitrile (22mg, 0.25mmol) in dichloromethane (8mL) at ambient temperature was added 2,4,6-tripropyl-1,3,5,2λ5,4λ5,6λ5-trioxatriphosphinane 2,4,6-trioxide (50 % in EtOAc, 810mg, 1.27mmol), followed by 4-dimethylaminopyridine (78mg, 0.64mmol). The reaction mixture was heated to 100°C in a microwave for 45min. Then HPLC-MS analysis showed that the reaction was completed. The reaction mixture was allowed to cool to ambient temperature, filtered and the filtrate was concentrated under reduced pressure. The crude product was purified by column chromatography (silica gel, cyclohexane / EtOAc 100:0 to 0:100, gradient) afforded 2-chloro-N-(1-cyano-1-methyl-ethyl)-5-[[5-(3,5-dichloro-4-fluoro-phenyl)-5-(trifluoromethyl)-4H-isoxazol-3-yl]amino]benzamide (53mg, 46%).
LCMS retention time: 1.295 min, m/z = 538 ([M+H]⁺)
¹H-NMR (400MHz, methanol-d₄, RT) δ 7.71 (d, J = 6.2Hz, 2H), 7.55 (d, J = 2.5Hz, 1H), 7.44 (dd, J = 8.8, 2.7Hz, 1H), 7.36 (d, J = 8.8Hz, 1H), 3.95 (d, J = 16.8Hz, 1H), 3.70 (d, J = 16.8Hz, 1H), 2.15 (s, 3H), 1.75 (s, 3H).

### Example 7: Synthesis 2-chloro-5-[[5-(3,5-dichloro-4-fluoro-phenyl)-5-(trifluoromethyl)-4H-isoxazol-3-yl]amino]-N-(4-fluoro-3-pyridyl)benzamide [I-79].

Step 1: To a solution of 2-chloro-5-[[5-(3,5-dichloro-4-fluoro-phenyl)-5-(trifluoromethyl)-4H-isoxazol-3-yl]amino]benzoic acid (100mg, 0.21mmol, prepared as described above) and (4-fluoro-3-pyridyl)ammonium chloride (38mg, 0.25mmol) in dichloromethane (6mL) at ambient temperature was added 2,4,6-tripropyl-1,3,5,2λ5,4λ5,6λ5-trioxatriphosphinane 2,4,6-trioxide (50 % in ethyl acetate, 810mg, 1.27mmol), followed by iPr₂NEt (82mg, 0.64mmol) and 4-dimethylaminopyridine (78mg, 0.64mmol). The reaction mixture was heated to 40°C in a microwave for 30min. Then HPLC-MS analysis showed that the reaction was completed. The reaction mixture was diluted with EtOAc (10mL) and washed with water (3x5mL). The organic phase was dried over Na₂SO₄, filtered and concentrated under reduced pressure. The crude product was purified by column chromatography (RP, acetonitrile / water 0:100 to 100:0, gradient) afforded 2-chloro-5-[[5-(3,5-dichloro-4-fluoro-phenyl)-5-(trifluoromethyl)-4H-isoxazol-3-yl]amino]-N-(4-fluoro-3-pyridyl)benzamide (16mg, 13%).
LCMS retention time: 1.205 min, m/z = 566 ([M+H]⁺)
¹H-NMR (400MHz, methanol-d₄, RT) δ 9.13 (d, J = 9.4Hz, 1H), 8.46 - 8.38 (m, 1H), 7.74-7.67 (m, 3H), 7.50 (dd, J = 8.8, 2.7Hz, 1H), 7.45 - 7.32 (m, 2H), 3.97 (d, J = 16.8Hz, 1H), 3.72 (d, J = 16.8 Hz, 1H).

### Example 8: Synthesis of 2-chloro-5-[[5-(3,5-dichloro-4-fluoro-phenyl)-5-(trifluoromethyl)-4H-isoxazol-3-yl]amino]-N-[1-(difluoromethylsulfonyl)azetidin-3-yl]benzamide [I-98].

Step1: To a solution of tert-butyl N-(azetidin-3-yl)carbamate (1.00 g, 5.81 mmol) and iPr₂NEt (1.88g, 14.5mmol) in dichloromethane (10mL) at 0°C under an atmosphere of argon was added a solution of difluoromethanesulfonyl chloride (1.75g, 11.6mmol) in dichloromethane (5mL) dropwise, and the resulting mixture was stirred at that temperature for 1h. Then the reaction mixture was warmed up to ambient temperature, stirred at that temperature for 1h, and then quenched with water (20mL). The organic phase was separated, dried over Na₂SO₄, filtered and concentrated under reduced pressure. Purification by column chromatography (NH₂-Column, cyclohexane / ethyl acetate 100:0 to 50:50) afforded tert-butyl N-[1-(difluoromethylsulfonyl)azetidin-3-yl]carbamate (386mg, 23%).
LCMS retention time: 0.889min, m/z = 256 ([M+H]⁺)
¹H-NMR (400MHz, CDC13, RT) δ 4.97 (s, 1H), 4.58 (s, 1H), 4.39 (t, J = 8.1 Hz, 2H), 4.15 - 4.07 (m, 2H), 1.45 (s, 9H).

Step 2: [1-(difluoromethylsulfonyl)azetidin-3-yl]ammonium chloride: tert-butyl N-[1-(difluoromethylsulfonyl)azetidin-3-yl]carbamate (386mg, 1.35mmol) was dissolved in HCI solution (4 N in 1,4-dioxane, 15mL) and stirred at ambient temperature for 17h. After that time, the reaction mixture was concentrated under reduced pressure, the residue re-dissolved in dichloromethane and concentrated under reduced pressure (3x) to give [1-(difluoromethylsulfonyl)azetidin-3-yl]ammonium chloride (367mg, quantitative). The crude product was used in the next step without further purification.
¹H-NMR (400MHz, methanol-d₄, RT) δ 4.46 (t, J = 7.8 Hz, 2H), 4.25 - 4.18 (m, 2H).

Step 3: To a solution of 2-chloro-5-[[5-(3,5-dichloro-4-fluoro-phenyl)-5-(trifluoromethyl)-4H-isoxazol-3-yl]amino]benzoic acid (100mg, 0.21 mmol, prepared as described in the examples above) and [1-(difluoromethylsulfonyl)azetidin-3-yl]ammonium chloride (59mg, 0.27mmol) in dichloromethane (6mL) at ambient temperature was added bromo(tripyrrolidin-1-yl)phosphonium hexafluorophosphate (124mg, 0.27mmol) and iPr₂NEt (110mg, 0.85mmol). The resulting reaction mixture was stirred at that temperature for 17h, and then concentrated under reduced pressure. Purification by column chromatography (RP, acetonitrile / water 0:100 to 100:0, gradient) afforded 2-chloro-5-[[5-(3,5-dichloro-4-fluoro-phenyl)-5-(trifluoromethyl)-4H-isoxazol-3-yl]amino]-N-[1-(difluoromethylsulfonyl)azetidin-3-yl]benzamide as a beige solid (69mg, 43%).
LCMS retention time: 1.304min, m/z = 641 ([M+H]⁺)
¹H-NMR (500MHz, methanol-d₄, RT) δ 7.69 (d, J = 6.1Hz, 2H), 7.59 (d, J = 2.7Hz, 1H), 7.40 (dd, J = 8.8, 2.7Hz, 1H), 7.35 (d, J = 8.8Hz, 1H), 4.45 (t, J = 8.1 Hz, 2H), 4.28 - 4.22 (m, 2H), 3.95 (d, J = 16.7Hz, 1H), 3.70 (d, J = 16.8Hz, 1H).

### Example 9: Synthesis of 2-chloro-5-[[5-(3,5-dichloro-4-fluoro-phenyl)-5-(trifluoromethyl)-4H-isoxazol-3-yl]amino]-N-[1-(trifluoromethylsulfonyl)azetidin-3-yl]benzamide [l-99].

Step1: To a solution of tert-butyl N-(azetidin-3-yl)carbamate (500mg, 2.90mmol) and iPr₂NEt (938mg, 7.26mmol) in dichloromethane (10mL) at 0°C under an atmosphere of argon was added a solution of trifluoromethanesulfonyl chloride (979mg, 5.81 mmol) in dichloromethane (5mL) dropwise, and the resulting mixture was stirred at that temperature for 1h. Then the reaction mixture was warmed to ambient temperature, stirred at that temperature for 1.5h, and then quenched with water (10mL). The organic phase was separated, dried over Na₂SO₄, filtered and concentrated under reduced pressure to give tert-butyl N-[1-(trifluoromethylsulfonyl)azetidin-3-yl]carbamate (806mg, 91%) as a white solid.
¹H-NMR (400MHz, CDCl₃, RT): δ 5.09 (s, 1H), 4.60 (s, 1H), 4.42 (t, J = 8.1Hz, 2H), 4.17 - 4.09 (m, 2H), 1.45 (s, 9H).

Step 2: tert-butyl N-[1-(trifluoromethylsulfonyl)azetidin-3-yl]carbamate (806mg, 2.65mmol) was dissolved in HCI (4N in 1,4-dioxane, 20ml) and stirred at ambient temperature overnight. The resulting precipitate was filtered off, washed with 1,4-dioxane and dried under reduced pressure to afford [1-(trifluoromethylsulfonyl)azetidin-3-yl]ammonium chloride (483mg, 76%) as a withe solid.
¹H-NMR (400MHz, DMSO-d₆, RT): δ 8.87 (s, 3H), 4.46 - 4.31 (m, 4H), 4.27 - 4.16 (m, 1H).

Step 3: To a solution of 2-chloro-5-[[5-(3,5-dichloro-4-fluoro-phenyl)-5-(trifluoromethyl)-4H-isoxazol-3-yl]amino]benzoic acid (50mg, 0.11 mmol) and [1-(trifluoromethylsulfonyl)azetidin-3-yl]ammonium chloride (51 mg, 0.21 mmol) in dichloromethane (6mL) at ambient temperature was added 2,4,6-tripropyl-1,3,5,2λ5,4λ5,6λ5-trioxatriphosphinane 2,4,6-trioxide (405mg, 0.64mmol) followed by iPr₂NET (55 mg, 0.42 mmol) and 4-dimethylaminopyridine (13mg, 0.11mmol). The resulting reaction mixture was stirred for 2h at ambient temperature. Then dichloromethane (5 ml) was added and the reaction mixture was washed with water (10ml). The organic phase was dried over Na₂SO₄ and concentrated under reduced pressure. Purification by column chromatography (silica gel, cyclohexane / ethyl acetate 100:0 to 60:40) afforded 2-chloro-5-[[5-(3,5-dichloro-4-fluoro-phenyl)-5-(trifluoromethyl)-4H-isoxazol-3-yl]amino]-N-[1-(trifluoromethylsulfonyl)azetidin-3-yl]benzamide (34mg, 49%) as a beige wax.
LCMS retention time: 1.365 min, m/z = 658 ([M+H]⁺)
¹H-NMR (400MHz, CDCl₃, RT) δ 7.52 - 7.44 (m, 2H), 7.37 - 7.28 (m, 2H), 7.15 (d, J = 8.9Hz, 1H), 4.85 - 4.74 (m, 1H), 4.47 (t, J = 8.3Hz, 2H), 4.33 (dd, J = 10.4, 3.9Hz, 2H), 3.84 (d, J = 16.5Hz, 1H), 3.49 (d, J = 16.5Hz, 1H), 1.91 (s, 1H), 1.43 (s, 1H).

### Example 10: Synthesis of 2-chloro-N-(1-cyanocyclopropyl)-5-[cyclopropylmethyl-[5-(3,5-dichlorophenyl)-5-(trifluoromethyl)-4H-isoxazol-3-yl]amino]benzamide [I-25].

Step 1: To a solution of 1-(3,5-dichlorophenyl)-2,2,2-trifluoro-ethanone (30.0g, 0.123mol) in THF (400 mL) at ambient temperature was added 2-diethoxyphosphorylacetonitrile (21.7g, 0.125 mmol), Et₃N (34.8g, 246mmol) and LiBr (10.8g, 0.123 mol), before the resulting reaction mixture was heated to 70 °C and stirred at that temperature for 16 hours. After that time, TLC analysis (petroleum ether / EtOAc = 10:1) showed that the reaction was completed. The mixture was poured into water (300 mL), extracted with ethyl acetate (3x150mL), and the combined organic extracts were washed with NaCl solution (2x150mL), dried over Na₂SO₄, filtered and concentrated. Purification by column chromatography (petroleum ether / EtOAc = 10:1) afforded (Z)-3-(3,5-dichlorophenyl)-4,4,4-trifluoro-but-2-enenitrile (29.0 g, yield 88%) as a brown oil.
¹H-NMR (400MHz, CDC13, RT): δ 7.49 - 7.55 (m, 1 H) 7.40 (d, J=1.38Hz, 1 H) 7.33 (s, 1 H) 5.96 - 6.31 (m, 1 H).

### Step 2: To a solution of (Z)-3-(3,5-dichlorophenyl)-4,4,4-trifluoro-but-2-enenitrile (29.0g,

0.104 mol) in MeOH (300 mL) at ambient temperature was added NaOMe (8.16g, 0.155mol) and hydroxyurea (8.61g, 0.114mol), before the reaction was heated to 80°C and stirred at that temperature for 16 hours. After that time, the mixture was poured into water (100 mL), extracted with ethyl acetate (3x100mL), and the combined organic extracts were washed with NaCl solution (100 mL, sat. aq.), dried over Na₂SO₄, filtered and concentrated to give 5-(3,5-dichlorophenyl)-5-(trifluoromethyl)-4H-isoxazol-3-amine (22g, 70%) as white solid.
¹H-NMR (400MHz, CDCl₃, RT): δ 7.45 (s,2H) 7.40 - 7.43 (m, 1 H) 4.07 (br s, 2 H) 3.68 - 3.74 (m, 1 H) 3.34 (d, J=16.4 Hz, 1 H).

Step 3: To a solution of 5-(3,5-dichlorophenyl)-5-(trifluoromethyl)-4H-isoxazol-3-amine (10.0g, 33.4mmol) in 1,4-dioxane (100mL) at ambient temperature was added Pd₂(dba)₃ (3.06g, 3.34 mmol, CAS-No 51364-51-3), Xantphos (2.90g, 5.02mmol, CAS-No 161265-03-8), Cs₂CO₃ (16.4g, 50.2mmol) and tert-butyl 5-bromo-2-chloro-benzoate (10.7g, 36.7mmol), before the reaction was heated to 80 °C and stirred at that temperature for 16 hours. Then LCMS and TLC analyses showed that the reaction was completed. The mixture was poured into water (80 mL), extracted with ethyl acetate (3x100 mL), and the combined organic extracts were washed with NaCl solution (80mL, sat. aq.), dried over Na₂SO₄, filtered and concentrated. Purification by column chromatography (petroleum ether / EtOAc=3:1) afforded tert-butyl 2-chloro-5-[[5-(3,5-dichlorophenyl)-5-(trifluoromethyl)-4H-isoxazol-3-yl]amino]benzoate (12.0g, 71%) as a brown solid.
¹H-NMR (400MHz, DMSO-d₆, RT): δ 9.61 (s, 1 H) 7.76 (d, J=2.43Hz, 2 H) 7.65 (s, 2 H) 7.44 - 7.48 (m, 2 H) 3.74 - 3.98 (m, 2 H) 1.54 (s, 9 H).

Step 4: To a solution of tert-butyl 2-chloro-5-[[5-(3,5-dichlorophenyl)-5-(trifluoromethyl)-4H-isoxazol-3-yl]amino]benzoate (1.00g, 1.96mmol) in DMF (10 mL) at 0°C was added NaH (0.157g, 3.92mmol) in portions, and the mixture was stirred at that temperature for 0.5 h. Bromomethylcyclopropane (0.397g, 2.94mmol) was added, and the reaction was allowed to warm to ambient temperature and was stirred at that temperature for 16 h. Then LCMS analysis showed that the reaction was completed. The mixture was poured into H₂O (10mL), extracted with ethyl acetate (3x10mL), and the combined organic extracts were washed NaCl solution (20mL, sat. aq.), dried over Na₂SO₄, filtered and concentrated. Purification by column chromatography (petroleum ether / EtOAc = 1:1) and Prep-HPLC afforded tert-butyl 2-chloro-5-[cyclopropylmethyl-[5-(3,5-dichlorophenyl)-5-(trifluoromethyl)-4H-isoxazol-3-yl]amino]-benzoate (700mg, 59%) as a brown solid.
¹H-NMR (400MHz, CDCl₃, RT): δ 7.44 - 7.56 (m, 2 H) 7.39 (s, 3 H) 7.16 - 7.26 (m, 1 H) 3.75 (ddd, J=6.62, 4.17, 2.51Hz, 1 H) 3.47 - 3.55 (m, 2 H) 3.08 - 3.18 (m, 1 H) 1.62 (s, 9 H) 1.01 - 1.13 (m, 1 H) 0.47 (d, J=7.91 Hz, 2 H) -0.01 - 0.21 (m, 2 H).

Step 5: To a solution of tert-butyl 2-chloro-5-[cyclopropylmethyl-[5-(3,5-dichlorophenyl)-5-(trifluoromethyl)-4H-isoxazol-3-yl]amino]-benzoate (700mg, 1.25mmol) in MeCN (8mL) and H₂O (1 mL) at ambient temperature was added l₂ (316mg, 1.25mmol), before the reaction mixture was heated to 80 °C and stirred at that temperature for 16 hours. Then LCMS and TLC analysis (petroleum ether / EtOAc = 3:1) showed that the reaction was completed. The mixture was poured into water (10mL), the pH of the aqueous phase was adjusted 3 with aq. HCI (2N) and extracted with ethyl acetate (3x20mL). The combined organic phases were washed with NaCl solution (10mL), dried over Na₂SO₄, filtered and concentrated to give 2-chloro-5-[cyclopropylmethyl-[5-(3,5-dichlorophenyl)-5-(trifluoromethyl)-4H-isoxazol-3-yl]amino]benzoic acid (0.30g, yield 47%) as a brown solid.
¹H-NMR (400MHz, DMSO-d₆, RT) δ 7.64 - 7.68 (m, 1 H) 7.39 - 7.59 (m, 3 H) 7.12 - 7.21 (m, 1 H) 6.96 - 7.03 (m, 1 H) 3.44 - 3.64 (m, 4 H) 0.43 - 0.59 (m, 1 H) 0.30 (br d, J=8.00 Hz, 2 H) 0.00 (br s, 2 H).

Step 6: To the solution of 2-chloro-5-[cyclopropylmethyl-[5-(3,5-dichlorophenyl)-5-(trifluoromethyl)-4H-isoxazol-3-yl]amino]benzoic acid (300mg, 0.592mmol) in MeCN (5mL) at ambient temperature was added NMI (195mg, 4.70mmol), TCFH (301mg, 0.892mmol), and 1-aminocyclopropanecarbonitrile (134mg, 2.10mmol), and the resulting reaction mixture was stirred at that temperature for 18h. After that time, LCMS analysis showed consumption of the starting material. The reaction mixture was quenched into water (10mL), extracted with EtOAc (2x20mL), and the combined organic extracts were washed with NaCl solution (40 mL, sat. aqueous), dried over Na₂SO₄, filtered and concentrated. Purification by column chromatography (petroleum ether / EtOAc=1:1) afforded the title 2-chloro-N-(1-cyanocyclopropyl)-5-[cyclopropyl-methyl-[5-(3,5-dichlorophenyl)-5-(trifluoromethyl)-4H-isoxazol-3-yl]amino]benzamide [I-25] (220 mg, 65%) as a white solid.
¹H-NMR (400MHz, DMSO-d₆, RT): δ ppm 9.34 (s, 1 H) 7.70 - 7.83 (m, 1 H) 7.51 - 7.61 (m, 3 H) 7.41 - 7.48 (m, 2 H) 3.51 (d, J=6.88Hz, 2 H) 1.54 - 1.60 (m, 2 H) 1.23 - 1.32 (m, 2 H) 0.91 - 1.04 (m, 1 H) 0.32 - 0.43 (m, 2 H)0.02 - 0.13 (m, 2 H).

### Example 11: Synthesis of 2-chloro-5-[[5-(3,5-dichloro-4-fluoro-phenyl)-5-(trifluoromethyl)-4H-isoxazol-3-yl]amino]-N-(1-oxothietan-3-yl)benzamide [I-96].

Step 1: To a solution of 2-chloro-5-[[5-(3,5-dichloro-4-fluoro-phenyl)-5-(trifluoromethyl)-4H-isoxazol-3-yl]amino]-N-(thietan-3-yl)benzamide (161mg, 0.3mmol; synthesized analogously as described in the examples above) in dichloromethane (10mL) at -78°C was added 3-chloroperoxybenzoic acid (66mg, 0.3mmol). The cooling bath was removed, and the resulting mixture was allowed to warm to -20°C over 30min. The reaction mixture was quenched with Na₂S₂O₃ solution (sat. aq., 2mL) and NaHCO₃ solution (sat. aq., 10mL) and extracted with ethyl acetate 2×15ml). The organic phase was dried over Na₂SO₄, filtered and concentrated under reduced pressure. The crude residue was recrystallized from dichloromethane to give 2-chloro-5-[[5-(3,5-dichloro-4-fluoro-phenyl)-5-(trifluoromethyl)-4H-isoxazol-3-yl]amino]-N-(1-oxothietan-3-yl)benzamide (102 mg, 62 % yield) as a white solid.
LC/MS retention time: 1.090min, m/z = 519 ([M+H]⁺)
¹H-NMR (400MHz, methanol-d4, RT) δ 7.70 (d, J = 6.2Hz, 2H), 7.60 (d, J = 2.5Hz, 1H), 7.42-7.35 (m, 2H), 4.56 (tt, J = 10.2, 7.5Hz, 1H), 4.22 (ddt, J = 11.7, 7.4, 2.6Hz, 2H), 3.95 (d, J = 16.8Hz, 1H), 3.82-3.66 (m, 2H), 3.35 (d, J = 3.1Hz, 1H).

### Example 12: Synthesis of N-(3-amino-2,4-difluoro-phenyl)-2-chloro-5-[[5-(3,5-dichlorophenyl)-5-(trifluoromethyl)-4H-isoxazol-3-yl]amino]benzamide [I-4].

Step 1: To a solution of 2-chloro-5-[[5-(3,5-dichlorophenyl)-5-(trifluoromethyl)-4H-isoxazol-3-yl]amino]benzoic acid (18.0g, 39.6mmol, prepared analogously as described in the above examples) and tert-butyl N-(3-amino-2,6-difluoro-phenyl)-N-tert-butoxycarbonyl-carbamate (12.0 g, 34.8 mmol, prepared as described in WO2016/168059) in MeCN (100 mL) was added TFCH (14.67g, 52.3mmol) and NMI (3.79 g, 69.8 mmol), and the resulting reaction mixture was stirred at 50°C for 16 hours. Then TLC analysis (petroleum ether / EtOAc = 3:1) showed that the reaction was completed. The mixture was poured into water (200mL), extracted with ethyl acetate (2x20mL), and the combined organic extracts were washed with water (3×20 mL) and NaCl solution (2x20mL), dried over Na₂SO₄, filtered and concentrated. The residue was purified by column chromatography (petroleum ether / EtOAc = 3:1) to give tert-butyl N-tert-butoxycarbonyl-N-[3-[[2-chloro-5-[[5-(3,5-dichlorophenyl)-5-(trifluoromethyl)-4H-isoxazol-3-yl]amino]benzoyl]amino]-2,6-difluoro-phenyl]carbamate (12.5g, 46%).
¹H-NMR (400MHz, DMSO-d₆, RT): δ 10.4 (1H, s), 9.58 (1H, s), 7.75-7.62 (1H, m), 7.621 (3H, m), 7.44 (2H, s), 7.25-7.20 (1H, s), 6.73-6.67 (1H, t, J = 8.8Hz) 3.93 (2H, q, J = 17.2, 27.2Hz) 1.37 (18H, m).

Step 2: To a solution of tert-butyl N-tert-butoxycarbonyl-N-[3-[[2-chloro-5-[[5-(3,5-dichlorophenyl)-5-(trifluoromethyl)-4H-isoxazol-3-yl]amino]benzoyl]amino]-2,6-difluoro-phenyl]carbamate (24g, 31mmol) in EtOAc (50mL) was added EtOAc/HCl (300mL), and the reaction was stirred at ambient temperature for 3 hours. After that time, TLC analysis (petroleum ether / EtOAc = 3:1) showed that the reaction was completed, and the mixture was filtered, and the filtrate was concentrated. The residue was recrystallized from MTBE (10mL) to give N-(3-amino-2,4-difluoro-phenyl)-2-chloro-5-[[5-(3,5-dichlorophenyl)-5-(trifluoromethyl)-4H-isoxazol-3-yl]amino]benzamide (13.0 g, 73%).
¹H-NMR (400MHz, DMSO-d₆, RT): δ 10.10 (1H, s), 9.51 (1H, s), 7.79-7.65 (2H, m), 7.61 (3H, m), 7.40 (1H, s), 6.92-6.87 (1H, m), 6.87-6.75 (1H, m), 5.30 (2H, s), 3.99-3.33 (2H, m).

### Example 13: Synthesis of 2-chloro-5-[[5-(3,5-dichlorophenyl)-5-(trifluoromethyl)-4H-isoxazol-3-yl]amino]-N-[3-[(2,2-difluoroacetyl)amino]-2,4-difluoro-phenyl]benzamide [I-5].

Step 1: To a solution of N-(3-amino-2,4-difluoro-phenyl)-2-chloro-5-[[5-(3,5-dichlorophenyl)-5-(trifluoromethyl)-4H-isoxazol-3-yl]amino]benzamide (700mg, 1.20mmol, prepared as described above) and 2,2-difluoroacetic acid (190mg, 1.98mmol) in MeCN (10mL) was added NMI (380mg, 4.63mmol) and TFCH (556mg, 1.98mmol), and the resulting reaction was heated to 80°C and stirred at that temperature for 16 hours. Then TLC analysis (petroleum ether / EtOAc = 3:1) showed that the reaction was completed. The mixture was poured into water (20 mL), extracted with ethyl acetate (2 × 20 mL), and the combined organic extracts were washed with water (3 × 20 mL) and NaCl solution (20mL, sat. aq.), dried over Na₂SO₄, filtered and concentrated. The residue was purified by column chromatography (petroleum ether / EtOAc = 3:1) and Prep-HPLC to give 2-chloro-5-[[5-(3,5-dichlorophenyl)-5-(trifluoromethyl)-4H-isoxazol-3-yl]amino]-N-[3-[(2,2-difluoroacetyl)amino]-2,4-difluoro-phenyl]benzamide [I-5] (90 mg, 23%).
¹H-NMR (400MHz, DMSO-d₆, RT) δ10.79 (1H, s), 10.45 (1H, s), 9.63 (1H, s), 7.79-7.78 (1H, s), 7.71-7.69 (1H, s), 7.65 (3H, m), 7.48 -7.48(2H, m), 7.27-7.24 (1H, s), 6.68-6.41 (1H, m), 3.99-3.86 (2H, m).

### Example 14: Synthesis of 2-chloro-5-[[5-(3,5-dichlorophenyl)-5-(trifluoromethyl)-4H-isoxazol-3-yl]-methyl-amino]-N-[1-(1-methylpyrazol-4-yl)cyclopropyl]benzamide [I-102].

Step 1: To a solution of 5-(3,5-dichlorophenyl)-5-(trifluoromethyl)-4H-isoxazol-3-amine (8.00g, 26.8mmol; synthesized analogously as described in the examples above) and Et₃N (11.0g, 107mmol) in THF (80mL) was added Boc₂O (17.6 g, 80.5 mmol) and DMAP (654mg, 5.36 mmol), and the resulting reaction mixture was stirred at 20°C for 16 hours. Then TLC analysis (petroleum ether/EtOAc = 3:1) showed that the reaction was complete. The mixture was poured into water (120mL), extracted with ethyl acetate (2×30mL), and the combined organic phases were washed with NaCl solution (sat. aq., 30mL), dried over Na₂SO₄, filtered and concentrated under reduced pressure. Purification by column chromatography (petroleum ether/EtOAc = 3:1) gave tert-butyl N-tert-butoxycarbonyl-N-[5-(3,5-dichlorophenyl)-5-(trifluoromethyl)-4H-isoxazol-3-yl]carbamate (12.7 g, 96% yield) as white solid.

Step 2: To a solution of tert-butyl N-tert-butoxycarbonyl-N-[5-(3,5-dichlorophenyl)-5-(trifluoromethyl)-4H-isoxazol-3-yl]carbamate (15.0g, 30.1 mmol) in EtOH (150mL) at ambient temperature was added N₂H₄·H₂O (3.80g, 75.3mmol), and the reaction mixture was stirred at that temperature for 3 hours. Then TLC analysis (petroleum ether/EtOAc = 3:1) showed that the reaction was complete. The mixture was concentrated, and the residue was poured into water (40mL), extracted with ethyl acetate (2×15mL), and the combined organic phases were washed with NaCl solution (30mL, sat. aq.), dried over Na₂SO₄, filtered and concentrated under reduced pressure. Purification by column chromatography (petroleum ether/EtOAc = 3:1) afforded tert-butyl N-[5-(3,5-dichlorophenyl)-5-(trifluoromethyl)-4H-isoxazol-3-yl]carbamate (9.08 g, 75%) as a white solid.

Step 3: To a solution of tert-butyl N-[5-(3,5-dichlorophenyl)-5-(trifluoromethyl)-4H-isoxazol-3-yl]carbamate (6.00g, 15.1mmol) in MeCN (60mL) was added K₂CO₃ (6.30g, 45.3mmol) and Mel (4.40g, 30.1mmol), before the reaction mixture was heated to 60 °C and stirred at that temperature for 16 hours. After that time, TLC analysis (petroleum ether/EtOAc = 3:1) showed that the reaction was complete. The mixture was poured into water (100 mL), extracted with ethyl acetate (3×20mL), and the combined organic phases were washed with NaCl solution (30mL, sat. aq.), dried over Na₂SO₄, filtered and concentrated under reduced pressure. Purification by column chromatography (petroleum ether/EtOAc = 3:1) to give tert-butyl N-[5-(3,5-dichlorophenyl)-5-(trifluoromethyl)-4H-isoxazol-3-yl]-N-methyl-carbamate (5.58g, 94% yield) as brown solid.

Step 4: To a solution of tert-butyl N-[5-(3,5-dichlorophenyl)-5-(trifluoromethyl)-4H-isoxazol-3-yl]-N-methyl-carbamate (8.55g, 2.08mmol) in EtOAc (30mL) at 0°C was added HCl in EtOAc (4 M, 200mL), before the reaction mixture was allowed to warm to ambient temperature and stirred at that temperature for 16 hours. Then TLC analysis (petroleum ether/EtOAc = 3:1) showed that the reaction was complete. The mixture was concentrated, and the crude product was purified by column chromatography (petroleum ether/EtOAc = 3:1) to give 5-(3,5-dichlorophenyl)-N-methyl-5-(trifluoromethyl)-4H-isoxazol-3-amine (5.55g, 85%) as a white solid.
¹H-NMR (400MHz, CDCl₃, RT) δ 7.47 (br s, 2H), 7.40 (s, 1H), 3.75 (br s, 1H), 3.36 (br s, 1H), 2.93 (br s, 3H).

Step 5: To a solution of 5-(3,5-dichlorophenyl)-N-methyl-5-(trifluoromethyl)-4H-isoxazol-3-amine (2.0g, 6.39mmol) and methyl 5-bromo-2-chloro-benzoate (1.59g, 6.39mmol) in 1,4-dioxane (40mL) under an atmosphere of argon at ambient temperature was added caesium carbonate (6.24g, 19.16mmol) followed by 4,6-bis(diphenylphosphino)-9,9-dimethylxanthene (443mg, 0.77mmol) and tris(dibenzylideneacetone)dipalladium(0) (292 mg, 0.32 mmol). The reaction mixture was heated to 80°C and stirred at that temperature overnight. The mixture was cooled to ambient temperature, diluted with ethyl acetate (50mL), and the organic phase was washed with NH₄Cl solution (sat. aq., 40mL), NaCl solution (sat. aq., 40mL), dried over Na₂SO₄, filtered and concentrated under reduced pressure. Purification by column chromatography (silica gel, cyclohexane/ethyl acetate 100:0 to 65:35) afforded methyl 2-chloro-5-[[5-(3,5-dichlorophenyl)-5-(trifluoromethyl)-4H-isoxazol-3-yl]-methyl-amino]benzoate (3.17 g, quantitative).
LC/MS retention time: 1.413 min, m/z = 481 ([M+H]⁺)
¹H-NMR (400MHz, CDCl₃, RT) δ 7.61 (d, J = 2.7Hz, 1H), 7.48 (d, J = 8.6Hz, 1H), 7.40 (q, J = 1.6Hz, 3H), 7.29 - 7.19 (m, 1H), 3.95 (s, 3H), 3.63 (d, J = 16.5Hz, 1H), 3.29 (s, 3H), 3.20 (d, J = 16.6 Hz, 1H).

Step 6: To a solution of methyl 2-chloro-5-[[5-(3,5-dichlorophenyl)-5-(trifluoromethyl)-4H-isoxazol-3-yl]-methyl-amino]benzoate (3.17g, 6.58mmol) in THF (20mL) and methanol (20mL) at ambient temperature was added a solution of lithium hydroxide (2.76g, 65.81mmol) in water (10mL) dropwise. After 4 hours, HPLC-MS analysis showed that the reaction was complete. The reaction mixture was treated with HCl (aqueous, 2N) until pH1 was reached and concentrated under reduced pressure. The residue was dissolved in water (5mL), and the aqueous phase was extracted with ethyl acetate (3x5mL). The combined organic extracts were washed with water (5 mL) and NaCl solution (sat. aqueous, 5mL), dried over Na₂SO₄, filtered and concentrated under reduced pressure to give 2-chloro-5-[[5-(3,5-dichlorophenyl)-5-(trifluoromethyl)-4H-isoxazol-3-yl]-methyl-amino]benzoic acid (2.52g, 82%) as a yellow solid.
LC/MS retention time: 1.266 min, m/z = 467 ([M+H]⁺).
¹H-NMR (400MHz, methanol-d₄, RT) δ 7.71 (d, J = 2.7Hz, 1H), 7.52 (d, J = 8.2Hz, 4H), 7.40 (dd, J = 8.6, 2.8Hz, 1H), 3.82 (d, J = 17.0Hz, 1H), 3.58 (d, J = 17.0Hz, 1H), 3.28 (s, 3H).

Step 7: To a solution of 2-chloro-5-[[5-(3,5-dichlorophenyl)-5-(trifluoromethyl)-4H-isoxazol-3-yl]-methyl-amino]benzoic acid (100mg, 0.21 mmol) and 1-(1-methyl-1H-pyrazol-4-yl)cyclopro-panamine (35mg, 0.26mmol) in dichloromethane (6mL) at ambient temperature was added 2,4,6-tripropyl-1,3,5,2λ5,4λ5,6λ5-trioxatriphosphinane 2,4,6-trioxide (50% in EtOAc, 816 mg, 1.28mmol) and DMAP (78mg, 0.64mmol), and the resulting reaction mixture was stirred at that temperature for 3h. After that time, dichloromethane (5mL) was added, the reaction mixture was washed with water (10mL), and the organic phase was dried over Na₂SO₄, filtered and concentrated under reduced pressure. Purification by column chromatography (silica gel, cyclohexane / ethyl acetate 100:0 to 0:100) afforded 2-chloro-5-[[5-(3,5-dichlorophenyl)-5-(trifluoromethyl)-4H-isoxazol-3-yl]-methyl-amino]-N-[1-(1-methylpyrazol-4-yl)cyclopropyl]benzamide (96mg, 77 %).
LC/MS retention time: 1.260min, m/z = 588 ([M+H]⁺)
¹H-NMR (500MHz, CDCl₃, RT) δ 7.47 - 7.41 (m, 2H), 7.39 (s, 4H), 7.15 (dd, J = 8.6, 2.8Hz, 1H), 7.06 (s, 1H), 3.83 (s, 3H), 3.64 (d, J = 16.6Hz, 1H), 3.26 (s, 3H), 3.21 (d, J = 16.6 Hz, 1H), 2.00 (s, 1H), 1.30 - 1.24 (m, 2H), 1.21 - 1.15 (m, 2H).

### Example 15: Synthesis of ethyl N-[4-chloro-3-[(1-cyanocyclopropyl)carbamoyl]phenyl]-N-[5-(3,5-dichlorophenyl)-5-(trifluoromethyl)-4H-isoxazol-3-yl]carbamate [I-28] and ethyl N-[4-chloro-3-[(1-cyanocyclopropyl)-ethoxycarbonyl-carbamoyl]-phenyl]-N-[5-(3,5-dichlorophenyl)-5-(trifluoromethyl)-4H-isoxazol-3-yl]carbamate [I-142].

Step 1: To a solution of 2-chloro-N-(1-cyanocyclopropyl)-5-[[5-(3,5-dichlorophenyl)-5-(trifluoromethyl)-4H-isoxazol-3-yl]amino]benzamide (1.00g, 1.98mmol, synthesized analogously as described in the examples above) in DMF (10mL) at 0°C was added NaH (93.0mg, 3.88mmol) in portions, and the reaction mixture was stirred at that temperature for 0.5 hours. Ethyl chloroformate (413mg, 3.88mmol) was added dropwise at 0°C, before the reaction was allowed to warm to ambient temperature and stirred at that temperature for 16 h. Then LCMS analysis showed that the reaction was completed. The mixture was poured into H₂O (10mL), extracted with ethyl acetate (3×10mL), and the combined organic extracts were washed with NaCl solution (sat. aq., 10mL), dried over Na₂SO₄, filtered and concentrated under reduced pressure. Separation and purification by column chromatography (petroleum ether/ EtOAc = 1:1) and preparative HPLC afforded ethyl N-[4-chloro-3-[(1-cyanocyclopropyl)carbamoyl]phenyl]-N-[5-(3,5-dichlorophenyl)-5-(trifluoromethyl)-4H-isoxazol-3-yl]carbamate [I-28] (140mg, 28%) as white solid and ethyl N-[4-chloro-3-[(1-cyanocyclopropyl)-ethoxycarbonyl-carbamoyl]-phenyl]-N-[5-(3,5-dichlorophenyl)-5-(trifluoromethyl)-4H-isoxazol-3-yl]carbamate (166mg, 29%).

### Ethyl N-[4-chloro-3-[(1-cyanocyclopropyl)carbamoyl]phenyl]-N-[5-(3,5-dichlorophenyl)-5-(tri-fluoromethyl)-4H-isoxazol-3-yl]carbamate [I-28]:

¹H-NMR (400MHz, DMSO-d₆, RT) δ 9.51 (s, 1 H) 7.80 (t, J=1.88Hz, 1 H) 7.56 - 7.69 (m, 3 H) 7.40 - 7.55 (m, 2 H) 4.50 (d, J=18.51Hz, 1 H) 4.26 (d, J=18.51Hz, 1 H) 4.11 - 4.18 (m, 2 H) 1.50 - 1.62 (m, 2 H) 1.20 - 1.32 (m, 2 H) 1.16 (t, J=7.07 Hz, 3 H).

### Ethyl N-[4-chloro-3-[(1-cyanocyclopropyl)-ethoxycarbonyl-carbamoyl]-phenyl]-N-[5-(3,5-dichlorophenyl)-5-(trifluoromethyl)-4H-isoxazol-3-yl]carbamate [I-142]:

¹H-NMR (400MHz, DMSO-d₆, RT) δ ppm 7.79 (t, J=1.81Hz, 1 H) 7.63 (d, J=1.50 Hz, 2 H) 7.56 - 7.60 (m, 2 H) 7.48 - 7.52 (m, 1 H) 4.47 (d, J=18.51Hz, 1 H) 4.23 (d, J=18.51Hz, 1 H) 4.14 (q, J=7.00Hz, 2 H) 4.05 (q, J=7.21Hz, 2 H) 1.85 - 1.90 (m, 2 H) 1.47 - 1.58 (m, 2 H) 1.14 (t, J=7.07Hz, 3 H) 0.93 (t, J=7.13Hz, 3 H).

### Example 16: Synthesis of 2-chloro-5-[[5-[chloro(difluoro)methyl]-5-(3,5-dichlorophenyl)-4H-isoxazol-3-yl]amino]-N-(2,4-difluorophenyl)benzamide [I-17].

Step 1: To a solution 1,3-dichloro-5-iodo-benzene (40.0g, 0.147mol) in MTBE (600mL) at -20°C was added iPrMgCl·LiCl solution (1.3M in THF, 150mL, 0.191mol) dropwise, and the resulting mixture was stirred at that temperature for 2h. Then, methyl 2-chloro-2,2-difluoro-acetate (32.0g, 0.220mol) was added dropwise to the above mixture, the cooling bath was removed, and stirring was continued at ambient temperature for 16 h. After that time, TLC analysis (PE/EtOAc = 10:1) showed the reaction was completed. The resulting mixture was poured in water (500mL), the aqueous phase was extracted with MTBE (2x200 mL), and the combined organic extracts were washed with water (300mL) and NaCl solution (sat. aq., 300mL), dried over Na₂SO₄, filtered and concentrated under reduced pressure. Purification by column chromatography on silica gel (PE/EtOAc= 100:1) afforded 2-chloro-1-(3,5-dichlorophenyl)-2,2-difluoroethanone (10g, 26%) as a yellow oil.
¹H NMR (400MHz, CDCl₃, RT) δ ppm 7.97 (s, 2 H) 7.67 - 7.71 (m, 1 H)

Step 2: To a mixture of 2-chloro-1-(3,5-dichlorophenyl)-2,2-difluoroethanone (10g, 38.6mmol) and Ph₃PMeBr (20g, 57.9mmol) in THF (200mL) at 0°C under an atmosphere of N₂ was slowly added t-BuOK (6.4g, 58mmol), the cooling bath was removed, and the reaction mixture stirred at ambient temperature for 16 h. Then TLC analysis (PE/EtOAc = 10:1) showed the reaction was completed. The reaction mixture was poured into water (500 mL), the aqueous phase was extracted with MTBE (2x200 mL), and the combined organic extracts were washed with water (300 mL) and NaCl solution (sat. aqu., 300mL), dried over Na₂SO₄, filtered and concentrated to dryness. Purification by column chromatography on silica gel (PE/EtOAc = 100:1) afforded 1,3-dichloro-5-[1-[chloro(difluoro)methyl]vinyl]benzene (18g, 61%) as yellow oil.
¹H-NMR (400MHz, CDCl₃, RT) δ ppm 7.41 (t, J=1.8Hz, 1 H) 7.36 (d, J=1.8Hz, 2 H) 6.00 (s, 1 H) 5.66 (t, J=1.6Hz, 1 H).

Step 3: To a solution of 1,3-dichloro-5-[1-[chloro(difluoro)methyl]vinyl]benzene (5.0 g, 19 mmol) in EtOAc (100mL) at ambient temperature under an atmosphere of N₂ was added Na-HCO₃ (2.5g, 29mmol) and ethyl 2-chloro-2-hydroxyimino-acetate (4.4g, 29mmol) in one portion, before the reaction mixture was heated to 50°C and the stirred at that temperature for 16h. Then TLC analysis (PE/EtOAc = 5:1) showed that the reaction was complete. The reaction mixture was poured in water (500mL), the aqueous phase was extracted with MTBE (2×200mL), and the combined organic extracts were washed with water (300mL) and NaCl solution (sat. aq., 300mL), dried over Na₂SO₄, filtered and concentrated under reduced pressure. Purification by column chromatography on silica gel (PE/EtOAc= 10:1) afforded ethyl 5-[chloro(difluoro)methyl]-5-(3,5-dichlorophenyl)-4H-isoxazole-3-carboxylate (4.07 g, 56%) as yellow solid.
1H-NMR (400MHz, CDCl₃, RT): δ ppm 7.41 - 7.52 (m, 3 H) 4.37 (q, J=7.1Hz, 2 H) 4.01 (d, J=18.5Hz, 1 H) 3.60 (d, J=18.5Hz, 1 H) 1.38 (t, J=7.1Hz, 3 H).

Step 4: To a solution of ethyl 5-[chloro(difluoro)methyl]-5-(3,5-dichlorophenyl)-4H-isoxazole-3-carboxylate (10g, 22mol) in THF (150 mL) at 0°C was added LiOH·H₂O (4.3g, 107mmol) in H₂O (40 mL) dropwise, and stirring was continued for further 4 hour. Then TLC analysis (PE/EtOAc = 5:1) showed that the reaction was complete. The resulting mixture was poured into water (30mL), and the THF was removed under reduced pressure at 35°C. The pH of the residue was adjusted to ca. 2-3 with 2N aq. HCl (50mL) at 0°C, the aqueous phase was extracted with EtOAc (2x200 mL), and the combined organic extracts were washed with NaCl solution (sat. aqueous, 50mL), dried over Na₂SO₄, filtered and concentrated under reduced pressure to give 5-[chloro(difluoro)methyl]-5-(3,5-dichlorophenyl)-4H-isoxazole-3-carboxylic acid (crude) as a yellow solid, which could be used in the next step without further purification.
¹H-NMR (400MHz, DMSO-d₆, RT): δ ppm 8.01 (s, 1 H) 7.74 (s, 3 H) 3.93 - 4.01 (m, 1 H) 3.36 (br d, J=16.9Hz, 1 H).

Step 5: To a solution of 5-[chloro(difluoro)methyl]-5-(3,5-dichlorophenyl)-4H-isoxazole-3-carboxylic acid (3.0g, 8.1mmol) in THF (60mL) at ambient temperature was added iPr₂NEt (2.25 g, 17.4mmol), 2,4,6-tripropyl-1,3,5,2λ5,4λ5,6λ5-trioxatriphosphinane 2,4,6-trioxide ("T3P") (7.2g, 11mmol), and (H₃C)₃SiN₃ (1.3 g, 11 mmol) in one portion, and the reaction mixture was stirred at that temperature for 30 min. Then, t-BuOH (2.5g, 34.8mmol) was added and the mixture was heated to 70 °C and stirred at that temperature for 16 h. The resulting mixture was poured into water (500mL), the aqueous phase was extracted with EtOAc (2x200 mL), and the combined organic extracts were washed with water (300 mL) and NaCl solution (sat. aq., 300mL), dried over Na₂SO₄, filtered and concentrated to under reduced pressure. Purification by column chromatography on silica gel (PE/EtOAc = 5:1) afforded tert-butyl N-[5-[chloro-(difluoro)methyl]-5-(3,5-dichlorophenyl)-4H-isoxazol-3-yl]carbamate (1.9g, 56%) as a yellow solid.
¹H-NMR (400MHz, CDCl₃, RT): δ ppm 7.48 (s, 2 H) 7.41 (s, 1 H) 7.11 (br s, 1 H) 4.28 (d, J=18.4Hz, 1 H) 3.88 (d, J=18.5 Hz, 1 H) 1.50 (s, 9 H).

Step 6: To a solution of (5.0g, 12mmol) in EtOAc (50mL) at ambient temperature was added HCl/EtOAc (100mL) in one portion, and the reaction mixture was stirred at that temperature for 4h. The reaction mixture was concentrated under reduced pressure and the residue was poured in NaHCO₃ solution (sat. aqueous, 50mL), the aqueous phase was extracted with EtOAc (2×100mL), and the combined organic extracts were washed with water (300mL) and NaCl solution (sat. aq., 300mL), dried over Na₂SO₄, filtered and concentrated to dryness. The residue was triturated by n-hexane (50mL) then filtered and the filter cake was concentrated to afford 5-[chloro(difluoro)methyl]-5-(3,5-dichlorophenyl)-4H-isoxazol-3-amine (2.5g, 67%) as a light-yellow solid.
¹H-NMR (400MHz, DMSO-d₆, RT) δ ppm 7.74 (s, 1 H) 7.58 (s, 2 H) 6.00 (s, 2 H) 3.61 - 3.69 (m, 1 H) 3.48 - 3.55 (m, 1 H).

This product could then be transformed further into e.g. the compound of formula I-17, analogously as described in the above examples.

### Example 17: tert-Butyl 5-bromo-2-(trifluoromethoxy)benzoate:

Step 1: To iPr₂NLi solution (2M in THF, 24.9mL) at -60°C under an atmosphere of N₂ was added a solution of 1-bromo-4-(trifluoromethoxy)benzene (10.0g, 41.5mmol) in THF (60.0mL) slowly, and the resulting mixture was stirred at that temperature for 2 hours. Then, solid CO₂ (18.3g, 415mmol) was added, and the mixture was allowed to warm to ambient temperature and stirred at that temperature for 16 hours. Then TLC analysis (petroleum ether/ EtOAc = 5/1, product Rf = 0.1) indicated full conversion. The solvent was distilled off, and the crude product was re-dissolved in aqueous NaOH (1N, 15.0mL), the aqueous phase was washed with DCM (3×20mL), and the pH adjusted to ca. 3-4 with aq. HCl solution (4M). The resulting suspension was filtered, and the filter cake washed with H₂O (2 × 5.0 mL), and dried in vacuo to give 5-bromo-2-(trifluoromethoxy)benzoic acid (5.70g, 48%) as a white solid.
¹H-NMR (400MHz, DMSO-d₆, RT) δ 8.04 (d, J = 2.80Hz, 1H), 7.92 (dd, J = 2.40Hz, 1H), 7.47-7.45 (m, 1H).

Step 2: To a suspension of MgSO₄ (8.78g, 73.0mmol) in CH₂Cl₂ (30mL) at ambient temperature was added H₂SO₄ (1.79g, 18.2mmol, 973uL), followed after 10 min by 5-bromo-2-(trifluoro-methoxy)benzoic acid (5.20 g, 18.2 mmol) and t-BuOH (6.76g, 91.2mmol, 8.72mL). The resulting mixture was stirred at ambient temperature for 48 hours, after which time TLC analysis (petroleum ether/ EtOAc = 5/1, product Rf = 0.8) indicated full conversion of the starting material. The mixture was quenched with NaHCO₃ solution (sat. aqueous, 50 mL), extracted with ethyl acetate (3×25mL), and the combined organic phases were dried over Na₂SO₄, filtered and concentrated under reduced pressure. Purification by column chromatography (SiO₂, petroleum ether/ EtOAc = 50/1 to 3/1) afforded tert-butyl 5-bromo-2-(trifluoromethoxy)benzoate (4.20g, 68%) as a light yellow oil.
¹H-NMR (400MHz, CDCl₃, RT): δ 8.00 (d, J = 2.40Hz, 1H), 7.64 (dd, J = 2.40 Hz, 1H), 7.18 (d, J = 8.40Hz, 1H), 1.59 (s, 9H).

This product could then be transformed further into e.g. the compounds of formula I-150, I-159, I-167, I-172, and I-179, analogously as described in the above examples.

### Example 18: Synthesis of 2-cyano-3-[[5-(3,5-dichlorophenyl)-5-(trifluoromethyl)-4H-isoxazol-3-yl]amino]benzoic acid.

Step 1: To a solution of methyl 3-bromo-2-iodo-benzoate (30.0 g, 124 mmol) in THF (60.0 mL) and H₂O (60.0 mL) at ambient temperature was added LiOH·H₂O (5.24 g, 124 mmol), and the resulting mixture was stirred at that temperature for 12 hours. Then the pH of the mixture was adjusted to 1-2 with 1 N HCl, the aqueous phase was exacted with EtOAc (2×200mL), and the combined organic extracts were dried over Na₂SO₄, filtered and concentrated under reduced pressure to give 3-bromo-2-iodo-benzoic acid (27.0g, 91%) as a yellow oil.
¹H-NMR (400MHz, CDCl₃, RT): δ 7.84-7.82 (m, 1H), 7.74-7.72 (m, 1H), 7.33-7.30 (m, 1H).

Step 2: To a suspension of MgSO₄ (39.7g, 330mmol) in CH₂Cl₂ (190mL) at ambient temperature was added H₂SO₄ (8.10g, 82.5mmol), followed after 15 min by 3-bromo-2-iodo-benzoic acid (27.0g, 82.5mmol) and t-BuOH (30.6g, 412mmol), and the resulting mixture was stirred at that temperature for 16 hours. Then the mixture was poured into NaHCO₃ solution (sat. aqueous, 150mL), the aqueous phase was exacted with EtOAc (2×100mL), and the combined organic extracts were dried over Na₂SO₄, filtered and concentrated under reduce pressure. Purification by column chromatography (SiO₂, petroleum ether/EtOAc = 100/1 to 1/1) afforded tert-butyl 3-bromo-2-iodo-benzoate (29.0g, 91%) as a white solid.
¹H-NMR (400MHz, CDCl₃, RT): δ 7.70-7.67 (m, 1H), 7.37-7.34 (m, 1H), 7.24 (d, J = 8.0Hz, 1H), 1.62 (s, 9H).

Step 3: To a solution of tert-butyl 3-bromo-2-iodo-benzoate (29.0g, 75.7mmol) in N-methyl-2-pyrrolidone (300mL) at ambient temperature was added CuCN (8.14g, 90.8mmol), and the resulting mixture was heated to 60 °C and stirred at that temperature for 5 hours. After that time, the reaction mixture was filtered, the filter cake was washed with EtOAc (3×70mL), and the combined filtrates were concentrated under reduce pressure. Purification by column chromatography (SiO₂, PE/EtOAc = 100/1 to 3/1) afforded tert-butyl 3-bromo-2-cyano-benzoate (18.0g, 63.1 mmol, 83 %) as a white solid.
¹H-NMR (400MHz, CDCl₃, RT): δ 8.01-7.99 (m, 1H), 7.87-7.85 (m, 1H), 7.52-7.48 (m, 1H), 1.65 (s, 9H).

Step 4: To a solution of 5-(3,5-dichlorophenyl)-5-(trifluoromethyl)-4H-isoxazol-3-amine (10.0 g, 33.4 mmol) and tert-butyl 3-bromo-2-cyano-benzoate (9.91g, 35.1mmol) in 1,4-dioxane (60.0mL) at ambient temperature under an atmosphere of N₂ was added Xantphos (2.90g, 5.02mmol), Cs₂CO₃ (16.3g, 50.1mmol) and Pd₂(dba)₃ (3.06g, 3.34mmol), and the resulting mixture was heated to 80°C and stirred at that temperature for 18 hours. The reaction mixture was filtered, the filter cake was washed with EtOAc (2×80mL), and the combined filtrates were concentrated under reduced pressure. Purification column chromatography (SiO₂, petroleum ether/ethyl acetate = 50/1 to 1/1) afforded tert-butyl 2-cyano-3-[[5-(3,5-dichlorophenyl)-5-(trifluoromethyl)-4H-isoxazol-3-yl]amino]benzoate (6.00g, 11.9mmol, 36%) as a white solid.
¹H-NMR (400MHz, CDCl₃, RT): δ 9.14 (s, 1H), 8.19 (d, J = 12.0Hz, 1H), 7.78-7.73 (m, 2H), 7.64-7.62 (m, 3H), 4.23-3.93 (m, 2H), 1.54 (s, 9H).

This product could then be transformed further into e.g. the compounds of formula I-151, I-160, I-173 and I-180, analogously as described in the above examples.

### Example 19: Methyl 5-bromo-2-cyano-benzoate:

Step 1: To a solution of methyl 5-bromo-2-iodo-benzoate (10.0g, 29.3mmol) in N-methyl-2-pyr-rolidon (100mL) at ambient temperature was added CuCN (3.15g, 35.2mmol, 7.69mL) and the resulting mixture was heated to 60°C and stirred at that temperature for 2.5 hours. Then TLC analysis (petroleum ether/EtOAc = 5:1, product Rf = 0.5) indicated full conversion of the starting material to one new product. The reaction mixture was filtered, and the filter cake washed with EtOAc (3×15mL). The combined filtrates were diluted with water (50 mL), the aqueous phase was extracted with EtOAc (3×30mL), and the combined organic extracts were dried over Na₂SO₄, filtered and concentrated under reduced pressure to give methyl 5-bromo-2-cyano-benzoate (7.00g, 99%) as a light yellow solid.
¹H-NMR: (400MHz, CDCl₃, RT) δ 8.29 (d, J = 2.0Hz, 1H), 7.82-7.79 (m, 1H), 7.68-7.66 (m, 1H), 4.02 (s, 3H).

This product could then be transformed further into e.g. the compound of formula I-143 analogously as described in the above examples.

### Example 20: Synthesis of 3-chloro-6-[[5-(3,5-dichlorophenyl)-5-(trifluoromethyl)-4H-isoxazol-3-yl]amino]pyridine-2-carboxylic acid.

Step 1: To a suspension of MgSO₄ (25.1g, 208mmol) and H₂SO₄ (5.11g, 52.1mmol, 2.78mL) in CH₂Cl₂ (70mL) at ambient temperature was added, after 15 min, 3,6-dichloropyridine-2-carboxylic acid (10.0g, 52.1mmol) and t-BuOH (19.3g, 260mmol, 24.9mL), and the resulting reaction mixture was stirred at that temperature for 24 hours. Then TLC analysis (petroleum ether/EtOAc = 3:1; 3,6-dichloropyridine-2-carboxylic acid: Rf = 0.10; product: Rf = 0.50) indicated that the reaction was complete. The reaction mixture was quenched by the addition of Na-HCO₃ solution (sat. aqueous, 50mL), the aqueous phase was extracted with ethyl acetate (2×50mL), and the combined organic extracts were dried over Na₂SO₄, filtered and concentrated under reduced pressure. Purification by column chromatography (SiO₂, petroleum ether/EtOAc = 30:1 to 3:1) afforded tert-butyl 3,6-dichloropyridine-2-carboxylate (9.60g, 74%) as a yellow solid.
¹H-NMR (400MHz, CDCl₃, RT) δ 7.70 (d, J = 8.4Hz, 1H) 7.35 (d, J = 8.8Hz, 1H) 6.84 (d, J = 8.8Hz, 1H) 1.63 (s, 9H).

Step 2: To a solution of 5-(3,5-dichlorophenyl)-5-(trifluoromethyl)-4H-isoxazol-3-amine (10.0 g, 33.4 mmol) and tert-butyl 3,6-dichloropyridine-2-carboxylate (8.30 g, 33.4 mmol) in 1,4-dioxane (200mL) under an atmosphere of N₂ was added Cs₂CO₃ (16.4g, 50.2mmol), Xantphos (2.90g, 5.02 mmol) and Pd₂(dba)₃ (3.06g, 3.34mmol), before the reaction mixture was heated to 80°C and stirred at that temperature for 24 hours. After that time, the mixture was filtered and concentrated. Purification by column chromatography (SiO₂, petroleum ether/EtOAc=100:1 to 1:1) afforded tert-butyl 3-chloro-6-[[5-(3,5-dichlorophenyl)-5-(trifluoromethyl)-4H-isoxazol-3-yl]amino]pyridine-2-carboxylate (9.60g, 74 %) as a yellow solid.
¹H-NMR (400 MHz, CDCl₃, RT): δ 7.55 (d, J = 2.0 Hz, 4H), 7.28 (d, J = 2.0 Hz, 1H), 3.78-3.71 (m, 2H), 1.69 (s, 9H).

Step 3: tert-Butyl 3-chloro-6-[[5-(3,5-dichlorophenyl)-5-(trifluoromethyl)-4H-isoxazol-3-yl]amino]pyridine-2-carboxylate (7.00g, 13.7mmol) was dissolved in TFA (35.0mL), heated to 40°C, and stirred at that temperature for 12 hours. After that time, the mixture was poured into water (10mL), the obtained solid washed with water (2×15mL), and dried. Purification by reversed-phase HPLC (neutral condition) afforded 3-chloro-6-[[5-(3,5-dichlorophenyl)-5-(trifluoromethyl)-4H-isoxazol-3-yl]amino]pyridine-2-carboxylic acid (2.25 g, 45%) as a pink solid.
¹H-NMR (400MHz, CDCl₃, RT) δ 7.83-7.75 (m, 2H), 7.64-7.59 (m, 3H), 7.30 (s, 1H), 4.01-3.59-3.76 (m, 2H).

This product could then be transformed further into e.g. the compounds of formula I-153, I-175, and I-182, analogously as described in the above examples.

### Example 21: Synthesis of methyl 5-bromo-2-(difluoromethoxy)benzoate.

Step 1: To a solution of methyl 5-bromo-2-hydroxy-benzoate (100g, 432mmol) and Na₂CO₃ (55.0g, 518mmol) in DMF (1.00L) at ambient temperature was added sodium chlorodifluoroace-tate (79.1g, 519mmol), before the mixture was heated to 90°C and stirred at that temperature for 14 hours. Then the reaction mixture was allowed to cool to ambient temperature, the pH of the mixture was adjusted to 6-7 with 1N aqueous HCl, and the aqueous phase was extracted with EtOAc (2×500mL). The combined organic extracts were dried over Na₂SO₄, filtered and concentrated under reduced pressure. Purification by column chromatography (SiO₂, petroleum ether/ethyl acetate = 100:1 to 1:1) afforded methyl 5-bromo-2-(difluoromethoxy)benzoate (15.0g, 6%) as a yellow oil.
¹H-NMR (400MHz, CDCl₃). δ 8.00 (d, J = 4.0Hz, 1H), 7.64-7.61 (m, H), 7.15 (d, J = 4.0Hz, 1H), 6.55-6.36 (m, 1H), 3.92 (s, 1H).

This product could then be transformed further into e.g. the compounds of formula I-148, 1-157, 1-165, 1-171, and I-178, analogously as described in the above examples.

### Example 22: Synthesis of methyl 5-bromo-2-(trifluoromethyl)benzoate.

Step 1: A mixture of methyl 5-bromo-2-iodo-benzoate (20.0g, 58.6mmol), methyl 2,2-difluoro-2-fluorosulfonyl-acetate (16.9g, 87.9mmol), and CuBr (1.01g, 7.04mmol) in N-methyl-2-pyrrolidone (120mL) at ambient temperature was degassed and purged with N₂ for 3 times, before the mixture was heated to 100°C and stirred at that temperature for 18 hours. After that time, the reaction mixture was allowed to cool to ambient temperature, filtered and the filter cake was washed with EtOAc (3×100mL). The combined filtrates were diluted with water (100mL), the phases were separated, and the aqueous phase extracted with ethyl acetate (3×100mL). The combined organic extracts were dried over Na₂SO₄, filtered and concentrated under reduced pressure. Purification by column chromatography (SiO₂, petroleum ether/EtOAc = 50:1 to 3:1) afforded methyl 5-bromo-2-(trifluoromethyl)benzoate (15.0g, 72%) as a light-yellow oil.
¹H-NMR (400MHz, CDCl₃, RT): δ 7.93 (s, 1H), 7.74-7.72 (m, 1H), 7.60 (d, J = 8.0Hz, 1H), 3.94 (s, 3H).

This product could then be transformed further into e.g. the compounds of formula I-149, I-158, and I-166, analogously as described in the above examples.

**Table I.1 - compounds of formula I (U = O, Y = direct bond)**

| No. | WP | R¹ | X | G | R⁵ | R⁶ | phys. data [HPLC Rt [min], M+H [m/z]] | HPLC method |
|---|---|---|---|---|---|---|---|---|
| I-1 | W-9 | CF₃ | NH | G1, R⁴¹: Cl, R⁴²: H | c-C₃H₅ | H | 1.308, 494.0 | A |
| I-2 | W-9 | CF₃ | NH | G1, R⁴¹: Cl, R⁴²: H | 2,4-F₂-C₆H₃ | H | 1.430, 566.0 | A |
| I-3 | W-9 | CF₃ | NH | G1, R⁴¹: Cl, R⁴²: H | (1-CN)c-C₃H₄ | H | 1.270, 517.0 | A |
| I-4 | W-9 | CF₃ | NH | G1, R⁴¹: Cl, R⁴²: H | 2,4-F₂-,3-NH₂-C₆H₂ | H | 1.328, 580.6 | A |
| I-5 | W-9 | CF₃ | NH | G1, R⁴¹: Cl, R⁴²: H | 2,4-F₂-,3-NHCOCHF₂-C₆H₂ | H | 1.324, 656.9 | A |
| I-6 | W-9 | CF₃ | NH | G1, R⁴¹: Cl, R⁴²: H | 2,4-F₂-,3-NHCOCH₃-C₆H₂ | H | 1.275, 623.0 | A |
| I-7 | W-9 | CF₃ | NH | G1, R⁴¹: Cl, R⁴²: H | 2,4-F₂-,3-NHCOCClF₂-C₆H₂ | H | 1.386, 692.9 | A |
| I-8 | W-9 | CF₃ | NH | G1, R⁴¹: Cl, R⁴²: H | 2,4-F₂-,3-NHCOCF₃-C₆H₂ | H | 1.375, 674.9 | A |
| I-9 | W-9 | CF₃ | NH | G1, R⁴¹: Cl, R⁴²: H | 2,4-F₂-,3-NHCOCH₂CF₃-C₆H₂ | H | 1.341, 690.9 | A |
| I-10 | W-9 | CF₃ | NH | G1, R⁴¹: Cl, R⁴²: H | 2,4-F₂-,3-NHCOCH₂-c-C₃H₅-C₆H₂ | H | 1.347, 663.0 | A |
| I-11 | W-9 | CF₃ | NH | G1, R⁴¹: F, R⁴²: F | (1-CN)c-C₃H₄ | H | 1.270, 518.8 | A |
| I-12 | W-9 | CF₃ | NH | G1, R⁴¹: H, R⁴²: F | (1-CN)c-C₃H₄ | H | 1.280, 500.9 | A |
| I-13 | W-9 | CF₃ | NH | G1, R⁴¹: H, R⁴²: F | 2,4-F₂-C₆H₃ | H | 1.416, 548.0 | A |
| I-14 | W-11 | CF₃ | NH | G1, R⁴¹: Cl, R⁴²: H | 2,4-F₂-C₆H₃ | H | 1.397, 583.9 | A |
| I-15 | W-8 | CF₃ | NH | G1, R⁴¹: Cl, R⁴²: H | 2,4-F₂-C₆H₃ | H | 1.322, 548.0 | A |
| I-16 | W-26 | CF₃ | NH | G1, R⁴¹: Cl, R⁴²: H | 2,4-F₂-C₆H₃ | H | 1.357, 582.0 | A |
| I-17 | W-9 | CF₂Cl | NH | G1, R⁴¹: Cl, R⁴²: H | 2,4-F₂-C₆H₃ | H | 1.417, 580.2 | A |
| I-18 | W-9 | CF₃ | NH | G2, R⁴¹: Cl, R⁴²: H | CH₂CF₃ | H | 1.301, 536.9 | A |
| I-19 | W-9 | CF₃ | NCH₃ | G1, R⁴¹: Cl, R⁴²: H | 2,4-F₂-C₆H₃ | H | 1.405, 578.0 | A |
| I-20 | W-18 | CF₃ | NH | G1, R⁴¹: Cl, R⁴²: H | 2,4-F₂-C₆H₃ | H | 1.330, 564.0 | A |
| I-21 | W-11 | CF₃ | NH | G1, R⁴¹: Cl, R⁴²: H | (1-CN)c-C₃H₄ | H | 1.276, 534.7 | A |
| I-22 | W-26 | CF₃ | NH | G1, R⁴¹: Cl, R⁴²: H | (1-CN)c-C₃H₄ | H | 1.216, 535.0 | A |
| I-23 | W-9 | CF₃ | NCH₃ | G1, R⁴¹: Cl, R⁴²: H | (1-CN)c-C₃H₄ | H | 1.279, 532.6 | A |
| I-24 | W-18 | CF₃ | NH | G1, R⁴¹: Cl, R⁴²: H | (1-CN)c-C₃H₄ | H | 1.198, 517.0 | A |
| I-25 | W-9 | CF₃ | NCH₂-c-C₃H₅ | G1, R⁴¹: Cl, R⁴²: H | (1-CN)c-C₃H₄ | H | 1.363, 571.0 | A |
| I-26 | W-9 | CF₃ | NCH₂-C≡N | G1, R⁴¹: Cl, R⁴²: H | (1-CN)c-C₃H₄ | H | 1.270, 558.2 | A |
| I-27 | W-9 | CF₃ | NCH₂OCH₃ | G1, R⁴¹: Cl, R⁴²: H | (1-CN)c-C₃H₄ | H | 1.274, 528.9 | A |
| I-28 | W-9 | CF₃ | NCOOC₂H₅ | G1, R⁴¹: Cl, R⁴²: H | (1-CN)c-C₃H₄ | H | 1.354, 589.0 | A |
| I-29 | W-9 | CF₃ | NCH₂CH(OCH₂ CH₃)₂ | G1, R⁴¹: Cl, R⁴²: H | (1-CN)c-C₃H₄ | H | 1.375, 587.3 | A |
| I-30 | W-9 | CF₃ | 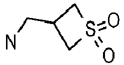 | G1, R⁴¹: Cl, R⁴²: H | (1-CN)c-C₃H₄ | H | | |
| I-31 | W-9 | CF₃ | NH | G1, R⁴¹: Cl, R⁴²: H | (1-CN)c-C₃H₄ | CH₂-c-C₃H₅ | 1.396, 572.9 | A |
| I-32 | W-9 | CF₃ | NCH₂-c-C₃H₅ | G1, R⁴¹: Cl, R⁴²: H | (1-CN)c-C₃H₄ | CH₂-c-C₃H₅ | 1.474, 627.0 | A |
| I-33 | W-9 | CF₃ | NH | G1, R⁴¹: Cl, R⁴²: H | 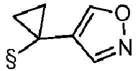 | H | | |
| I-34 | W-9 | CF₃ | NH | G1, R⁴¹: Cl, R⁴²: H | 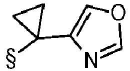 | H | | |
| I-35 | W-11 | CF₃ | NH | G1, R⁴¹: Cl, R⁴²: H | 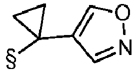 | H | | |
| I-36 | W-11 | CF₃ | NH | G1, R⁴¹: Cl, R⁴²: H | 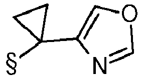 | H | | |
| I-37 | W-9 | CF₃ | NH | G1, R⁴¹: Cl, R⁴²: H | 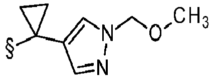 | H | | |
| I-38 | W-9 | CF₃ | NH | G1, R⁴¹: Cl, R⁴²: H | 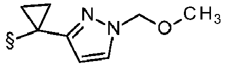 | H | | |
| I-39 | W-9 | CF₃ | NH | G1, R⁴¹: Cl, R⁴²: H | 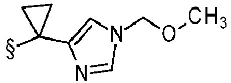 | H | | |
| I-40 | W-9 | CF₃ | NH | G1, R⁴¹: Cl, R⁴²: H | 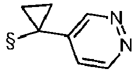 | H | | |
| I-41 | W-9 | CF₃ | NCH₃ | G1, R⁴¹: Br, R⁴²: H | (1-CN)c-C₃H₄ | H | | |
| I-42 | W-9 | CF₃ | O | G1, R⁴¹: Cl, R⁴²: H | (1-CN)c-C₃H₄ | H | | |
| I-43 | W-9 | CF₃ | S | G1, R⁴¹: Cl, R⁴²: H | (1-CN)c-C₃H₄ | H | | |
| I-44 | W-9 | CF₃ | SO₂ | G1, R⁴¹: Cl, R⁴²: H | (1-CN)c-C₃H₄ | H | | |
| I-45 | W-9 | CF₃ | N-OH | G1, R⁴¹: Cl, R⁴²: H | (1-CN)c-C₃H₄ | H | | |
| I-46 | W-9 | CF₃ | N-OC₂H₅ | G1, R⁴¹: Cl, R⁴²: H | (1-CN)c-C₃H₄ | H | | |
| I-47 | W-9 | CF₃ | NC₂H₅ | 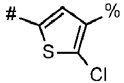 | (1-CN)c-C₃H₄ | H | | |
| I-48 | W-9 | CF₃ | NC₂H₅ | 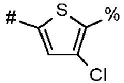 | (1-CN)c-C₃H₄ | H | | |
| I-49 | W-9 | CF₃ | NC₂H₅ | 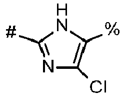 | (1-CN)c-C₃H₄ | H | | |
| I-50 | W-9 | CF₃ | NC₂H₅ | 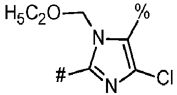 | (1-CN)c-C₃H₄ | H | | |
| I-51 | W-9 | CF₃ | NC₂H₅ | 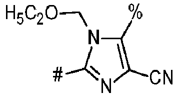 | (1-CN)c-C₃H₄ | H | | |
| I-52 | W-9 | CF₃ | NC₂H₅ | 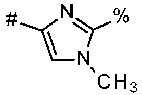 | (1-CN)c-C₃H₄ | H | | |
| I-53 | W-20 | CF₃ | NH | G1, R⁴¹: Cl, R⁴²: H | (1-CN)c-C₃H₄ | H | 1.278, 551.3 | A |
| I-54 | W-21 | CF₃ | NH | G1, R⁴¹: Cl, R⁴²: H | (1-CN)c-C₃H₄ | H | 1.288, 597.2 | A |
| I-55 | W-32 | CF₃ | NH | G1, R⁴¹: Cl, R⁴²: H | (1-CN)c-C₃H₄ | H | | |
| I-56 | W-33 | CF₃ | NH | G1, R⁴¹: Cl, R⁴²: H | (1-CN)c-C₃H₄ | H | | |
| I-57 | 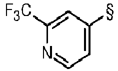 | CF₃ | NH | G1, R⁴¹: Cl, R⁴²: H | (1-CN)c-C₃H₄ | H | | |
| I-58 | 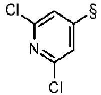 | CF₃ | NH | G1, R⁴¹: Cl, R⁴²: H | (1-CN)c-C₃H₄ | H | | |
| I-59 | 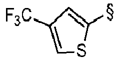 | CF₃ | NH | G1, R⁴¹: Cl, R⁴²: H | (1-CN)c-C₃H₄ | H | | |
| I-60 | 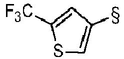 | CF₃ | NH | G1, R⁴¹: Cl, R⁴²: H | (1-CN)c-C₃H₄ | H | | |
| I-61 | 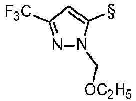 | CF₃ | NH | G1, R⁴¹: Cl, R⁴²: H | (1-CN)c-C₃H₄ | H | | |
| I-62 | 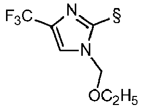 | CF₃ | NH | G1, R⁴¹: Cl, R⁴²: H | (1-CN)c-C₃H₄ | H | | |
| I-63 | W-9 | CF₃ | NH | G1, R⁴¹: Cl, R⁴²: H | 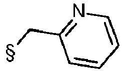 | H | 1.073, 543.0 | A |
| I-64 | W-9 | CF₃ | NH | G1, R⁴¹: Cl, R⁴²: H | 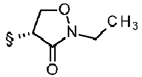 | H | 1.265, 564.8 | A |
| I-65 | W-9 | CF₃ | NH | G1, R⁴¹: Cl, R⁴²: H | 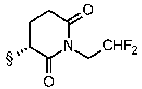 | H | 1.290, 628.0 | A |
| I-66 | W-9 | CF₃ | NH | G1, R⁴¹: Cl, R⁴²: H | CH₂CF₃ | H | 1.309, 533.7 | A |
| I-67 | W-9 | CF₃ | NH | G1, R⁴¹: Cl, R⁴²: H | CH₂C(O)NHCH₂CF₃ | H | 1.274, 593.0 | A |
| I-68 | W-9 | CF₃ | NH | G1, R⁴¹: Cl, R⁴²: H | CH₂C(O)NH(CH₂)₂CF₃ | H | 1.268, 604.7 | A |
| I-69 | W-9 | CF₃ | NH | G1, R⁴¹: H, R⁴²: Cl | 2,4-F₂-C₆H₃ | H | 1.407, 563.7 | A |
| I-70 | W-9 | CF₃ | NH | 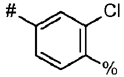 | 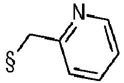 | H | 1.084, 543.0 | A |
| I-71 | W-9 | CF₃ | NH | 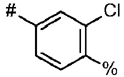 | 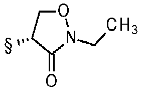 | H | 1.257, 567.0 | A |
| I-72 | W-9 | CF₃ | NH | G1, R⁴¹: Cl, R⁴²: H | 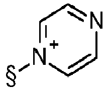 | | 1.179, 531.6 | A |
| I-73 | W-11 | CF₃ | NH | G1, R⁴¹: Cl, R⁴²: H | CH₂C≡N | H | 1.248, 510.5 | A |
| I-74 | W-9 | CF₃ | NH | G1, R⁴¹: Cl, R⁴²: H | (1-CN)c-C₄H₆ | H | 1.308, 530.8 | A |
| I-75 | W-11 | CF₃ | NH | G1, R⁴¹: Cl, R⁴²: H | C(CH₃)₂C≡N | H | 1.295, 538.9 | A |
| I-76 | W-11 | CF₃ | NH | G1, R⁴¹: Cl, R⁴²: H | (1-C(O)NH(CH₂)₂CF₃)C-C₃H₄ | H | 1.286, 651.0 | A |
| I-77 | W-9 | CF₃ | NH | G1, R⁴¹: Cl, R⁴²: H | 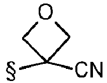 | H | 1.250, 535.0 | A |
| I-78 | W-9 | CF₃ | NH | G3, R⁴¹: Cl | CH₂CF₃ | H | 1.282, 534.9 | A |
| I-79 | W-11 | CF₃ | NH | G1, R⁴¹: Cl, R⁴²: H | 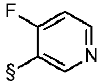 | H | 1.205, 566.9 | A |
| I-80 | W-9 | CF₃ | NH | G1, R⁴¹: Cl, R⁴²: H | 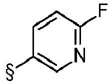 | H | 1.330, 548.6 | A |
| I-81 | W-11 | CF₃ | NH | G1, R⁴¹: Cl, R⁴²: H | 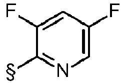 | H | 1.313, 584.9 | A |
| I-82 | W-11 | CF₃ | NH | G1, R⁴¹: Cl, R⁴²: H | 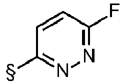 | H | 1.319, 568.0 | A |
| I-83 | W-11 | CF₃ | NH | G1, R⁴¹: Cl, R⁴²: H | 2-CN,4-F-C₆H₃ | H | 1.368, 591.1 | A |
| I-84 | W-8 | CF₃ | NH | G1, R⁴¹: Cl, R⁴²: H | (1-CF₂H)-c-C₃H₄ | H | 1.267, 525.7 | A |
| I-85 | W-8 | CF₃ | NH | G1, R⁴¹: Cl, R⁴²: H | (1-CF₃)-c-C₃H₄ | H | 1.293, 543.9 | A |
| I-86 | W-8 | CF₃ | NH | G1, R⁴¹: Cl, R⁴²: H | (1-CF₃)-c-C₄H₆ | H | 1.335, 557.9 | A |
| I-87 | W-8 | CF₃ | NH | G1, R⁴¹: Cl, R⁴²: H | (3,3-F₂)-c-C₄H₅ | H | 1.277, 525.7 | A |
| I-88 | W-8 | CF₃ | NH | G1, R⁴¹: Cl, R⁴²: H | 2-F,4-CF₃-C₆H₃ | H | 1.446, 598.1 | A |
| I-89 | W-8 | CF₃ | NH | G1, R⁴¹: Cl, R⁴²: H | 2-CF₃,4-F-C₆H₃ | H | 1.370, 598.0 | A |
| I-90 | W-8 | CF₃ | NH | G1, R⁴¹: Cl, R⁴²: H | 2-Br,4-CF₃-C₆H₃ | H | 1.497, 659.8 | A |
| I-91 | W-8 | CF₃ | NH | G1, R⁴¹: Cl, R⁴²: H | 2-CH₃,4-CF(CF₃)₂-C₆H₃ | H | 1.501, 694.0 | A |
| I-92 | W-8 | CF₃ | NH | G1, R⁴¹: Cl, R⁴²: H | 2-F,4-CN-C₆H₃ | H | 1.331, 554.9 | A |
| I-93 | W-8 | CF₃ | NH | G1, R⁴¹: Cl, R⁴²: H | 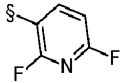 | H | 1.309, 548.9 | A |
| I-94 | W-11 | CF₃ | NH | G1, R⁴¹: Cl, R⁴²: H | C(CH₃)₂CH₂SO₂CH₃ | H | 1.274, 605.5 | A |
| I-95 | W-11 | CF₃ | NH | G1, R⁴¹: Cl, R⁴²: H | 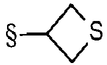 | H | 1.326, 542.1 | A |
| I-96 | W-11 | CF₃ | NH | G1, R⁴¹: Cl, R⁴²: H | 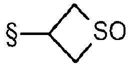 | H | 1.167, 559.9 | A |
| I-97 | W-11 | CF₃ | NH | G1, R⁴¹: Cl, R⁴²: H | 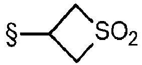 | H | 1.219, 576.1 | A |
| I-98 | W-11 | CF₃ | NH | G1, R⁴¹: Cl, R⁴²: H | 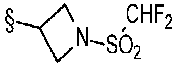 | H | 1.304, 641.1 | A |
| I-99 | W-11 | CF₃ | NH | G1, R⁴¹: Cl, R⁴²: H | 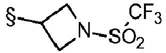 | H | 1.365, 558.5 | A |
| I-100 | W-11 | CF₃ | NH | G1, R⁴¹: Cl, R⁴²: H | 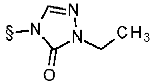 | H | 1.232, 582.9 | A |
| I-101 | W-11 | CF₃ | NH | G1, R⁴¹: Cl, R⁴²: H | 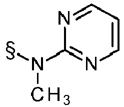 | H | 1.266, 576.9 | A |
| I-102 | W-9 | CF₃ | NCH₃ | G1, R⁴¹: Cl, R⁴²: H | 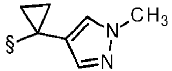 | H | 1.260, 588.0 | A |
| I-103 | W-9 | CF₃ | NCH₃ | G1, R⁴¹: Cl, R⁴²: H | 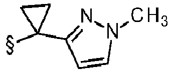 | H | 1.283, 587.9 | A |
| I-104 | W-9 | CF₃ | NCH₃ | G1, R⁴¹: Cl, R⁴²: H | 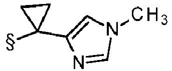 | H | | |
| I-105 | W-9 | CF₃ | NCH₃ | G1, R⁴¹: Cl, R⁴²: H | 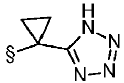 | H | 1.204, 575.9 | A |
| I-106 | W-9 | CF₃ | NCH₃ | G1, R⁴¹: Cl, R⁴²: H | 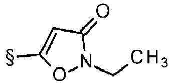 | H | | |
| I-107 | W-9 | CF₃ | NCH₃ | G1, R⁴¹: Cl, R⁴²: H | 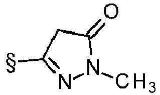 | H | 1.204, 563.9 | A |
| I-108 | W-9 | CF₃ | NCH₃ | G1, R⁴¹: Cl, R⁴²: H | 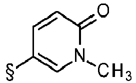 | H | 1.198, 574.9 | A |
| I-109 | W-9 | CF₃ | NCH₃ | G1, R⁴¹: Cl, R⁴²: H | 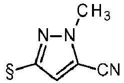 | H | 1.327, 573.3 | A |
| I-110 | W-9 | CF₃ | NCH₃ | G1, R⁴¹: Cl, R⁴²: H | 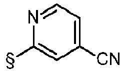 | H | 1.386, 567.9 | A |
| I-111 | W-9 | CF₃ | NCH₃ | G1, R⁴¹: Cl, R⁴²: H | 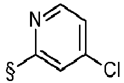 | H | 1.471, 578.9 | A |
| I-112 | W-9 | CF₃ | NCH₃ | G1, R⁴¹: Cl, R⁴²: H | 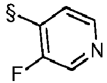 | H | | |
| I-113 | W-9 | CF₃ | NCH₃ | G1, R⁴¹: Cl, R⁴²: H | 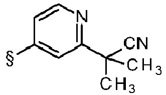 | H | 1.382, 610.0 | A |
| I-114 | W-11 | CF₃ | NH | G1, R⁴¹: Cl, R⁴²: H | 4-F-C₆H₄ | CH₃ | 1.403, 579.9 | A |
| I-115 | W-11 | CF₃ | NH | G1, R⁴¹: Cl, R⁴²: H | 4-F-C₆H₄ | C₂H₅ | 1.428, 594.3 | A |
| I-116 | W-11 | CF₃ | NH | G1, R⁴¹: Cl, R⁴²: H | C₆H₅ | C₂H₅ | 1.427, 576.2 | A |
| I-117 | W-11 | CF₃ | NH | G1, R⁴¹: Cl, R⁴²: H | (1-CN)c-C₃H₄ | CH₃ | 1.319, 549.3 | A |
| I-118 | W-11 | CF₃ | NH | G1, R⁴¹: Cl, R⁴²: H | 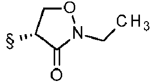 | H | 1.260, 583.2 | A |
| I-119 | W-11 | CF₃ | NH | G1, R⁴¹: Cl, R⁴²: H | 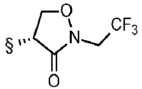 | H | 1.303, 637.3 | A |
| I-120 | W-11 | CF₃ | NCH₃ | G1, R⁴¹: Cl, R⁴²: H | (1-CN)c-C₃H₄ | H | 1.274, 549.2 | A |
| I-121 | W-11 | CF₃ | NCH₃ | G1, R⁴¹: Cl, R⁴²: H | (1-CN)c-C₃H₄ | CH₃ | 1.331, 565.3 | A |
| I-122 | W-11 | CF₃ | NCH₃ | G1, R⁴¹: Cl, R⁴²: H | C₆H₅ | C₂H₅ | 1.462, 590.3 | A |
| I-123 | W-11 | CF₃ | NCH₃ | G1, R⁴¹: Cl, R⁴²: H | 4-F-C₆H₄ | CH₃ | 1.418, 592.3 | A |
| I-124 | W-11 | CF₃ | NCH₃ | G1, R⁴¹: Cl, R⁴²: H | 2,4-F₂-C₆H₃ | H | 1.409, 598.3 | A |
| I-125 | W-11 | CF₃ | NCH₃ | G1, R⁴¹: Cl, R⁴²: H | 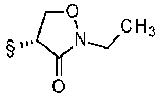 | H | 1.268, 597.3 | A |
| I-126 | W-9 | CF₃ | NCH₃ | G1, R⁴¹: Cl, R⁴²: H | 3-CN,4-F-C₆H₃ | H | 1.401, 584.9 | A |
| I-127 | W-8 | CF₃ | NH | G1, R⁴¹: Cl, R⁴²: H | CH(CN)-C₆H₅ | H | 1.316, 551.1 | A |
| I-128 | W-8 | CF₃ | NH | G1, R⁴¹: Cl, R⁴²: H | 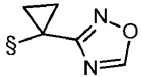 | H | 1.235, 546.0 | A |
| I-129 | W-9 | CF₃ | NCH₃ | G1, R⁴¹: Cl, R⁴²: H | 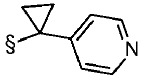 | H | | |
| I-130 | W-9 | CF₃ | NCH₃ | G1, R⁴¹: Cl, R⁴²: H | 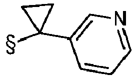 | H | 1.074, 585.3 | A |
| I-131 | W-8 | CF₃ | NH | G1, R⁴¹: Cl, R⁴²: H | 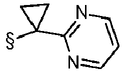 | H | 1.234, 553.9 | A |
| I-132 | W-9 | CF₃ | NH | G1, R⁴¹: H, R⁴²:CN | CH₂CF₃ | H | 1.488, 524.9 | A |
| I-133 | W-8 | CF₃ | NH | G1, R⁴¹: Cl, R⁴²: H | 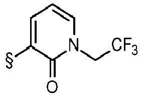 | H | 1.335,611.3 | A |
| I-134 | W-9 <R> | CF₃ | NH | G1, R⁴¹: Cl, R⁴²: H | 2,4-F₂-C₆H₃ | H | | |
| I-135 | W-9 <S> | CF₃ | NH | G1, R⁴¹: Cl, R⁴²: H | 2,4-F₂-C₆H₃ | H | | |
| I-136 | W-9 <R> | CF₃ | NH | G1, R⁴¹: Cl, R⁴²: H | 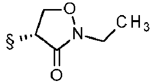 | H | | |
| I-137 | W-9 <S> | CF₃ | NH | G1, R⁴¹: Cl, R⁴²: H | 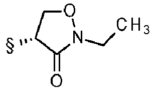 | H | | |
| I-138 | W-9 <R> | CF₃ | NCH₃ | G1, R⁴¹: Cl, R⁴²: H | (1-CN)c-C₃H₄ | H | | |
| I-139 | W-9 <S> | CF₃ | NCH₃ | G1, R⁴¹: Cl, R⁴²: H | (1-CN)c-C₃H₄ | H | | |
| I-140 | W-9 <R> | CF₃ | NCH₃ | G1, R⁴¹: Cl, R⁴²: H | 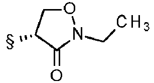 | H | | |
| I-141 | W-9 <S> | CF₃ | NCH₃ | G1, R⁴¹: Cl, R⁴²: H | 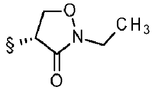 | H | | |
| I-142 | W-9 | CF₃ | NC(O)OC₂H₅ | G1, R⁴¹: Cl, R⁴²: H | (1-CN)c-C₃H₄ | C(O)OC₂H₅ | 1.466, 662.9 | A |
| I-143 | W-11 | CF₃ | NH | G1, R⁴¹: CN, R⁴²: H | 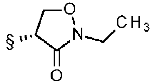 | H | | |
| I-144 | W-11 | CF₃ | NH | G1, R⁴¹: CH₃, R⁴²: H | 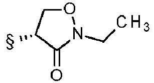 | H | | |
| I-145 | W-11 | CF₃ | NC₂H₅ | G1, R⁴¹: CH₃, R⁴²: H | 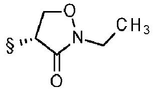 | H | | |
| I-146 | W-9 | CF₃ | NH | G1, R⁴¹: F, R⁴²: H | (1-CN)c-C₃H₄ | H | 1.290, 500.9 | A |
| I-147 | W-9 | CF₃ | NH | G1, R⁴¹: OCH₃, R⁴²: H | (1-CN)c-C₃H₄ | H | 1.298, 513.0 | A |
| I-148 | W-9 | CF₃ | NH | G1, R⁴¹: OCHF₂, R⁴²: H | (1-CN)c-C₃H₄ | H | 1.312, 549.0 | A |
| I-149 | W-9 | CF₃ | NH | G1, R⁴¹: CF₃, R⁴²: H | (1-CN)c-C₃H₄ | H | 1.326, 551.0 | A |
| I-150 | W-9 | CF₃ | NH | G1, D⁴¹: OCF₃, R⁴²: H | (1-CN)c-C₃H₄ | H | 1.346, 566.9 | A |
| I-151 | W-9 | CF₃ | NH | G1, R⁴¹: H, R⁴²: CN | (1-CN)c-C₃H₄ | H | 1.454, 508.0 | A |
| I-152 | W-9 | CF₃ | NH | G2, R⁴¹: Cl, R⁴²: H | (1-CN)c-C₃H₄ | H | 1.245, 517.9 | A |
| I-153 | W-9 | CF₃ | NH | G3, R⁴¹: Cl | (1-CN)c-C₃H₄ | H | 1.218, 519.9 | A |
| I-154 | W-9 | CF₃ | NH | G1, R⁴¹: F, R⁴²: H | 2,4-F₂-C₆H₃ | H | 1.457, 548.0 | A |
| I-155 | W-9 | CF₃ | NH | G1, R⁴¹: CH₃, R⁴²: H | 2,4-F₂-C₆H₃ | H | 1.418, 544.0 | A |
| I-156 | W-9 | CF₃ | NH | G1, R⁴¹: OCH₃, R⁴²: H | 2,4-F₂-C₆H₃ | H | 1.495, 559.9 | A |
| I-157 | W-9 | CF₃ | NH | G1, R⁴¹: OCHF₂, R⁴²: H | 2,4-F₂-C₆H₃ | H | 1.470, 596.0 | A |
| I-158 | W-9 | CF₃ | NH | G1, R⁴¹: CF₃, R⁴²: H | 2,4-F₂-C₆H₃ | H | 1.441, 598.0 | A |
| I-159 | W-9 | CF₃ | NH | G1, R⁴¹: OCF₃, R⁴²: H | 2,4-F₂-C₆H₃ | H | 1.482, 614.0 | A |
| I-160 | W-9 | CF₃ | NH | R⁴²: CN | 2,4-F₂-C₆H₃ | H | 1.542, 555.0 | A |
| I-161 | W-9 | CF₃ | NH | G2, R⁴¹: Cl, R⁴²: H | 2,4-F₂-C₆H₃ | H | 1.381, 564.9 | A |
| I-162 | W-9 | CF₃ | NH | G1, R⁴¹: F, R⁴²: H | 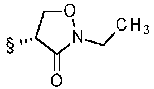 | H | 1.261, 549.3 | A |
| I-163 | W-9 | CF₃ | NH | G1, R⁴¹: CH₃, R⁴²: H | 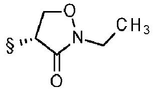 | H | 1.249, 545.3 | A |
| I-164 | W-9 | CF₃ | NH | G1, R⁴¹: OCH₃, R⁴²: H | 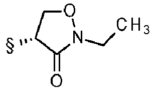 | H | 1.302, 561.0 | A |
| I-165 | W-9 | CF₃ | NH | G1, R⁴¹: OCHF₂, R⁴²: H | 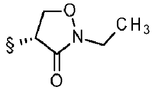 | H | 1.285, 597.3 | A |
| I-166 | W-9 | CF₃ | NH | G1, R⁴¹: CF₃, R⁴²: H | 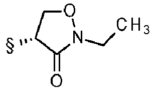 | H | 1.300, 599.3 | A |
| I-167 | W-9 | CF₃ | NH | G1, D⁴¹: OCF₃, R⁴²: H | 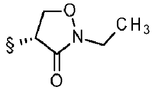 | H | 1.323, 615.3 | A |
| I-168 | W-9 | CF₃ | NH | G2, R⁴¹: Cl, R⁴²: H | 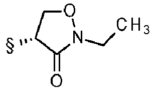 | H | 1.210, 566.2 | A |
| I-169 | W-9 | CF₃ | NH | G1, R⁴¹: CH₃, R⁴²: H | c-C₃H₅ | H | 1.288, 472.0 | A |
| I-170 | W-9 | CF₃ | NH | G1, R⁴¹: OCH₃, R⁴²: H | c-C₃H₅ | H | 1.326, 488.0 | A |
| I-171 | W-9 | CF₃ | NH | G1, R⁴¹: OCHF₂, R⁴²: H | c-C₃H₅ | H | 1.337, 524.0 | A |
| I-172 | W-9 | CF₃ | NH | G1, R⁴¹: OCF₃, R⁴²: H | c-C₃H₅ | H | 1.373, 542.0 | A |
| I-173 | W-9 | CF₃ | NH | R⁴²: CN | c-C₃H₅ | H | 1.486, 483.0 | A |
| I-174 | W-9 | CF₃ | NH | G2, R⁴¹: Cl, R⁴²: H | c-C₃H₅ | H | 1.242, 494.9 | A |
| I-175 | W-9 | CF₃ | NH | G3, R⁴¹: Cl | c-C₃H₅ | H | 1.226, 492.9 | A |
| I-176 | W-9 | CF₃ | NH | G1, R⁴¹: CH₃, R⁴²: H | CH₂CF₃ | H | 1.347, 514.0 | A |
| I-177 | W-9 | CF₃ | NH | G1, R⁴¹: OCH₃, R⁴²: H | CH₂CF₃ | H | 1.367, 530.0 | A |
| I-178 | W-9 | CF₃ | NH | G1, R⁴¹: OCHF₂, R⁴²: H | CH₂CF₃ | H | 1.372, 565.9 | A |
| I-179 | W-9 | CF₃ | NH | G1, R⁴¹: OCF₃, R⁴²: H | CH₂CF₃ | H | 1.403, 584.0 | A |

| | | | | | | | | |
|---|---|---|---|---|---|---|---|---|
| § = bond to the remainder of the molecule; # = bond to X; % = bond to Y <S> S-Enantiomer (Formula I.A) <R> R-Enantiomer (Formula I.B) | | | | | | | | |

**Table I.2 - compounds of formula I.2 (U = S, Y = direct bond)**

| No. | W | R¹ | X | G | R⁵ | R⁶ | phys. data [HPLC Rt [min], M+H [m/z]] | HPLC method |
|---|---|---|---|---|---|---|---|---|
| II-1 | W-9 | CF₃ | NH | G1, R⁴¹: Cl, R⁴²: H | (1-CN)c-C₃H₄ | H | | |

**Table I.3 - compounds of formula I.3 (U = O, Y = CR⁶R⁷)**

| No. | W | R¹ | X | G | Y | R⁵ | R⁶ | phys. data [HPLC Rt [min], M+H [m/z]] | HPLC method |
|---|---|---|---|---|---|---|---|---|---|
| III-1 | W-8 | CF₃ | NH | G1, R⁴¹: CH₃, R⁴²: H | CH₂ | (1-CN)c-C₃H₄ | H | 1.201, 515.3 | A |
| III-2 | W-8 | CF₃ | NH | G1, R⁴¹: F, R⁴²: H | (1,1)c-C₄H₆ | (1-CN)c-C₃H₄ | H | | |
| III-3 | W-8 | CF₃ | NH | G1, R⁴¹: CH₃, R⁴²: H | CF₂ | (1-CN)c-C₃H₄ | H | | |
| III-4 | W-8 | CF₃ | NH | G1, R⁴¹: CH₃, R⁴²: H | CF₂ | 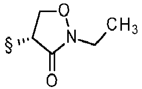 | H | | |

### Biological examples

If not otherwise specified, the test solutions were prepared as follows:
The active compound was dissolved at the desired concentration in a mixture of 1:1 (vol:vol) distilled water: acetone. The test solution was prepared at the day of use.
Test solutions were prepared in general at a concentration of 2500ppm (wt/vol).

### B.1. Boll weevil (Anthonomus grandis)

For evaluating control of boll weevil, the test unit consisted of 96-well-microtiter plates containing an insect diet and 5-10 *A. grandis* eggs.

The compounds were formulated using a solution containing 75% v/v water and 25% v/v DMSO. Different concentrations of formulated compounds were sprayed onto the insect diet at 5µl, using a custom-built micro atomizer, at two replications.

After application, microtiter plates were incubated at about 25 + 1°C and about 75 + 5 % relative humidity for 5 days. Egg and larval mortality were then visually assessed.

In this test, compounds I-1, I-2, I-3, I-4, I-5, I-6, I-7, I-8, I-9, I-10, I-1, I-12, I-13, I-14, I-15, I-16, I-18, I-19, I-20, I-21, I-22, I-23, I-24, I-25, I-26, I-27, I-28, I-29, I-31, I-32, I-63, I-64, I-65, I-66, I-67, I-68, I-69, I-70, I-72, I-74, I-75, I-76, I-77, I-80, I-81, I-83, I-84, I-85, I-87, I-88, I-89, I-90, 1-91, I-92, I-93, I-94, I-95, I-96, I-98, I-99, I-100, I-101, I-114, I-115, I-116, I-117, I-118, I-119, 1-120, 1-123, 1-124, 1-127, 1-133, 1-142, 1-146, I-147, I-148, I-149, I-150, I-152, I-154, I-156, I-157, 1-158, 1-159, 1-162, 1-163, 1-164, 1-165, 1-166, I-167, I-168, I-169, I-170, I-171, I-172, I-174, I-176, I-178, and I-179, resp., at 2500 ppm showed at least 75% mortality in comparison with untreated controls.

### B.8. Greenhouse Whitefly (Trialeurodes vaporarirorum)

For evaluating control of Greenhouse Whitefly, the test unit consisted of 96-well-microtiter plates containing a leaf disk of eggplant leaf disk with Whitefly eggs. The compounds or mixtures were formulated using a solution containing 75% water and 25% DMSO. Different concentrations of formulated were sprayed onto the insect diet at 2.5µl, using a custom-built micro atomizer, at two replications. After application, microtiter plates were incubated at 23 + 1°C, 65 + 5% RH for 6 days. Mortality of hatched crawlers was then visually assessed.

In this test, compounds 1-15, 1-19, I-20, I-27, I-32, I-76, 1-81, I-94, 1-114, and I-171, resp., at 2500ppm showed at least 75% mortality in comparison with untreated controls.

### B.9. Green Peach Aphid (Myzus persicae) (mixed life stages)

For evaluating control of Green Peach Aphid through systemic means the test unit consisted of 96-well-microtiter plates containing liquid artificial diet under an artificial membrane. The compounds were formulated using a solution containing 75% v/v water and 25% v/v DMSO. Different concentrations of formulated compounds were pipetted into the aphid diet, using a custom-built pipette, at two replications. After application, 5 - 8 adult aphids were placed on the artificial membrane inside the microtiter plate wells. The aphids were then allowed to suck on the treated aphid diet and incubated at about 23 + 1°C and about 50 + 5 % relative humidity for 3 days. Aphid mortality and fecundity was then visually assessed.

In this test, compounds I-1, I-2, I-3, I-4, I-5, I-6, I-7, I-8, I-9, 1-12, 1-13, I-14, I-15, I-16, I-18, I-19, I-20, 1-21, I-22, I-24, I-26, I-27, 1-31, I-32, I-63, I-64, I-65, I-66, I-68, I-74, I-76, I-77, I-80, I-81, I-83, I-85, I-87, I-88, I-89, I-92, I-93, I-94, I-95, I-96, I-98, I-99, I-100, I-118, I-119, I-120, I-124, I-127, I-146, I-150, I-152, I-154, I-157, I-158, I-162, I-163, I-164, I-165, I-166, I-167, I-168, I-169, I-171, I-172, I-178, and I-179, resp., at 2500 ppm showed at least 75 % mortality in comparison with untreated controls.

### B.19. Tobacco Budworm (Heliothis virescens)

For evaluating control of Tobacco Budworm, the test unit consisted of 96-well-microtiter plates containing an insect diet and 15-25 *H. virescens* eggs. The compounds were formulated using a solution containing 75% v/v water and 25% v/v DMSO. Different concentrations of formulated compounds were sprayed onto the insect diet at 10µl, using a custom-built micro atomizer, at two replications. After application, microtiter plates were incubated at about 28 + 1°C and about 80 + 5 % relative humidity for 5 days. Egg and larval mortality was then visually assessed.

In this test, compounds I-1, I-2, I-3, I-4, I-5, I-6, I-7, I-8, I-9, I-10, I-11, I-12, I-13, I-14, I-15, I-16, I-18, I-19, I-20, I-21, I-22, I-23, I-24, I-25, I-26, I-27, I-28, I-29, I-31, I-63, I-64, I-65, I-66, I-67, I-68, I-69, I-70, I-74, I-75, I-76, I-77, I-80, I-81, I-83, I-84, I-85, I-87, I-88, I-89, I-90, I-91, I-92, I-93, I-94, I-95, I-96, I-98, I-99, I-100, I-101, I-114, I-115, I-116, I-117, I-118, I-119, I-120, I-123, I-124, I-127, I-142, I-146, I-148, I-149, I-150, I-152, I-154, I-156, I-157, I-158, I-159, I-162, I-163, I-164, I-165, I-166, I-167, I-168, I-169, I-170, I-171, I-172, I-173, I-174, I-176, I-178, and I-179, resp., at 2500ppm showed at least 75 % mortality in comparison with untreated controls.

### B.24. Yellow Fever Mosquito (Aedes aegypti)

For evaluating control of Yellow Fever Mosquito the test unit consisted of 96-well-microtiter plates containing 200µl of tap water per well and 5-15 freshly hatched *A. aegypti* larvae. The active compounds were formulated using a solution containing 75% (v/v) water and 25% (v/v) DMSO. Different concentrations of formulated compounds or mixtures were sprayed onto the insect diet at 2.5µl, using a custom-built micro atomizer, at two replications.

After application, microtiter plates were incubated at 28 + 1°C, 80 + 5 % RH for 2 days. Larval mortality was then visually assessed.

In this test, compounds I-1, I-2, I-3, I-4, I-5, I-6, I-7, I-8, I-9, I-10, I-11, I-12, I-13, I-14, I-15, I-16, I-1, I-2, I-3, I-4, I-5, I-6, I-7, I-8, I-9, I-10, I-11, I-12, I-13, I-14, I-15, 1-16, 18, I-1, I-2, I-3, I-4, I-5, I-6, I-7, I-8, I-9, I-10, I-11, 1-12, 1-13, 1-14, 1-15, I-16, I-19, I-21, I-22, I-23, I-24, I-25, I-26, I-27, I-28, I-29, 1-31, I-63, I-64, I-66, I-68, I-69, I-70, I-71, I-72, I-74, I-76, I-80, I-81, I-84, I-85, I-87, I-88, I-92, I-93, I-94, I-95, I-96, I-98, I-100, I-101, I-114, I-115, I-116, I-117, I-118, I-119, I-120, I-123, I-124, I-132, I-142, I-146, I-147, I-148, I-149, I-150, I-154, I-157, I-158, I-159, I-162, I-163, I-166, I-168, I-169, I-170, I-171, I-172, I-173, I-174, I-176, I-178, I-179, resp., at 2500ppm showed at least 75% mortality in comparison with untreated controls.

## Claims

1. Compounds of formula I 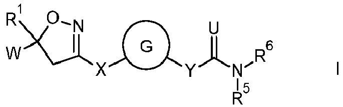 wherein
R¹ is C₁-C₂-haloalkyl;
W is phenyl, or 5- or 6-membered heteroaryl comprising as ring members 1, 2, or 3 heteroatoms selected from N, O, and S; wherein W is unsubstituted, partially or fully substituted with R²;
R² is halogen, OR²¹, NR²²R²³, CN, NO₂, Si(CH₃)₃, SbF₅, C₁-C₄-alkyl, C₁-C₄-haloalkyl, C₂-C₆-alkenyl, C₂-C₄-haloalkenyl, C₂-C₄-alkynyl, C2-C4-haloalkynyl, C₁-C₃-alkoxy, C₁-C₃-haloalkoxy, C₁-C₃-alkyl-S(O)ₘ, C₁-C₃-haloalkyl-S(O)ₘ, C₁-C₃-alkoxy-C₁-C₄-alkyl, C₁-C₃-haloalkoxy-C₁-C₄-alkyl, C₁-C₃-alkyl-S(O)ₘ-C₁-C₄-alkyl, C₁-C₃-haloalkyl-S(O)ₘ-C₁-C₄-alkyl, C₃-C₆-cyclo¬alkyl, C₃-C₆-halocyclo¬alkyl; which groups are optionally substituted with R²¹¹;
m is 0, 1, or 2;
R²¹ H, C₁-C₆-alkyl, C₂-C₆-alkenyl, C₂-C₆-alkynyl, C₃-C₃-cycloalkyl, Si(C₁-C₄-alkyl)₃, which groups are unsubstituted, partially or fully substituted with R²¹¹,
R²¹¹ halogen, C₁-C₄-alkoxy, C₁-C₄-haloalkoxy, C₃-C₄-alkenyloxy, C₃-C₄-haloalkenyloxy, C₃-C₄-alkynyloxy, C₃-C₄-haloalkynyloxy, C₁-C₄-alkyl-S(O)ₘ, C₁-C₄-haloalkyl-S(O)ₘ, C₃-C₄-alkenyl-S(O)ₘ, C₃-C₄-haloalkenyl-S(O)ₘ, C₃-C₄-alkynyl-S(O)ₘ, C₃-C₄-haloalkynyl-S(O)ₘ, and oxo; C3-C8-cycloalkyl, C₃-C₈-halocycloalkyl, C₃-C₈-cycloalkenyl, C₃-C₈-halocycloalkenyl;
R²², R²³ H, C₁-C₆-alkyl, C₁-C₆-haloalkyl, C₂-C₆-alkenyl, C₂-C₆-haloalkenyl, C₂-C₆-alkynyl, C₂-C₆-haloalkynyl, C₁-C₆-alkoxy, C₁-C₆-haloalkoxy, which are unsubstituted or partially or fully substituted with R²²¹; or C₁-C₆-alkyl-C(=O)OR¹³, C₁-C₆-alkyl-C(=U)N(R^{12a})R^{12b}, C₁-C₆-alkyl-C(=NR¹²)N(R^{12a})R^{12b},
S(O)ₘR¹³, S(O)ₘN(R^{12a})R^{12b}, C(=U)R¹¹, C(=O)OR¹³, C(=U)N(R^{12a})R^{12b}, C(=S)SR¹³, C(=NR¹²)R¹¹;
C3-C8-cycloalkyl, C₃-C₈-halocycloalkyl;
R²²¹ CN, NO₂, OH, SH, SCN, SF₅, Si(C₁-C₄-alkyl)₃, C₁-C₆-alkoxy, C₁-C₆-haloalkoxy, C₁-C₆-alkyl-S(O)ₘ, C₁-C₆-haloalkyl-S(O)ₘ, C(=O)N(R^{12a})R^{12b}; C₃-C₈-cycloalkyl which is unsubstituted, partially or fully halogenated and/or partially or fully substituted with C₁-C₄-alkyl, C₃-C₄-cycloalkyl, C₁-C₄-alkoxy, C₁-C₄-haloalkoxy, and oxo; or
two R²²¹ present on the same carbon atom of an alkyl, alkenyl, alkynyl or cycloalkyl group may together be =O, =CH(C₁-C₄-alkyl), =C(C₁-C₄-alkyl)₂, =N(C₁-C₆-alkyl), or =NO(C₁-C₆-alkyl); or
R²² and R²³ form together with the nitrogen atom they are bonded to a 3-, 4-, 5-, or 6-membered fully unsaturated heterocycle, which heterocycle may additionally contain one heteroatom selected from N, O, and S(O)ₘ as ring members, and which heterocycle is unsubstituted or partially or fully substituted with R¹⁴; or
R²² and R²³ together form a group =C(R¹¹)₂, =S(O)m(R¹³)₂, =S(O)ₘR¹³N(R^{12a})R^{12b};
X is NR³, O, or S(O)ₘ;
R³ is OR²¹, or a group as defined for R⁵;
G is phenyl, or 5- or 6-membered heteroaryl comprising as ring members 1, 2, or 3 heteroatoms selected from N, O and S; wherein G is unsubstituted, partially, or fully substituted with R⁴;
R⁴ is a group as defined for R²;
U is O or S;
R⁵ is H, C₁-C₆-alkyl, C₁-C₆-haloalkyl, C₂-C₆-alkenyl, C₂-C₆-haloalkenyl, C₂-C₆-alkynyl, C₂-C₆-haloalkynyl, C₁-C₆-alkoxy, C₁-C₆-haloalkoxy, C₁-C₆-alkylthio, C₁-C₆-haloalkylthio, which are unsubstituted or partially or fully substituted with R⁵¹; or C₁-C₆-alkyl-C(=O)OR¹³, C₁-C₆-alkyl-C(=U)N(R^{12a})R^{12b}, C₁-C₆-alkyl-C(=NR¹²)N(R^{12a})R^{12b},
N(R^{12a})R^{12b}, S(O)ₘR¹³, S(O)ₘN(R^{12a})R^{12b}, C(=U)R¹¹, C(=O)OR¹³, C(=U)N(R^{12a})R^{12b}, C(=S)SR¹³, C(=NR¹²)R¹¹;
C₃-C₈-cycloalkyl, C₃-C₈-halocycloalkyl, phenyl, a 3-, 4-, 5-, 6- or 7-membered saturated or partially unsaturated heterocycle comprising 1, 2, 3 or 4 heteroatoms selected from N, O, and S(O)ₘ as ring members, or a 5- or 6-membered hetaryl comprising 1, 2, 3 or 4 heteroatoms selected from N, O, and S(O)ₘ as ring members, which rings are unsubstituted or partially or fully substituted with R⁵²;
R⁵¹ halogen, CN, NO₂, OH, SH, SCN, SF₅, Si(C₁-C₄-alkyl)₃, N(R^{12a})R^{12b}, C₁-C₆-alkoxy, C₁-C₆-haloalkoxy, C₁-C₆-alkyl-S(O)ₘ, C₁-C₆-haloalkyl-S(O)ₘ, C(=O)N(R^{12a})R^{12b};
C₃-C₈-cycloalkyl which is unsubstituted, partially or fully halogenated and/or partially or fully substituted with CN, C₁-C₄-alkyl, C₃-C₄-cycloalkyl, C₁-C₄-alkoxy, C₁-C₄-haloalkoxy, and oxo;
N(R^{12a})R^{12b}, S(O)ₘR¹³, S(O)ₘN(R^{12a})R^{12b}, C(=U)R¹¹, C(=O)OR¹³, C(=U)N(R^{12a})R^{12b}, C(=S)SR¹³, C(=NR¹²)R¹¹;
phenyl, benzyl, phenoxy, or 3-, 4-, 5-, 6- or 7-membered saturated, partially or fully unsaturated heterocycle containing 1, 2, or 3 heteroatoms selected from N, O, S(O)ₘ as ring members, wherein the rings are unsubstituted or partially or fully substituted with R¹⁴; or
two R⁵¹ present on the same carbon atom of an alkyl, alkenyl, alkynyl or cycloalkyl group may together be =O, =CH(C₁-C₄-alkyl), =C(C₁-C₄-alkyl)₂, =N(C₁-C₆-alkyl), or =NO(C₁-C₆-alkyl);
R⁵² is a group as defined in R⁵¹ or selected from C₁-C₆-alkyl, C₂-C₆-alkenyl, and C₂-C₆-alkynyl, which groups are unsubstituted, partially or fully halogenated and/or substituted with one or two CN, C₃-C₄-cycloalkyl, C₁-C₄-alkoxy, C₁-C₄-haloalkoxy, or oxo;
R⁶ is a group as defined for R⁵; or
R⁵ and R⁶ form together with the nitrogen atom they are bonded to a 3-, 4-, 5-, 6-, 7- or 8-membered saturated, partially or fully unsaturated heterocycle which heterocycle may additionally contain one or two heteroatoms selected from N, O, and S(O)ₘ as ring members, and which heterocycle is unsubstituted or partially or fully substituted with R¹⁴; or
R⁵ and R⁶ together form a group =C(R¹¹)₂, =S(O)ₘ(R¹³)₂, =S(O)ₘN(R^{12a})R^{12b}, =NR¹², or =NOR¹³; or
R⁵ and R⁶ together form with the nitrogen they are bound to form a mesoionic entity;
Y having at G one or two ring atoms of G between connection points of G to X and Y; Y being a direct bond or CR⁷R⁸;
R⁷, R⁸ are each independently selected from H, halogen, CN, C₁-C₆-alkyl, C₃-C₃-cycloalkyl, C₂-C₆-alkenyl, and C₂-C₆-alkynyl, which groups may be partially or fully halogenated and/or may be substituted by one or more R⁸¹;
R⁸¹ is CN, N₃, NO₂, SCN, SF₅, C₃-C₈-cycloalkyl, C₃-C₈-halocycloalkyl; Si(C₁-C₄-alkyl)₃, OR¹³, OSO₂R¹³, S(O)ₘR¹³, NR²²R²³, C(=O)NR²²R²³; or
R⁷ and R⁸, together with the carbon atom they are bonded to, form a 3-, 4-, 5-, 6-, 7- or 8-membered saturated or partially unsaturated carbocycle or heterocycle, wherein the heterocycle comprises 1, 2, 3 or 4 heteroatoms or heteroatom groups independently selected from N, O, and S(O)ₘ as ring members, which rings are unsubstituted or substituted with 1 or 2 groups R¹⁴;
R¹¹ is CN, NO₂, OH, SH, SCN, SF₅, C₁-C₆-alkoxy, C₁-C₆-haloalkoxy, C₁-C₆-alkylS(O)ₘ, C₁-C₆-haloalkyl-S(O)ₘ, Si(C₁-C₄-alkyl)₃, C(=O)N(R^{12a})R^{12b}, C₃-C₈-cycloalkyl which is unsubstituted or partially or fully halogenated and/or substituted with 1 or 2 C₁-C₄-alkyl, C₃-C₄-cycloalkyl, C₁-C₄-alkoxy, C₁-C₄-haloalkoxy, and oxo;
phenyl, benzyl, phenoxy, a 3-, 4-, 5-, 6- or 7-membered saturated, partially or fully unsaturated heterocycle containing 1, 2 or 3 heteroatoms N, O, and S(O)ₘ as ring members, which rings are unsubstituted or partially or fully halogenated and/or substituted with R¹⁴; or
two R¹¹ present on the same carbon atom of an alkyl, alkenyl, alkynyl or cycloalkyl may together form =O, =CH(C₁-C₄-alkyl), =C(C₁-C₄-alkyl)C₁-C₄-alkyl, =N(C₁-C₆-alkyl), or =NO(C₁-C₆-alkyl); and
R¹¹ bonded to a cycloalkyl ring is additionally C₁-C₆-alkyl, C₂-C₆-alkenyl, and C₂-C₆-alkynyl, which are unsubstituted, partially or fully halogenated and/or substituted with 1 or 2 groups CN, C₃-C₄-cycloalkyl, C₁-C₄-alkoxy, C₁-C₄-haloalkoxy, and oxo; and
R¹¹ in groups =C(R¹¹)₂, N=C(R¹¹)₂, C(=U)R¹¹, and C(=NR¹²)R¹¹ is additionally selected from H, halogen, C₁-C₆-alkyl, C₂-C₆-alkenyl, and C₂-C₆-alkynyl, which are unsubstituted, partially or fully halogenated and/or substituted with 1 or 2 groups CN, C₃-C₄-cycloalkyl, C₁-C₄-alkoxy, C₁-C₄-haloalkoxy, and oxo;
R¹² is H, C₁-C₆-alkoxy, C₁-C₆-haloalkoxy, S(O)ₘ-C₁-C₄-alkyl, S(O)ₘ-C₁-C₄-haloalkyl, Si(C₁-C₄-alkyl)₃,
C₁-C₆-alkyl, C₃-C₆-alkenyl, C₃-C₆-alkynyl, which are unsubstituted or partially or fully halogenated and/or substituted with CN, C₁-C₄-alkoxy, C₁-C₄-haloalkoxy, S(O)ₘ-C₁-C₄-alkyl, S(O)ₘ-C₁-C₄-haloalkyl, C₃-C₆-cycloalkyl which is unsubstituted or substituted with 1 or 2 halogen and/or CN; and oxo; C₃-C₈-cycloalkyl which is unsubstituted or partially or fully halogenated and/or substituted with CN, C₁-C₄-alkyl, C₁-C₄-alkoxy, C₁-C₄-haloalkoxy, C₁-C₄-alkylthio, C₃-C₄-cycloalkyl, C₃-C₄-cycloalkyl-C₁-C₄-alkyl which rings may be substituted with 1 or 2 halogen and/or CN; and oxo;
phenyl, benzyl, pyridyl, phenoxy, which are unsubstituted or partially or fully halogenated and/or substituted with halogen, CN, NO₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C2-C4-alkenyl, C2-C4-haloalkenyl, C2-C4-alkynyl, C2-C4-haloalkynyl, C₃-C₆-cycloalkyl, C₃-C₆-halocycloalkyl, and (C₁-C₆-alkoxy)carbonyl; and a 3-, 4-, 5- or 6-membered saturated, partially or fully unsaturated heterocycle comprising 1, 2 or 3 heteroatoms selected from N, O, and S(O)ₘ as ring members, where the heterocycle is optionally substituted with one or more R¹⁴;
R^{12a} and R^{12b} have the meanings given for R¹²; or
R^{12a} and R^{12b}, together with the nitrogen atom to which they are bound, form a 3-, 4-, 5-, 6- or 7-membered saturated, partially unsaturated or maximally unsaturated heterocyclic ring, wherein the heterocyclic ring may additionally contain 1 or 2 heteroatoms or heteroatom groups selected from N, O, and S(O)ₘ as ring members, which heterocycle is unsubstituted or substituted with one or more substituents halogen, C₁-C₄-alkyl, C₁-C₄-haloalkyl, C₁-C₄-alkoxy, C₁-C₄-haloalkoxy, and oxo; or
R^{12a} and R¹², together with the nitrogen atoms to which they are bound in the group C(=NR¹²)N(R^{12a})R^{12b}, form a 3-, 4-, 5-, 6- or 7-membered saturated, partially unsaturated or maximally unsaturated heterocyclic ring, wherein the heterocyclic ring may additionally contain 1 or 2 heteroatoms or heteroatom groups selected from N, O, and S(O)ₘ as ring members, which heterocycle is unsubstituted or substituted with one or more substituents halogen, C₁-C₄-alkyl, C₁-C₄-haloalkyl, C₁-C₄-alkoxy, C₁-C₄-haloalkoxy, and oxo;
R¹³ is H, Si(C₁-C₄-alkyl)₃, C₁-C₆-alkyl, C₂-C₆-alkenyl, C₂-C₆-alkynyl, which are unsubstituted or partially or fully halogenated and/or substituted with C₃-C₄-cycloalkyl, C₁-C₄-alkoxy, C₁-C₄-haloalkoxy, S(O)ₘ-C₁-C₄-alkyl, and oxo; C₃-C₈-cycloalkyl which is unsubstituted or partially or fully halogenated and/or substituted with C₁-C₄-alkyl, C₃-C₄-cycloalkyl, C₁-C₄-alkoxy, C₁-C₄-haloalkoxy, S(O)ₘ-C₁-C₄-alkyl, and oxo;
phenyl, benzyl, pyridyl and phenoxy, which are unsubstituted or partially or fully halogenated and/or substituted with C₁-C₆-alkyl, C₁-C₆-haloalkyl, C₁-C₆-alkoxy, C₁-C₆-haloalkoxy, C₁-C₆-alkylthio, C₁-C₆-haloalkylthio, and (C₁-C₆-alkoxy)carbonyl;
R¹⁴ is halogen, NO₂, CN, OH, SH, C₁-C₆-alkoxy, C₁-C₆-haloalkoxy, S(O)ₘ-C₁-C₄-alkyl, S(O)ₘ-C₁-C₄-haloalkyl, C₁-C₄-alkylcarbonyl, C₁-C₄-haloalkylcarbonyl, C₁-C₄-alkoxycarbonyl, C₁-C₄-haloalkoxycarbonyl, C(=O)NR^{12a}R^{12b}, Si(C₁-C₄-alkyl)₃;
C₁-C₄-alkyl, C₂-C₆-alkenyl, C₂-C₆-alkynyl, which are unsubstituted or partially or fully halogenated and/or substituted with C₃-C₄-cycloalkyl, C₁-C₄-alkoxy, C₁-C₄-haloalkoxy, S(O)ₘ-C₁-C₄-alkyl, and oxo;
C₃-C₈-cycloalkyl which is unsubstituted or partially or fully halogenated and/or substituted with C₁-C₄-alkyl, C₃-C₄-cycloalkyl, C₁-C₄-alkoxy, C₁-C₄-haloalkoxy, S(O)ₘ-C₁-C₄-alkyl, and oxo;
phenyl, benzyl, pyridyl and phenoxy, which are unsubstituted or partially or fully halogenated and/or substituted with C₁-C₆-alkyl, C₁-C₆-haloalkyl, C₁-C₆-alkoxy, C₁-C₆-haloalkoxy, C₁-C₆-alkylthio, C₁-C₆-haloalkylthio, and (C₁-C₆-alkoxy)carbonyl; or
two R¹⁴ present together on the same atom of an unsaturated or partially unsaturated ring may be =O, =S, =N(C₁-C₆-alkyl), =NO(C₁-C₆-alkyl), =CH(C₁-C₄-alkyl) or =C(C₁-C₄-alkyl)C₁-C₄-alkyl; or
two R¹⁴ on two adjacent carbon atoms form together with the carbon atoms they are bonded to a 4-, 5-, 6-, 7- or 8-membered saturated, partially unsaturated or maximally unsaturated ring, wherein the ring may contain 1 or 2 heteroatoms or heteroatom groups selected from N, O, and S(O)ₘ as ring members, and wherein the ring is optionally substituted with one or more groups C₁-C₄-alkyl, C₁-C₄-haloalkyl, C₁-C₄-alkoxy, and/or C₁-C₄-haloalkoxy;
and the N-oxides, stereoisomers and agriculturally or veterinarily acceptable salts thereof.

2. Compounds of formula I according to claim 1, wherein U is O.

3. Compounds of formula I according to claim 1 or 2, wherein Y is a direct bond.

4. Compounds of formula I according to any one of claims 1 to 3, wherein
W is phenyl, which is substituted with one to three groups halogen, halomethyl, halomethoxy, halomethyl-S(O)ₘ.

5. Compounds of formula I according to any one of claims 1 to 4, wherein
X is NR³, and
R³ is H, alkoxy-carbonyl, C₁-C₆-alkyl, which alkyl is unsubstituted or substituted with CN, cycloalkyl, alkoxy;

6. Compounds of formula I according to any one of claims 1 to 5, wherein G is a group G1, G2, G3, or G4, 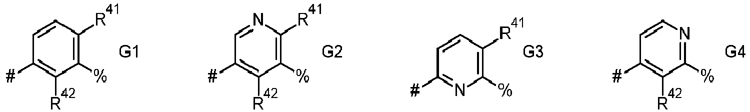 wherein # is the bond to X, % is the bond to Y, and R⁴¹ and R⁴² are H or a group R⁴, preferably H, or halogen, CN, halomethyl, halomethoxy.

7. Compounds of formula I according to claim 6, wherein G is G1 and R⁴² is H.

8. Compounds of formula I according to any one of claims 1 to 7, wherein
R⁵,R⁶ are independently from each other H, C₁-C₆-alkyl, C₁-C₆-haloalkyl, which are unsubstituted or partially or fully substituted with R⁵¹; or N(R^{12a})R^{12b}, S(O)ₘR¹³, C(=O)OR¹³, C(=U)N(R^{12a})R^{12b}, C₃-C₈-cycloalkyl, C₃-C₈-halocycloalkyl, phenyl or a 3-, 4-, 5-, 6- or 7-membered saturated, partially or fully unsaturated heterocycle comprising 1, 2, 3 or 4 heteroatoms selected from N, O, and S(O)ₘ as ring members, which rings are unsubstituted or partially or fully substituted with R⁵²;
R⁵¹ halogen, CN, N(R^{12a})R^{12b}, C(=O)N(R^{12a})R^{12b};
C₃-C₈-cycloalkyl which is unsubstituted, partially or fully halogenated and/or partially or fully substituted with CN;
S(O)ₘR¹³, C(=U)N(R^{12a})R^{12b};
phenyl, or 5-, 6- -membered saturated, partially or fully unsaturated heterocycle containing 1, 2, or 3 heteroatoms selected from N, O, S(O)ₘ as ring members, wherein the rings are unsubstituted or partially or fully substituted with R¹⁴; or
two R⁵¹ present on the same carbon atom of an alkyl, alkenyl, alkynyl or cycloalkyl group may together be =O, =CH(C₁-C₄-alkyl), =C(C₁-C₄-alkyl)₂, =N(C₁-C₆-alkyl), or =NO(C₁-C₆-alkyl);
R⁵² is a group as defined in R⁵¹ or selected from C₁-C₆-alkyl, C₂-C₆-alkenyl, and C₂-C₆-alkynyl, which groups are unsubstituted, partially or fully halogenated and/or substituted with one or two CN, C₃-C₄-cycloalkyl, C₁-C₄-alkoxy, C₁-C₄-haloalkoxy, or oxo;

9. Compounds of formula I according to any one of the preceding claims, which correspond to formula I.A

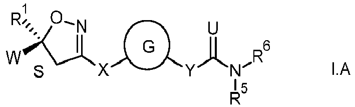

10. An agricultural or veterinary composition comprising at least one compound according to any one of claims 1 to 9 and/or at least one agriculturally or veterinarily acceptable salt thereof, and at least one inert liquid and/or solid agriculturally or veterinarily acceptable car-rier.

11. An agricultural composition for combating animal pests comprising at least one compound as defined in any of claims 1 to 9 and at least one inert liquid and/or solid acceptable car-rier and, if desired, at least one surfactant.

12. A method for combating or controlling invertebrate pests, which method comprises contact-ing said pest or its food supply, habitat or breeding grounds with a pesticidally effective amount of at least one compound as defined in any one of claims 1 to 9.

13. A method for protecting growing plants from attack or infestation by invertebrate pests, which method comprises contacting a plant, or soil or water in which the plant is growing, with a pesticidally effective amount of at least one compound as defined in any of claims 1 to 9.

14. Seed comprising a compound as defined in any of claims 1 to 9, or the enantiomers, diastereomers or salts thereof, in an amount of from 0.1 g to 10 kg per 100 kg of seed.

15. A method for treating or protecting an animal from infestation or infection by invertebrate pests which comprises bringing the animal in contact with a pesticidally effective amount of at least one compound of the formula I as defined in any of claims 1 to 9, a stereoisomer thereof and/or at least one veterinarily acceptable salt thereof.
